# EUROPEAN PATENT APPLICATION

(11) **EP 3 901 173 A1**
(43) Date of publication of application: **27.10.2021**
(21) Application number: 19898966.7
(22) Date of filing: 20.12.2019
(51) Int. Cl.: C07K 16/28, C07K 16/00, A61K 39/395, A61P 3/10, C12N 1/15, C12N 15/09, C12N 5/10

(54) **BISPECIFIC PROTEIN**

(30) Priority: 21.12.2018 CN 201811573634; 27.12.2018 CN 201811606887
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: CAO, Zhuoxiao, Shanghai 200245 (CN); LUO, Xiao, Shanghai 200245 (CN); HE, Ning, Shanghai 200245 (CN); HU, Qiyue, Shanghai 200245 (CN); ZHANG, Lianshan, Shanghai 200245 (CN); TAO, Weikang, Shanghai 200245 (CN)
(74) Representative: Potter Clarkson
(86) International application number: PCT/CN2019/126903
(87) International publication number: WO 2020/125744

(57) **Abstract**

Provided are a human GCGR antibody, a GLP-1 peptid and a mutant thereof, as well as a bispecific protein formed by fusion of the GCGR antibody and the GLP-1 peptide and a preparation method thereof, which can be used for weight loss and diabetes treatment.

Sequence listing

<110>  JIANGSU HENGRUI MEDICINE CO.,LTD.,
       SHANGHAI HENGRUI PHARMACEUTICAL CO., LTD.

<120>  BISPECIFIC PROTEIN

<160>  113

<170>  SIPOSequenceListing 1.0

<210>  1
<211>  477
<212>  PRT
<213>  Homo sapiens

<220>
<221>  PEPTIDE
<223>  full-length GCGR

<400>  1
Met Pro Pro Cys Gln Pro Gln Arg Pro Leu Leu Leu Leu Leu Leu Leu
1               5                   10                  15
Leu Ala Cys Gln Pro Gln Val Pro Ser Ala Gln Val Met Asp Phe Leu
            20                  25                  30
Phe Glu Lys Trp Lys Leu Tyr Gly Asp Gln Cys His His Asn Leu Ser
        35                  40                  45
Leu Leu Pro Pro Pro Thr Glu Leu Val Cys Asn Arg Thr Phe Asp Lys
    50                  55                  60
Tyr Ser Cys Trp Pro Asp Thr Pro Ala Asn Thr Thr Ala Asn Ile Ser
65                  70                  75                  80
Cys Pro Trp Tyr Leu Pro Trp His His Lys Val Gln His Arg Phe Val
                85                  90                  95
Phe Lys Arg Cys Gly Pro Asp Gly Gln Trp Val Arg Gly Pro Arg Gly
            100                 105                 110
Gln Pro Trp Arg Asp Ala Ser Gln Cys Gln Met Asp Gly Glu Glu Ile
        115                 120                 125
Glu Val Gln Lys Glu Val Ala Lys Met Tyr Ser Ser Phe Gln Val Met
    130                 135                 140
Tyr Thr Val Gly Tyr Ser Leu Ser Leu Gly Ala Leu Leu Leu Ala Leu
145                 150                 155                 160
Ala Ile Leu Gly Gly Leu Ser Lys Leu His Cys Thr Arg Asn Ala Ile
                165                 170                 175
His Ala Asn Leu Phe Ala Ser Phe Val Leu Lys Ala Ser Ser Val Leu
            180                 185                 190
Val Ile Asp Gly Leu Leu Arg Thr Arg Tyr Ser Gln Lys Ile Gly Asp
        195                 200                 205
Asp Leu Ser Val Ser Thr Trp Leu Ser Asp Gly Ala Val Ala Gly Cys
    210                 215                 220
Arg Val Ala Ala Val Phe Met Gln Tyr Gly Ile Val Ala Asn Tyr Cys
225                 230                 235                 240
Trp Leu Leu Val Glu Gly Leu Tyr Leu His Asn Leu Leu Gly Leu Ala
                245                 250                 255
Thr Leu Pro Glu Arg Ser Phe Phe Ser Leu Tyr Leu Gly Ile Gly Trp
            260                 265                 270
Gly Ala Pro Met Leu Phe Val Val Pro Trp Ala Val Val Lys Cys Leu
        275                 280                 285
Phe Glu Asn Val Gln Cys Trp Thr Ser Asn Asp Asn Met Gly Phe Trp
    290                 295                 300
Trp Ile Leu Arg Phe Pro Val Phe Leu Ala Ile Leu Ile Asn Phe Phe
305                 310                 315                 320
Ile Phe Val Arg Ile Val Gln Leu Leu Val Ala Lys Leu Arg Ala Arg

325                         330                         335
Gln Met His His Thr Asp Tyr Lys Phe Arg Leu Ala Lys Ser Thr Leu
            340                         345                         350
Thr Leu Ile Pro Leu Leu Gly Val His Glu Val Val Phe Ala Phe Val
        355                         360                         365
Thr Asp Glu His Ala Gln Gly Thr Leu Arg Ser Ala Lys Leu Phe Phe
    370                         375                         380
Asp Leu Phe Leu Ser Ser Phe Gln Gly Leu Leu Val Ala Val Leu Tyr
385                         390                         395                         400
Cys Phe Leu Asn Lys Glu Val Gln Ser Glu Leu Arg Arg Arg Trp His
                405                         410                         415
Arg Trp Arg Leu Gly Lys Val Leu Trp Glu Glu Arg Asn Thr Ser Asn
            420                         425                         430
His Arg Ala Ser Ser Ser Pro Gly His Gly Pro Pro Ser Lys Glu Leu
        435                         440                         445
Gln Phe Gly Arg Gly Gly Gly Ser Gln Asp Ser Ser Ala Glu Thr Pro
    450                         455                         460
Leu Ala Gly Gly Leu Pro Arg Leu Ala Glu Ser Pro Phe
465                         470                         475

<210>  2
<211>  122
<212>  PRT
<213>  Mus musculus

<220>
<221>  DOMAIN
<223>  m1803 heavy chain variable region


<400>  2
Gln Phe Gln Leu His Gln Ser Gly Ala Glu Leu Val Lys Pro Gly Ala
1               5                   10                  15
Ser Val Lys Leu Ser Cys Lys Ala Thr Gly Tyr Thr Phe Thr Asp Tyr
            20                  25                  30
Trp Ile Glu Trp Val Lys Gln Arg Pro Gly His Gly Leu Glu Trp Ile
        35                  40                  45
Gly Glu Ile Leu Pro Gly Ser Thr Tyr Thr Asn Tyr Asn Glu Lys Phe
    50                  55                  60
Lys Gly Arg Ala Thr Phe Thr Ala Glu Pro Ser Ser Ser Ser Ala Tyr
65                  70                  75                  80
Met Gln Leu Ser Gly Leu Thr Thr Glu Asp Ser Ala Ile Tyr Tyr Cys
                85                  90                  95
Ser Arg Gly Leu Ser Thr Leu Met Ala Val Asp Tyr Phe Asp Tyr Trp
            100                 105                 110
Gly Gln Gly Thr Thr Leu Thr Val Ser Ser
        115                 120

<210>  3
<211>  106
<212>  PRT
<213>  Mus musculus

<220>
<221>  DOMAIN
<223>  m1803 light chain variable region


<400>  3
Asp Ile Gln Met Thr Gln Thr Thr Ser Ser Leu Ser Ala Ser Leu Gly
1               5                   10                  15
Asp Arg Val Thr Ile Asn Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Ser Leu Thr Ile Ser His Leu Glu Gln
65                  70                  75                  80
Glu Asp Ile Ala Thr Tyr Phe Cys Gln Gln Thr Asn Ile Phe Pro Trp
                85                  90                  95
Thr Phe Gly Gly Gly Thr Lys Leu Glu Ile
            100                 105

<210>  4
<211>  120
<212>  PRT
<213>  Mus musculus

<220>
<221>  DOMAIN
<223>  m1805 heavy chain variable region


<400>  4
Glu Val Gln Leu Gln Gln Ser Gly Pro Glu Leu Val Lys Pro Gly Ala
1               5                   10                  15
Ser Val Lys Ile Pro Cys Lys Thr Ser Gly Tyr Thr Phe Thr Asp Tyr
            20                  25                  30
Asn Met Asp Trp Val Lys Gln Ser His Gly Arg Ser Leu Glu Trp Ile
        35                  40                  45
Gly Ser Ile Asp Pro Asp Asn Gly Gly Thr Ile Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Arg Ser Leu Thr Ser Glu Asp Thr Ala Val Tyr Tyr Cys
                85                  90                  95
Thr Arg Asp Tyr Tyr Gly Ser Ser Ser Trp Phe Ala Tyr Trp Gly Gln
            100                 105                 110
Gly Thr Leu Val Thr Val Ser Ala
        115                 120

<210>  5
<211>  107
<212>  PRT
<213>  Mus musculus

<220>
<221>  DOMAIN
<223>  m1805 light chain variable region


<400>  5
Asp Val Val Met Thr Gln Ser Pro Ala Thr Leu Ser Val Thr Pro Gly
1               5                   10                  15
Asp Arg Val Ser Leu Ser Cys Arg Ala Ser Gln Ser Ile Ser Asp Tyr
            20                  25                  30
Leu His Trp Tyr Gln Gln Lys Ser His Glu Ser Pro Arg Leu Leu Ile
        35                  40                  45
Lys Tyr Ala Ser Gln Ser Ile Ser Gly Ile Pro Ser Arg Phe Ser Gly
    50                  55                  60
Ser Gly Ser Gly Ser Asp Phe Thr Leu Ser Ile Asn Ser Val Glu Pro
65                  70                  75                  80
Glu Asp Val Gly Val Tyr Tyr Cys Gln Asn Gly His Ser Phe Pro Tyr
                85                  90                  95
Thr Phe Gly Gly Gly Thr Lys Leu Glu Ile Lys

100                 105

<210>  6
<211>  122
<212>  PRT
<213>  Mus musculus

<220>
<221>  DOMAIN
<223>  m1808 heavy chain variable region


<400>  6
Gln Val Gln Leu Gln Gln Ser Gly Ala Glu Leu Ala Arg Pro Gly Ala
1               5                   10                  15
Ser Val Lys Leu Ser Cys Lys Ala Ser Gly Asp Thr Phe Thr Thr Asn
            20                  25                  30
Gly Ile Ser Trp Val Lys Gln Arg Ile Gly Gln Gly Leu Glu Trp Ile
        35                  40                  45
Gly Glu Ile Tyr Pro Arg Ser Gly Asn Thr Tyr Tyr Asn Glu Asn Phe
    50                  55                  60
Lys Gly Lys Ala Thr Leu Thr Ala Asp Lys Ser Ser Thr Thr Ala Tyr
65                  70                  75                  80
Met Glu Leu Arg Arg Leu Thr Ser Glu Asp Ser Ala Val Tyr Phe Cys
                85                  90                  95
Ala Arg Ser Ile Thr Ser Val Ile Gly Ala Asp Tyr Phe Asp Tyr Trp
            100                 105                 110
Gly Gln Gly Thr Thr Leu Thr Val Ser Ser
        115                 120

<210>  7
<211>  107
<212>  PRT
<213>  Mus musculus

<220>
<221>  DOMAIN
<223>  m1808 light chain variable region


<400>  7
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Asn Tyr
            20                  25                  30
Leu Asn Trp Tyr Gln Lys Lys Pro Asp Gly Thr Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Phe Pro Trp
                85                  90                  95
Thr Phe Gly Gly Gly Thr Lys Leu Glu Ile Lys
            100                 105

<210>  8
<211>  123
<212>  PRT
<213> Mus musculus

<220>
<221>  DOMAIN

<223>  m1810 heavy chain variable region

<400>  8
Gln Val Gln Leu Gln Gln Ser Gly Thr Glu Leu Ala Arg Pro Gly Thr
1               5                   10                  15
Ser Val Thr Leu Ser Cys Lys Ala Ser Gly Tyr Thr Phe Thr Asn Tyr
            20                  25                  30
Ala Ile Ser Trp Val Lys Gln Arg Thr Gly Gln Gly Leu Glu Trp Ile
        35                  40                  45
Gly Glu Ile Tyr Pro Thr Ser Gly Asn Thr Tyr Tyr Asn Glu Lys Phe
    50                  55                  60
Lys Gly Lys Ala Thr Leu Thr Ala Asp Arg Ser Ser Lys Thr Met Tyr
65                  70                  75                  80
Met Glu Leu Arg Ser Leu Thr Ser Val Asp Ser Ala Val Tyr Phe Cys
                85                  90                  95
Ala Ser Gly Val Ile Thr Thr Val Val Ser Thr Asp Tyr Phe Asp Tyr
            100                 105                 110
Trp Gly Gln Gly Ser Pro Leu Thr Val Ser Ser
        115                 120

<210>  9
<211>  107
<212>  PRT
<213>  Mus musculus

<220>
<221>  DOMAIN
<223>  m1810 light chain variable region

<400>  9
Asp Ile Gln Met Thr Gln Ile Pro Ser Ser Leu Ser Val Ser Leu Gly
1               5                   10                  15
Asp Arg Val Thr Ile Ser Cys Arg Ala Ser Leu Asp Ile Ser Asn Tyr
            20                  25                  30
Leu Asn Trp Tyr Gln Leu Lys Pro Asp Gly Thr Val Lys Leu Leu Ile
        35                  40                  45
Tyr Tyr Thr Ser Thr Leu His Ser Gly Val Ser Ser Arg Phe Ser Gly
    50                  55                  60
Ser Gly Ser Gly Thr Glu Tyr Ser Leu Thr Ile Ser Asn Leu Glu Gln
65                  70                  75                  80
Glu Asp Ile Ala Thr Tyr Phe Cys Gln Gln Gly Asn Met Val Pro Tyr
                85                  90                  95
Thr Phe Gly Gly Gly Thr Lys Leu Glu Ile Lys
            100                 105

<210>  10
<211>  125
<212>  PRT
<213>  Rattus norvegicus

<220>
<221>  DOMAIN
<223>  rat1817 heavy chain variable region

<400>  10
Glu Val Gln Leu Val Glu Ser Gly Gly Asp Leu Val Gln Pro Gly Arg
1               5                   10                  15
Ser Met Lys Leu Ser Cys Ala Ala Ser Gly Phe Thr Phe Ser Asn Tyr
            20                  25                  30
Tyr Met Ala Trp Val Arg Gln Ala Pro Thr Lys Gly Leu Glu Trp Val

35                  40                  45
Ala Ser Ile Ser Thr Gly Gly Val Asn Thr Tyr Tyr Arg Asp Ser Val
    50                  55                  60
Lys Gly Arg Phe Thr Ile Ser Arg Asp Asn Ala Lys Asn Asn Leu Tyr
65                  70                  75                  80
Leu Gln Met Asp Ser Leu Arg Ser Glu Glu Thr Ala Thr Tyr Tyr Cys
                85                  90                  95
Ala Arg His Thr Thr Ala Asp Tyr Phe Tyr Gly Ile Tyr Phe Ala Leu
            100                 105                 110
Asp Ala Trp Gly Gln Gly Thr Ser Val Thr Val Ser Ser
        115                 120                 125

<210>  11
<211>  111
<212>  PRT
<213>  Rattus norvegicus

<220>
<221>  DOMAIN
<223>  rat1817 light chain variable region


<400>  11
Gln Phe Thr Leu Thr Gln Pro Lys Ser Val Ser Gly Ser Leu Arg Ser
1               5                   10                  15
Thr Ile Thr Ile Pro Cys Glu Arg Ser Ser Gly Asp Ile Gly Asp Ser
            20                  25                  30
Tyr Val Asn Trp Tyr Gln Gln His Leu Gly Arg Pro Pro Leu Asn Val
        35                  40                  45
Ile Tyr Ala Asp Val Gln Arg Pro Ser Glu Val Ser Asp Arg Phe Ser
    50                  55                  60
Gly Ser Ile Asp Ser Ser Ser Asn Ser Ala Ser Leu Thr Ile Thr Asn
65                  70                  75                  80
Leu Gln Met Asp Asp Glu Ala Asp Tyr Phe Cys Gln Ser Tyr Asp Thr
                85                  90                  95
Asn Ile Asp Ile Ile Phe Gly Gly Gly Thr Lys Leu Thr Val Leu
            100                 105                 110

<210>  12
<211>  125
<212>  PRT
<213>  Rattus norvegicus

<220>
<221>  DOMAIN
<223>  rat1822 heavy chain variable region


<400>  12
Glu Val Arg Leu Val Glu Ser Gly Gly Asp Phe Val Gln Pro Gly Arg
1               5                   10                  15
Ser Val Lys Leu Ser Cys Ala Ala Ser Gly Phe Thr Phe Ser Asn Tyr
            20                  25                  30
Tyr Met Ala Trp Val Arg Gln Ala Pro Thr Lys Gly Leu Glu Trp Val
        35                  40                  45
Gly Ser Ile Ser Thr Gly Gly Val Asn Thr Tyr Tyr Arg Asp Ser Val
    50                  55                  60
Lys Gly Arg Phe Thr Ile Ser Arg Asp Asn Ala Glu Ser Thr Leu Tyr
65                  70                  75                  80
Leu Gln Met Asp Ser Leu Arg Ser Glu Glu Thr Ala Thr Tyr Tyr Cys
                85                  90                  95
Ala Arg His Thr Thr Pro Asp Tyr His Tyr Gly Ile Tyr Phe Ala Met
            100                 105                 110

Asp Ala Trp Gly Gln Gly Thr Ser Val Thr Val Ser Ser
        115                 120                 125

<210>  13
<211>  111
<212>  PRT
<213>  Rattus norvegicus

<220>
<221>  DOMAIN
<223>  rat1822 light chain variable region


<400>  13
Gln Val Thr Leu Thr Gln Pro Lys Ser Val Ser Gly Ser Leu Arg Ser
1               5                   10                  15
Thr Ile Thr Ile Pro Cys Glu Arg Ser Ser Gly Asp Ile Gly Glu Ser
            20                  25                  30
Tyr Val Asn Trp Tyr Gln Gln His Leu Gly Arg Pro Pro Ile Asn Val
        35                  40                  45
Ile Tyr Ala Asp Asp Gln Arg Pro Ser Glu Val Ser Asp Arg Phe Ser
    50                  55                  60
Gly Ser Ile Asp Ser Ser Ser Asn Ser Ala Ser Leu Thr Ile Thr Asn
65                  70                  75                  80
Leu Gln Val Asp Asp Glu Ala Asp Tyr Phe Cys Gln Ser Tyr Asp Ser
                85                  90                  95
Ser Ile Asp Ile Phe Phe Gly Gly Gly Thr Lys Leu Thr Val Leu
            100                 105                 110

<210>  14
<211>  5
<212>  PRT
<213>  Artificial Sequence

<220>
<221>  DOMAIN
<223>  m1803 HCDR1


<400>  14
Asp Tyr Trp Ile Glu
1               5

<210>  15
<211>  17
<212>  PRT
<213>  Artificial Sequence

<220>
<221>  DOMAIN
<223>  m1803 HCDR2


<400>  15
Glu Ile Leu Pro Gly Ser Thr Tyr Thr Asn Tyr Asn Glu Lys Phe Lys
1               5                   10                  15
Gly


<210>  16
<211>  13
<212>  PRT
<213>  Artificial Sequence

<220>
<221>  DOMAIN
<223>  m1803 HCDR3


<400>  16
Gly Leu Ser Thr Leu Met Ala Val Asp Tyr Phe Asp Tyr
1               5                   10

<210>  17
<211>  11
<212>  PRT
<213>  Artificial Sequence

<220>
<221>  DOMAIN
<223>  m1803 LCDR1


<400>  17
Arg Ala Ser Gln Asp Ile Ser Asn Tyr Leu Asn
1               5                   10

<210>  18
<211>  7
<212>  PRT
<213>  Artificial Sequence

<220>
<221>  DOMAIN
<223>  m1803 LCDR2


<400>  18
Tyr Ser Ser Thr Leu His Ser
1               5

<210>  19
<211>  9
<212>  PRT
<213>  Artificial Sequence

<220>
<221>  DOMAIN
<223>  m1803 LCDR3


<400>  19
Gln Gln Thr Asn Ile Phe Pro Trp Thr
1               5

<210>  20
<211>  5
<212>  PRT
<213>  Artificial Sequence

<220>
<221>  DOMAIN
<223>  m1805 HCDR1


<400>  20

Asp Tyr Asn Met Asp
1               5

<210>  21
<211>  17
<212>  PRT
<213>  Artificial Sequence

<220>
<221>  DOMAIN
<223>  m1805 HCDR2


<400>  21
Ser Ile Asp Pro Asp Asn Gly Gly Thr Ile Tyr Asn Gln Lys Phe Lys
1               5                   10                  15
Gly


<210>  22
<211>  11
<212>  PRT
<213>  Artificial Sequence

<220>
<221>  DOMAIN
<223>  m1805 HCDR3


<400>  22
Asp Tyr Tyr Gly Ser Ser Ser Trp Phe Ala Tyr
1               5                   10

<210>  23
<211>  11
<212>  PRT
<213>  Artificial Sequence

<220>
<221>  DOMAIN
<223>  m1805 LCDR1


<400>  23
Arg Ala Ser Gln Ser Ile Ser Asp Tyr Leu His
1               5                   10

<210>  24
<211>  7
<212>  PRT
<213>  Artificial Sequence

<220>
<221>  DOMAIN
<223>  m1805 LCDR2


<400>  24
Tyr Ala Ser Gln Ser Ile Ser
1               5

<210>  25
<211>  9

<212>  PRT
<213>  Artificial Sequence

<220>
<221>  DOMAIN
<223>  m1805 LCDR3


<400>  25
Gln Asn Gly His Ser Phe Pro Tyr Thr
1               5

<210>  26
<211>  5
<212>  PRT
<213>  Artificial Sequence

<220>
<221>  DOMAIN
<223>  m1808 HCDR1


<400>  26
Thr Asn Gly Ile Ser
1               5

<210>  27
<211>  17
<212>  PRT
<213>  Artificial Sequence

<220>
<221>  DOMAIN
<223>  m1808 HCDR2


<400>  27
Glu Ile Tyr Pro Arg Ser Gly Asn Thr Tyr Tyr Asn Glu Asn Phe Lys
1               5                   10                  15
Gly


<210>  28
<211>  13
<212>  PRT
<213>  Artificial Sequence

<220>
<221>  DOMAIN
<223>  m1808 HCDR3


<400>  28
Ser Ile Thr Ser Val Ile Gly Ala Asp Tyr Phe Asp Tyr
1               5                   10

<210>  29
<211>  11
<212>  PRT
<213>  Artificial Sequence

<220>
<221>  DOMAIN

<223>  m1808 LCDR1

<400>  29
Arg Ala Ser Gln Asp Ile Ser Asn Tyr Leu Asn
1               5                   10

<210>  30
<211>  7
<212>  PRT
<213>  Artificial Sequence

<220>
<221>  DOMAIN
<223>  m1808 LCDR2

<400>  30
Tyr Ser Ser Thr Leu His Ser
1               5

<210>  31
<211>  9
<212>  PRT
<213>  Artificial Sequence

<220>
<221>  DOMAIN
<223>  m1808 LCDR3

<400>  31
Gln Gln Gly Asn Thr Phe Pro Trp Thr
1               5

<210>  32
<211>  5
<212>  PRT
<213>  Artificial Sequence

<220>
<221>  DOMAIN
<223>  m1810 HCDR1

<400>  32
Asn Tyr Ala Ile Ser
1               5

<210>  33
<211>  17
<212>  PRT
<213>  Artificial Sequence

<220>
<221>  DOMAIN
<223>  m1810 HCDR2

<400>  33
Glu Ile Tyr Pro Thr Ser Gly Asn Thr Tyr Tyr Asn Glu Lys Phe Lys
1               5                   10                  15
Gly

<210>  34
<211>  14
<212>  PRT
<213>  Artificial Sequence

<220>
<221>  DOMAIN
<223>  m1810 HCDR3


<400>  34
Gly Val Ile Thr Thr Val Val Ser Thr Asp Tyr Phe Asp Tyr
1               5                   10

<210>  35
<211>  11
<212>  PRT
<213>  Artificial Sequence

<220>
<221>  DOMAIN
<223>  m1810 LCDR1


<400>  35
Arg Ala Ser Leu Asp Ile Ser Asn Tyr Leu Asn
1               5                   10

<210>  36
<211>  7
<212>  PRT
<213>  Artificial Sequence

<220>
<221>  DOMAIN
<223>  m1810 LCDR2


<400>  36
Tyr Thr Ser Thr Leu His Ser
1               5

<210>  37
<211>  9
<212>  PRT
<213>  Artificial Sequence

<220>
<221>  DOMAIN
<223>  m1810 LCDR3


<400>  37
Gln Gln Gly Asn Met Val Pro Tyr Thr
1               5

<210>  38
<211>  5
<212>  PRT
<213>  Artificial Sequence

<220>
<221>  DOMAIN
<223>  rat1817 HCDR1


<400>  38
Asn Tyr Tyr Met Ala
1               5

<210>  39
<211>  17
<212>  PRT
<213>  Artificial Sequence

<220>
<221>  DOMAIN
<223>  rat1817 HCDR2


<400>  39
Ser Ile Ser Thr Gly Gly Val Asn Thr Tyr Tyr Arg Asp Ser Val Lys
1               5                   10                  15
Gly


<210>  40
<211>  16
<212>  PRT
<213>  Artificial Sequence

<220>
<221>  DOMAIN
<223>  rat1817 HCDR3


<400>  40
His Thr Thr Ala Asp Tyr Phe Tyr Gly Ile Tyr Phe Ala Leu Asp Ala
1               5                   10                  15

<210>  41
<211>  13
<212>  PRT
<213>  Artificial Sequence

<220>
<221>  DOMAIN
<223>  rat1817 LCDR1


<400>  41
Glu Arg Ser Ser Gly Asp Ile Gly Asp Ser Tyr Val Asn
1               5                   10

<210>  42
<211>  7
<212>  PRT
<213>  Artificial Sequence

<220>
<221>  DOMAIN
<223>  rat1817 LCDR2

<400>  42
Ala Asp Val Gln Arg Pro Ser
1               5

<210>  43
<211>  10
<212>  PRT
<213>  Artificial Sequence

<220>
<221>  DOMAIN
<223>  rat1817 LCDR3


<400>  43
Gln Ser Tyr Asp Thr Asn Ile Asp Ile Ile
1               5                   10

<210>  44
<211>  16
<212>  PRT
<213>  Artificial Sequence

<220>
<221>  DOMAIN
<223>  rat1822 HCDR3


<400>  44
His Thr Thr Pro Asp Tyr His Tyr Gly Ile Tyr Phe Ala Met Asp Ala
1               5                   10                  15

<210>  45
<211>  13
<212>  PRT
<213>  Artificial Sequence

<220>
<221>  DOMAIN
<223>  rat1822 LCDR1


<400>  45
Glu Arg Ser Ser Gly Asp Ile Gly Glu Ser Tyr Val Asn
1               5                   10

<210>  46
<211>  7
<212>  PRT
<213>  Artificial Sequence

<220>
<221>  DOMAIN
<223>  rat1822 LCDR2


<400>  46
Ala Asp Asp Gln Arg Pro Ser
1               5

<210>  47
<211>  10
<212>  PRT

<213>  Artificial Sequence

<220>
<221>  DOMAIN
<223>  rat1822 LCDR3


<400>  47
Gln Ser Tyr Asp Ser Ser Ile Asp Ile Phe
1               5                   10

<210>  48
<211>  5
<212>  PRT
<213>  Artificial Sequence

<220>
<221>  DOMAIN
<223>  mouse antibody HCDR1 general formula


<220>
<221>  DOMAIN
<222>  (1)..(3)
<223>  Xaa-Xaa-Xaa is selected from Asp-Tyr-Trp, Thr-Asn-Gly or Asn-Tyr-Ala.


<220>
<221>  DOMAIN
<222>  (5)..(5)
<223>  Xaa is selected from Glu or Ser.


<400>  48
Xaa Xaa Xaa Ile Xaa
1               5

<210>  49
<211>  17
<212>  PRT
<213>  Artificial Sequence

<220>
<221>  DOMAIN
<223>  mouse antibody HCDR2 general formula


<220>
<221>  DOMAIN
<222>  (3)..(3)
<223>  Xaa is selected from Leu or Tyr.


<220>
<221>  DOMAIN
<222>  (5)..(5)
<223>  Xaa is selected from Gly, Arg or Thr.


<220>
<221>  DOMAIN
<222>  (7)..(7)
<223>  Xaa is selected from Gly or Thr.

<220>
<221>  DOMAIN
<222>  (8)..(8)
<223>  Xaa is selected from Tyr or Asn.


<220>
<221>  DOMAIN
<222>  (10)..(10)
<223>  Xaa is selected from Asn or Tyr.


<400>  49
Glu Ile Xaa Pro Xaa Ser Xaa Xaa Thr Xaa Tyr Asn Glu Xaa Phe Lys
1               5                   10                  15
Gly


<210>  50
<211>  13
<212>  PRT
<213>  Artificial Sequence

<220>
<221>  DOMAIN
<223>  mouse antibody HCDR3 general formula

<220>
<221>  DOMAIN
<222>  (1)..(1)
<223>  Xaa is selected from Gly or Ser.

<220>
<221>  DOMAIN
<222>  (2)..(2)
<223>  Xaa is selected from Ile, Leu or Val-Ile.


<220>
<221>  DOMAIN
<222>  (3)..(3)
<223>  Xaa is selected from Ser or Thr


<220>
<221>  DOMAIN
<222>  (4)..(4)
<223>  Xaa is selected from Ser or Thr


<220>
<221>  DOMAIN
<222>  (5)..(5)
<223>  Xaa is selected from Leu or Val.


<220>
<221>  DOMAIN
<222>  (6)..(6)
<223>  Xaa is selected from Met, Val or Ile.

<220>
<221>  DOMAIN
<222>  (7)..(7)
<223>  Xaa is selected from Ser, Gly or Ala.


<220>
<221>  DOMAIN
<222>  (8)..(8)
<223>  Xaa is selected from Thr, Ala or Val.


<400>  50
Xaa Xaa Xaa Xaa Xaa Xaa Xaa Xaa Asp Tyr Phe Asp Tyr
1               5                   10

<210>  51
<211>  11
<212>  PRT
<213>  Artificial Sequence

<220>
<221>  DOMAIN
<223>  mouse antibody LCDR1 general formula

<220>
<221>  DOMAIN
<222>  (4)..(4)
<223>  Xaa is selected from Leu or Gln.


<400>  51
Arg Ala Ser Xaa Asp Ile Ser Asn Tyr Leu Asn
1               5                   10

<210>  52
<211>  7
<212>  PRT
<213>  Artificial Sequence

<220>
<221>  DOMAIN
<223>  mouse antibody LCDR2 general formula


<220>
<221>  DOMAIN
<222>  (2)..(2)
<223>  Xaa is selected from Ser or Thr.


<400>  52
Tyr Xaa Ser Thr Leu His Ser
1               5

<210>  53
<211>  9
<212>  PRT
<213>  Artificial Sequence

<220>
<221>  DOMAIN

<223>  mouse antibody LCDR3 general formula

<220>
<221>  DOMAIN
<222>  (3)..(3)
<223>  Xaa is selected from Thr or Gly.

<220>
<221>  DOMAIN
<222>  (5)..(5)
<223>  Xaa is selected from Met, Thr or Ile.

<220>
<221>  DOMAIN
<222>  (6)..(6)
<223>  Xaa is selected from Phe or Val.

<220>
<221>  DOMAIN
<222>  (8)..(8)
<223>  Xaa is selected from Trp or Tyr.

<400>  53
Gln Gln Xaa Asn Xaa Xaa Pro Xaa Thr
1               5

<210>  54
<211>  16
<212>  PRT
<213>  Artificial Sequence

<220>
<221>  DOMAIN
<223>  rat antibody HCDR3 general formula

<220>
<221>  DOMAIN
<222>  (4)..(4)
<223>  Xaa is selected from Pro or Ala.

<220>
<221>  DOMAIN
<222>  (7)..(7)
<223>  Xaa is selected from Phe or His.

<220>
<221>  DOMAIN
<222>  (14)..(14)
<223>  Xaa is selected from Leu or Met.

<400>  54
His Thr Thr Xaa Asp Tyr Xaa Tyr Gly Ile Tyr Phe Ala Xaa Asp Ala
1               5                   10                  15

<210>  55
<211>  13
<212>  PRT
<213>  Artificial Sequence

<220>
<221>  DOMAIN
<223>  rat antibody LCDR1 general formula


<220>
<221>  DOMAIN
<222>  (9)..(9)
<223>  Xaa is selected from Glu or Asp.



<400>  55
Glu Arg Ser Ser Gly Asp Ile Gly Xaa Ser Tyr Val Asn
1               5                   10

<210>  56
<211>  7
<212>  PRT
<213>  Artificial Sequence

<220>
<221>  DOMAIN
<223>  rat antibody LCDR2 general formula


<220>
<221>  DOMAIN
<222>  (3)..(3)
<223>  Xaa is selected from Asp or Val.



<400>  56
Ala Asp Xaa Gln Arg Pro Ser
1               5

<210>  57
<211>  10
<212>  PRT
<213>  Artificial Sequence

<220>
<221>  DOMAIN
<223>  rat antibody LCDR3 general formula


<220>
<221>  DOMAIN
<222>  (5)..(5)
<223>  Xaa is selected from Ser or Thr.


<220>
<221>  DOMAIN
<222>  (6)..(6)
<223>  Xaa is selected from Ser or Asn.

<220>
<221>  DOMAIN
<222>  (10)..(10)
<223>  Xaa is selected from Phe or Ile.

<400>  57
Gln Ser Tyr Asp Xaa Xaa Ile Asp Ile Xaa
1               5                   10

<210>  58
<211>  107
<212>  PRT
<213>  Artificial Sequence

<220>
<221>  DOMAIN
<223>  hu1803_VL.1

<400>  58
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Ile Phe Pro Trp
                85                  90                  95
Thr Phe Gly Gly Gly Thr Lys Val Glu Ile Lys
            100                 105

<210>  59
<211>  107
<212>  PRT
<213>  Artificial Sequence

<220>
<221>  DOMAIN
<223>  hu1803_VL.1A

<400>  59
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Gly Ala Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Phe Cys Gln Gln Thr Asn Ile Phe Pro Trp
                85                  90                  95
Thr Phe Gly Gly Gly Thr Lys Val Glu Ile Lys

100                 105

<210>  60
<211>  107
<212>  PRT
<213>  Artificial Sequence

<220>
<221>  DOMAIN
<223>  hu1803_VL.1B


<400>  60
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Lys Ala Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Thr Asn Ile Phe Pro Trp
                85                  90                  95
Thr Phe Gly Gly Gly Thr Lys Val Glu Ile Lys
            100                 105

<210>  61
<211>  122
<212>  PRT
<213>  Artificial Sequence

<220>
<221>  DOMAIN
<223>  hu1803_VH.1


<400>  61
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Trp Ile Glu Trp Val Arg Gln Ala Pro Gly Gln Gly Leu Glu Trp Met
        35                  40                  45
Gly Glu Ile Leu Pro Gly Ser Thr Tyr Thr Asn Tyr Asn Glu Lys Phe
    50                  55                  60
Lys Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Leu Ser Thr Leu Met Ala Val Asp Tyr Phe Asp Tyr Trp
            100                 105                 110
Gly Gln Gly Thr Thr Val Thr Val Ser Ser
        115                 120

<210>  62
<211>  122
<212>  PRT
<213>  Artificial Sequence

<220>
<221>  DOMAIN

<223>  hu1803_VH.1A

<400>  62
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Trp Ile Glu Trp Val Arg Gln Ala Pro Gly Gln Gly Leu Glu Trp Met
        35                  40                  45
Gly Glu Ile Leu Pro Gly Ser Thr Tyr Thr Asn Tyr Asn Glu Lys Phe
    50                  55                  60
Lys Gly Arg Val Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Leu Ser Thr Leu Met Ala Val Asp Tyr Phe Asp Tyr Trp
            100                 105                 110
Gly Gln Gly Thr Thr Val Thr Val Ser Ser
        115                 120

<210>  63
<211>  122
<212>  PRT
<213>  Artificial Sequence

<220>
<221>  DOMAIN
<223>  hu1803_VH.1B

<400>  63
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Thr Tyr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ser Arg Gly Leu Ser Thr Leu Met Ala Val Asp Tyr Phe Asp Tyr Trp
            100                 105                 110
Gly Gln Gly Thr Thr Val Thr Val Ser Ser
        115                 120

<210>  64
<211>  122
<212>  PRT
<213>  Artificial Sequence

<220>
<221>  DOMAIN
<223>  hu1803_VH.1C

<400>  64
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr

20                  25                  30
Trp Ile Glu Trp Val Lys Gln Ala Pro Gly Gln Gly Leu Glu Trp Ile
        35                  40                  45
Gly Glu Ile Leu Pro Gly Ser Thr Tyr Thr Asn Tyr Asn Glu Lys Phe
    50                  55                  60
Lys Gly Arg Ala Thr Phe Thr Ala Asp Pro Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ser Arg Gly Leu Ser Thr Leu Met Ala Val Asp Tyr Phe Asp Tyr Trp
            100                 105                 110
Gly Gln Gly Thr Thr Val Thr Val Ser Ser
        115                 120

<210>  65
<211>  107
<212>  PRT
<213>  Artificial Sequence

<220>
<221>  DOMAIN
<223>  Hu1810_VL.1


<400>  65
Asp Ile Gln Met Thr Gln Ser Pro Ser Ser Leu Ser Ala Ser Val Gly
1               5                   10                  15
Asp Arg Val Thr Ile Thr Cys Arg Ala Ser Leu Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Tyr Thr Ser Thr Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Gly Asn Met Val Pro Tyr
                85                  90                  95
Thr Phe Gly Gly Gly Thr Lys Val Glu Ile Lys
            100                 105

<210>  66
<211>  107
<212>  PRT
<213>  Artificial Sequence

<220>
<221>  DOMAIN
<223>  Hu1810_VL.1A


<400>  66
Asp Ile Gln Met Thr Gln Ser Pro Ser Ser Leu Ser Ala Ser Val Gly
1               5                   10                  15
Asp Arg Val Thr Ile Thr Cys Arg Ala Ser Leu Asp Ile Ser Asn Tyr
            20                  25                  30
Leu Asn Trp Tyr Gln Gln Lys Pro Gly Lys Ala Val Lys Leu Leu Ile
        35                  40                  45
Tyr Tyr Thr Ser Thr Leu His Ser Gly Val Ser Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Gly Asn Met Val Pro Tyr
                85                  90                  95

Thr Phe Gly Gly Gly Thr Lys Val Glu Ile Lys
            100                 105

<210>  67
<211>  107
<212>  PRT
<213>  Artificial Sequence

<220>
<221>  DOMAIN
<223>  Hu1810_VL.1B


<400>  67
Asp Ile Gln Met Thr Gln Ser Pro Ser Ser Leu Ser Ala Ser Val Gly
1               5                   10                  15
Asp Arg Val Thr Ile Thr Cys Arg Ala Ser Leu Asp Ile Ser Asn Tyr
            20                  25                  30
Leu Asn Trp Tyr Gln Leu Lys Pro Gly Lys Ala Val Lys Leu Leu Ile
        35                  40                  45
Tyr Tyr Thr Ser Thr Leu His Ser Gly Val Ser Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Gln Gln Gly Asn Met Val Pro Tyr
                85                  90                  95
Thr Phe Gly Gly Gly Thr Lys Val Glu Ile Lys
            100                 105

<210>  68
<211>  123
<212>  PRT
<213>  Artificial Sequence

<220>
<221>  DOMAIN
<223>  Hu1810_VH.1


<400>  68
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asn Tyr
            20                  25                  30
Ala Ile Ser Trp Val Arg Gln Ala Pro Gly Gln Gly Leu Glu Trp Met
        35                  40                  45
Gly Glu Ile Tyr Pro Thr Ser Gly Asn Thr Tyr Tyr Asn Glu Lys Phe
    50                  55                  60
Lys Gly Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Val Ile Thr Thr Val Val Ser Thr Asp Tyr Phe Asp Tyr
            100                 105                 110
Trp Gly Gln Gly Thr Leu Val Thr Val Ser Ser
        115                 120

<210>  69
<211>  123
<212>  PRT
<213>  Artificial Sequence

<220>

<221>  DOMAIN
<223>  Hu1810_VH.1A

<400>  69
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asn Tyr
            20                  25                  30
Ala Ile Ser Trp Val Arg Gln Ala Pro Gly Gln Gly Leu Glu Trp Met
        35                  40                  45
Gly Glu Ile Tyr Pro Thr Ser Gly Asn Thr Tyr Tyr Asn Glu Lys Phe
    50                  55                  60
Lys Gly Arg Val Thr Ile Thr Ala Asp Arg Ser Thr Ser Thr Met Tyr
65                  70                  75                  80
Met Glu Leu Ser Ser Leu Arg Ser Glu Asp Thr Ala Val Tyr Tyr Cys
                85                  90                  95
Ala Ser Gly Val Ile Thr Thr Val Val Ser Thr Asp Tyr Phe Asp Tyr
            100                 105                 110
Trp Gly Gln Gly Thr Leu Val Thr Val Ser Ser
        115                 120

<210>  70
<211>  123
<212>  PRT
<213>  Artificial Sequence

<220>
<221>  DOMAIN
<223>  Hu1810_VH.1B

<400>  70
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asn Tyr
            20                  25                  30
Ala Ile Ser Trp Val Arg Gln Ala Pro Gly Gln Gly Leu Glu Trp Ile
        35                  40                  45
Gly Glu Ile Tyr Pro Thr Ser Gly Asn Thr Tyr Tyr Asn Glu Lys Phe
    50                  55                  60
Lys Gly Arg Ala Thr Leu Thr Ala Asp Arg Ser Thr Ser Thr Met Tyr
65                  70                  75                  80
Met Glu Leu Ser Ser Leu Arg Ser Glu Asp Thr Ala Val Tyr Tyr Cys
                85                  90                  95
Ala Ser Gly Val Ile Thr Thr Val Val Ser Thr Asp Tyr Phe Asp Tyr
            100                 105                 110
Trp Gly Gln Gly Thr Leu Val Thr Val Ser Ser
        115                 120

<210>  71
<211>  123
<212>  PRT
<213>  Artificial Sequence

<220>
<221>  DOMAIN
<223>  Hu1810_VH.1C

<400>  71
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Asn Tyr
            20                  25                  30
Ala Ile Ser Trp Val Lys Gln Ala Pro Gly Gln Gly Leu Glu Trp Ile
        35                  40                  45
Gly Glu Ile Tyr Pro Thr Ser Gly Asn Thr Tyr Tyr Asn Glu Lys Phe
    50                  55                  60
Lys Gly Lys Ala Thr Leu Thr Ala Asp Arg Ser Thr Ser Thr Met Tyr
65                  70                  75                  80
Met Glu Leu Ser Ser Leu Arg Ser Glu Asp Thr Ala Val Tyr Tyr Cys
                85                  90                  95
Ala Ser Gly Val Ile Thr Thr Val Val Ser Thr Asp Tyr Phe Asp Tyr
            100                 105                 110
Trp Gly Gln Gly Thr Leu Val Thr Val Ser Ser
        115                 120

<210>  72
<211>  327
<212>  PRT
<213>  Artificial Sequence

<220>
<221>  DOMAIN
<223>  IgG4-AA heavy chain constant region


<400>  72
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Ala Ala Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser

290                 295                 300
Cys Ser Val Met His Glu Ala Leu His Asn His Tyr Thr Gln Lys Ser
305                 310                 315                 320
Leu Ser Leu Ser Leu Gly Lys
                325

<210>  73
<211>  107
<212>  PRT
<213>  Artificial Sequence

<220>
<221>  DOMAIN
<223>  antibody light chain (Kappa chain) constant region


<400>  73

<210>  74
<211>  449
<212>  PRT
<213>  Artificial Sequence

<220>
<221>  CHAIN
<223>  ch1803 heavy chain


<400>  74
   Gln Phe Gln Leu His Gln Ser Gly Ala Glu Leu Val Lys Pro Gly Ala
   1               5                   10                  15
   Ser Val Lys Leu Ser Cys Lys Ala Thr Gly Tyr Thr Phe Thr Asp Tyr
               20                  25                  30
   Trp Ile Glu Trp Val Lys Gln Arg Pro Gly His Gly Leu Glu Trp Ile
           35                  40                  45
   Gly Glu Ile Leu Pro Gly Ser Thr Tyr Thr Asn Tyr Asn Glu Lys Phe
       50                  55                  60
   Lys Gly Arg Ala Thr Phe Thr Ala Glu Pro Ser Ser Ser Ser Ala Tyr
   65                  70                  75                  80
   Met Gln Leu Ser Gly Leu Thr Thr Glu Asp Ser Ala Ile Tyr Tyr Cys
                   85                  90                  95
   Ser Arg Gly Leu Ser Thr Leu Met Ala Val Asp Tyr Phe Asp Tyr Trp
               100                 105                 110
   Gly Gln Gly Thr Thr Leu Thr Val Ser Ser Ala Ser Thr Lys Gly Pro
           115                 120                 125
   Ser Val Phe Pro Leu Ala Pro Cys Ser Arg Ser Thr Ser Glu Ser Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Lys Thr Tyr Thr Cys Asn Val Asp
           195                 200                 205
   His Lys Pro Ser Asn Thr Lys Val Asp Lys Arg Val Glu Ser Lys Tyr
       210                 215                 220
   Gly Pro Pro Cys Pro Pro Cys Pro Ala Pro Glu Ala Ala Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser Gln Glu Asp
               260                 265                 270
   Pro Glu Val Gln Phe Asn Trp Tyr Val Asp Gly Val Glu Val His Asn
           275                 280                 285
   Ala Lys Thr Lys Pro Arg Glu Glu Gln Phe Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Gly Leu Pro Ser Ser Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Gln Glu Glu Met Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Arg Leu Thr Val Asp Lys
                   405                 410                 415
   Ser Arg Trp Gln Glu Gly Asn Val Phe Ser Cys Ser Val Met His Glu
               420                 425                 430
   Ala Leu His Asn His Tyr Thr Gln Lys Ser Leu Ser Leu Ser Leu Gly
           435                 440                 445
   Lys


<210>  75
<211>  213
<212>  PRT
<213>  Artificial Sequence

<220>
<221>  CHAIN
<223>  ch1803 light chain


<400>  75
Asp Ile Gln Met Thr Gln Thr Thr Ser Ser Leu Ser Ala Ser Leu Gly
1               5                   10                  15
Asp Arg Val Thr Ile Asn Cys Arg Ala Ser Gln Asp Ile Ser Asn Tyr
            20                  25                  30
Leu Asn Trp Tyr Gln Gln Lys Pro Asp Gly Thr Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Ser Leu Thr Ile Ser His Leu Glu Gln
65                  70                  75                  80
Glu Asp Ile Ala Thr Tyr Phe Cys Gln Gln Thr Asn Ile Phe Pro Trp
                85                  90                  95
Thr Phe Gly Gly Gly Thr Lys Leu Glu Ile Arg Thr Val Ala Ala Pro

                100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210

<210>  76
<211>  449
<212>  PRT
<213>  Artificial Sequence

<220>
<221>  CHAIN
<223>  hu1803-1 heavy chain


<400>  76
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Trp Ile Glu Trp Val Arg Gln Ala Pro Gly Gln Gly Leu Glu Trp Met
        35                  40                  45
Gly Glu Ile Leu Pro Gly Ser Thr Tyr Thr Asn Tyr Asn Glu Lys Phe
    50                  55                  60
Lys Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Leu Ser Thr Leu Met Ala Val Asp Tyr Phe Asp Tyr Trp
            100                 105                 110
Gly Gln Gly Thr Thr Val Thr Val Ser Ser Ala Ser Thr Lys Gly Pro
        115                 120                 125
Ser Val Phe Pro Leu Ala Pro Cys Ser Arg Ser Thr Ser Glu Ser Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Lys Thr Tyr Thr Cys Asn Val Asp
        195                 200                 205
His Lys Pro Ser Asn Thr Lys Val Asp Lys Arg Val Glu Ser Lys Tyr
    210                 215                 220
Gly Pro Pro Cys Pro Pro Cys Pro Ala Pro Glu Ala Ala Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser Gln Glu Asp
            260                 265                 270
Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Gly Leu Pro Ser Ser Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Gln Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Arg Leu Thr Val Asp Lys
                405                 410                 415
Ser Arg Trp Gln Glu Gly Asn Val Phe Ser Cys Ser Val Met His Glu
            420                 425                 430
Ala Leu His Asn His Tyr Thr Gln Lys Ser Leu Ser Leu Ser Leu Gly
        435                 440                 445
Lys

<210>  77
<211>  214
<212>  PRT
<213>  Artificial Sequence

<220>
<221>  CHAIN
<223>  hu1803-1 light chain

<400>  77
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Thr Asn Ile Phe Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  78
<211>  449
<212>  PRT
<213>  Artificial Sequence

<220>
<221>  CHAIN
<223>  hu1803-9 heavy chain

<400>  78
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Trp Ile Glu Trp Val Arg Gln Ala Pro Gly Gln Gly Leu Glu Trp Met
        35                  40                  45
Gly Glu Ile Leu Pro Gly Ser Thr Tyr Thr Asn Tyr Asn Glu Lys Phe
    50                  55                  60
Lys Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Leu Ser Thr Leu Met Ala Val Asp Tyr Phe Asp Tyr Trp
            100                 105                 110
Gly Gln Gly Thr Thr Val Thr Val Ser Ser Ala Ser Thr Lys Gly Pro
        115                 120                 125
Ser Val Phe Pro Leu Ala Pro Cys Ser Arg Ser Thr Ser Glu Ser Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Lys Thr Tyr Thr Cys Asn Val Asp
        195                 200                 205
His Lys Pro Ser Asn Thr Lys Val Asp Lys Arg Val Glu Ser Lys Tyr
    210                 215                 220
Gly Pro Pro Cys Pro Pro Cys Pro Ala Pro Glu Ala Ala Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser Gln Glu Asp
            260                 265                 270
Pro Glu Val Gln Phe Asn Trp Tyr Val Asp Gly Val Glu Val His Asn
        275                 280                 285
Ala Lys Thr Lys Pro Arg Glu Glu Gln Phe Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Gly Leu Pro Ser Ser Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Gln Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Arg Leu Thr Val Asp Lys

                405                 410                 415
Ser Arg Trp Gln Glu Gly Asn Val Phe Ser Cys Ser Val Met His Glu
            420                 425                 430
Ala Leu His Asn His Tyr Thr Gln Lys Ser Leu Ser Leu Ser Leu Gly
        435                 440                 445
Lys

<210>  79
<211>  214
<212>  PRT
<213>  Artificial Sequence

<220>
<221>  CHAIN
<223>  hu1803-9 light chain

<400>  79
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Asn Tyr
            20                  25                  30
Leu Asn Trp Tyr Gln Gln Lys Pro Gly Lys Ala Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Thr Asn Ile Phe Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  80
<211>  447
<212>  PRT
<213>  Artificial Sequence

<220>
<221>  CHAIN
<223>  ch1805 heavy chain

<400>  80
Glu Val Gln Leu Gln Gln Ser Gly Pro Glu Leu Val Lys Pro Gly Ala
1               5                   10                  15
Ser Val Lys Ile Pro Cys Lys Thr Ser Gly Tyr Thr Phe Thr Asp Tyr
            20                  25                  30

Asn Met Asp Trp Val Lys Gln Ser His Gly Arg Ser Leu Glu Trp Ile
        35                  40                  45
Gly Ser Ile Asp Pro Asp Asn Gly Gly Thr Ile Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Arg Ser Leu Thr Ser Glu Asp Thr Ala Val Tyr Tyr Cys
                85                  90                  95
Thr Arg Asp Tyr Tyr Gly Ser Ser Ser Trp Phe Ala Tyr Trp Gly Gln
            100                 105                 110
Gly Thr Leu Val Thr Val Ser Ala Ala Ser Thr Lys Gly Pro Ser Val
        115                 120                 125
Phe Pro Leu Ala Pro Cys Ser Arg Ser Thr Ser Glu Ser Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Lys Thr Tyr Thr Cys Asn Val Asp His Lys
        195                 200                 205
Pro Ser Asn Thr Lys Val Asp Lys Arg Val Glu Ser Lys Tyr Gly Pro
    210                 215                 220
Pro Cys Pro Pro Cys Pro Ala Pro Glu Ala Ala Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser Gln Glu Asp Pro Glu
            260                 265                 270
Val Gln Phe Asn Trp Tyr Val Asp Gly Val Glu Val His Asn Ala Lys
        275                 280                 285
Thr Lys Pro Arg Glu Glu Gln Phe Asn Ser Thr Tyr Arg Val Val Ser
    290                 295                 300
Val Leu Thr Val Leu His Gln Asp Trp Leu Asn Gly Lys Glu Tyr Lys
305                 310                 315                 320
Cys Lys Val Ser Asn Lys Gly Leu Pro Ser Ser Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Gln Glu Glu Met Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Arg Leu Thr Val Asp Lys Ser Arg
                405                 410                 415
Trp Gln Glu Gly Asn Val Phe Ser Cys Ser Val Met His Glu Ala Leu
            420                 425                 430
His Asn His Tyr Thr Gln Lys Ser Leu Ser Leu Ser Leu Gly Lys
        435                 440                 445

<210>  81
<211>  214
<212>  PRT
<213>  Artificial Sequence

<220>
<221>  CHAIN
<223>  ch1805 light chain

<400>  81
Asp Val Val Met Thr Gln Ser Pro Ala Thr Leu Ser Val Thr Pro Gly
1               5                   10                  15
Asp Arg Val Ser Leu Ser Cys Arg Ala Ser Gln Ser Ile Ser Asp Tyr
            20                  25                  30
Leu His Trp Tyr Gln Gln Lys Ser His Glu Ser Pro Arg Leu Leu Ile
        35                  40                  45
Lys Tyr Ala Ser Gln Ser Ile Ser Gly Ile Pro Ser Arg Phe Ser Gly
    50                  55                  60
Ser Gly Ser Gly Ser Asp Phe Thr Leu Ser Ile Asn Ser Val Glu Pro
65                  70                  75                  80
Glu Asp Val Gly Val Tyr Tyr Cys Gln Asn Gly His Ser Phe Pro Tyr
                85                  90                  95
Thr Phe Gly Gly Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  82
<211>  449
<212>  PRT
<213>  Artificial Sequence

<220>
<221>  CHAIN
<223>  ch1808 heavy chain

<400>  82
Gln Val Gln Leu Gln Gln Ser Gly Ala Glu Leu Ala Arg Pro Gly Ala
1               5                   10                  15
Ser Val Lys Leu Ser Cys Lys Ala Ser Gly Asp Thr Phe Thr Thr Asn
            20                  25                  30
Gly Ile Ser Trp Val Lys Gln Arg Ile Gly Gln Gly Leu Glu Trp Ile
        35                  40                  45
Gly Glu Ile Tyr Pro Arg Ser Gly Asn Thr Tyr Tyr Asn Glu Asn Phe
    50                  55                  60
Lys Gly Lys Ala Thr Leu Thr Ala Asp Lys Ser Ser Thr Thr Ala Tyr
65                  70                  75                  80
Met Glu Leu Arg Arg Leu Thr Ser Glu Asp Ser Ala Val Tyr Phe Cys
                85                  90                  95
Ala Arg Ser Ile Thr Ser Val Ile Gly Ala Asp Tyr Phe Asp Tyr Trp
            100                 105                 110
Gly Gln Gly Thr Thr Leu Thr Val Ser Ser Ala Ser Thr Lys Gly Pro
        115                 120                 125
Ser Val Phe Pro Leu Ala Pro Cys Ser Arg Ser Thr Ser Glu Ser Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190
Val Pro Ser Ser Ser Leu Gly Thr Lys Thr Tyr Thr Cys Asn Val Asp
        195                 200                 205
His Lys Pro Ser Asn Thr Lys Val Asp Lys Arg Val Glu Ser Lys Tyr
    210                 215                 220
Gly Pro Pro Cys Pro Pro Cys Pro Ala Pro Glu Ala Ala Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser Gln Glu Asp
            260                 265                 270
Pro Glu Val Gln Phe Asn Trp Tyr Val Asp Gly Val Glu Val His Asn
        275                 280                 285
Ala Lys Thr Lys Pro Arg Glu Glu Gln Phe Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Gly Leu Pro Ser Ser Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Gln Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Arg Leu Thr Val Asp Lys
                405                 410                 415
Ser Arg Trp Gln Glu Gly Asn Val Phe Ser Cys Ser Val Met His Glu
            420                 425                 430
Ala Leu His Asn His Tyr Thr Gln Lys Ser Leu Ser Leu Ser Leu Gly
        435                 440                 445
Lys

<210>  83
<211>  214
<212>  PRT
<213>  Artificial Sequence

<220>
<221>  CHAIN
<223>  ch1808 light chain

<400>  83
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Asn Tyr
            20                  25                  30
Leu Asn Trp Tyr Gln Lys Lys Pro Asp Gly Thr Val Lys Leu Leu Ile
        35                  40                  45
Tyr Tyr Ser Ser Thr Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Phe Pro Trp
                85                  90                  95
Thr Phe Gly Gly Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly

            115                     120                     125
Thr Ala Ser Val Val Cys Leu Leu Asn Asn Phe Tyr Pro Arg Glu Ala
    130                     135                     140
Lys Val Gln Trp Lys Val Asp Asn Ala Leu Gln Ser Gly Asn Ser Gln
145                     150                     155                     160
Glu Ser Val Thr Glu Gln Asp Ser Lys Asp Ser Thr Tyr Ser Leu Ser
                165                     170                     175
Ser Thr Leu Thr Leu Ser Lys Ala Asp Tyr Glu Lys His Lys Val Tyr
            180                     185                     190
Ala Cys Glu Val Thr His Gln Gly Leu Ser Ser Pro Val Thr Lys Ser
        195                     200                     205
Phe Asn Arg Gly Glu Cys
    210

<210>  84
<211>  450
<212>  PRT
<213>  Artificial Sequence

<220>
<221>  CHAIN
<223>  hu1810-12 heavy chain

<400>  84
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Asn Tyr
            20                  25                  30
Ala Ile Ser Trp Val Lys Gln Ala Pro Gly Gln Gly Leu Glu Trp Ile
        35                  40                  45
Gly Glu Ile Tyr Pro Thr Ser Gly Asn Thr Tyr Tyr Asn Glu Lys Phe
    50                  55                  60
Lys Gly Lys Ala Thr Leu Thr Ala Asp Arg Ser Thr Ser Thr Met Tyr
65                  70                  75                  80
Met Glu Leu Ser Ser Leu Arg Ser Glu Asp Thr Ala Val Tyr Tyr Cys
                85                  90                  95
Ala Ser Gly Val Ile Thr Thr Val Val Ser Thr Asp Tyr Phe Asp Tyr
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Cys Ser Arg Ser Thr Ser Glu Ser
    130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
            180                 185                 190
Thr Val Pro Ser Ser Ser Leu Gly Thr Lys Thr Tyr Thr Cys Asn Val
        195                 200                 205
Asp His Lys Pro Ser Asn Thr Lys Val Asp Lys Arg Val Glu Ser Lys
    210                 215                 220
Tyr Gly Pro Pro Cys Pro Pro Cys Pro Ala Pro Glu Ala Ala Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser Gln Glu
            260                 265                 270
Asp Pro Glu Val Gln Phe Asn Trp Tyr Val Asp Gly Val Glu Val His
        275                 280                 285
Asn Ala Lys Thr Lys Pro Arg Glu Glu Gln Phe Asn Ser Thr Tyr Arg

290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Gly Leu Pro Ser Ser Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Gln Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Arg Leu Thr Val Asp
                405                 410                 415
Lys Ser Arg Trp Gln Glu Gly Asn Val Phe Ser Cys Ser Val Met His
            420                 425                 430
Glu Ala Leu His Asn His Tyr Thr Gln Lys Ser Leu Ser Leu Ser Leu
        435                 440                 445
Gly Lys
    450

<210>  85
<211>  214
<212>  PRT
<213>  Artificial Sequence

<220>
<221>  CHAIN
<223>  hu1810-12 light chain


<400>  85
Asp Ile Gln Met Thr Gln Ser Pro Ser Ser Leu Ser Ala Ser Val Gly
1               5                   10                  15
Asp Arg Val Thr Ile Thr Cys Arg Ala Ser Leu Asp Ile Ser Asn Tyr
            20                  25                  30
Leu Asn Trp Tyr Gln Leu Lys Pro Gly Lys Ala Val Lys Leu Leu Ile
        35                  40                  45
Tyr Tyr Thr Ser Thr Leu His Ser Gly Val Ser Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Gln Gln Gly Asn Met Val Pro Tyr
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  86

<211>  452
<212>  PRT
<213>  Artificial Sequence

<220>
<221>  CHAIN
<223>  ch1817 heavy chain

<400>  86
Glu Val Gln Leu Val Glu Ser Gly Gly Asp Leu Val Gln Pro Gly Arg
1               5                   10                  15
Ser Met Lys Leu Ser Cys Ala Ala Ser Gly Phe Thr Phe Ser Asn Tyr
            20                  25                  30
Tyr Met Ala Trp Val Arg Gln Ala Pro Thr Lys Gly Leu Glu Trp Val
        35                  40                  45
Ala Ser Ile Ser Thr Gly Gly Val Asn Thr Tyr Tyr Arg Asp Ser Val
    50                  55                  60
Lys Gly Arg Phe Thr Ile Ser Arg Asp Asn Ala Lys Asn Asn Leu Tyr
65                  70                  75                  80
Leu Gln Met Asp Ser Leu Arg Ser Glu Glu Thr Ala Thr Tyr Tyr Cys
                85                  90                  95
Ala Arg His Thr Thr Ala Asp Tyr Phe Tyr Gly Ile Tyr Phe Ala Leu
            100                 105                 110
Asp Ala Trp Gly Gln Gly Thr Ser Val Thr Val Ser Ser Ala Ser Thr
        115                 120                 125
Lys Gly Pro Ser Val Phe Pro Leu Ala Pro Cys Ser Arg Ser Thr Ser
    130                 135                 140
Glu Ser Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Lys Thr Tyr Thr Cys
        195                 200                 205
Asn Val Asp His Lys Pro Ser Asn Thr Lys Val Asp Lys Arg Val Glu
    210                 215                 220
Ser Lys Tyr Gly Pro Pro Cys Pro Pro Cys Pro Ala Pro Glu Ala Ala
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
Gln Glu Asp Pro Glu Val Gln Phe Asn Trp Tyr Val Asp Gly Val Glu
        275                 280                 285
Val His Asn Ala Lys Thr Lys Pro Arg Glu Glu Gln Phe Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Gly Leu Pro Ser Ser
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Gln Glu Glu Met Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Arg Leu Thr
                405                 410                 415
Val Asp Lys Ser Arg Trp Gln Glu Gly Asn Val Phe Ser Cys Ser Val

420                 425                 430
Met His Glu Ala Leu His Asn His Tyr Thr Gln Lys Ser Leu Ser Leu
        435                 440                 445
Ser Leu Gly Lys
    450

<210>  87
<211>  218
<212>  PRT
<213>  Artificial Sequence

<220>
<221>  CHAIN
<223>  ch1817 light chain


<400>  87
Gln Phe Thr Leu Thr Gln Pro Lys Ser Val Ser Gly Ser Leu Arg Ser
1               5                   10                  15
Thr Ile Thr Ile Pro Cys Glu Arg Ser Ser Gly Asp Ile Gly Asp Ser
            20                  25                  30
Tyr Val Asn Trp Tyr Gln Gln His Leu Gly Arg Pro Pro Leu Asn Val
        35                  40                  45
Ile Tyr Ala Asp Val Gln Arg Pro Ser Glu Val Ser Asp Arg Phe Ser
    50                  55                  60
Gly Ser Ile Asp Ser Ser Ser Asn Ser Ala Ser Leu Thr Ile Thr Asn
65                  70                  75                  80
Leu Gln Met Asp Asp Glu Ala Asp Tyr Phe Cys Gln Ser Tyr Asp Thr
                85                  90                  95
Asn Ile Asp Ile Ile Phe Gly Gly Gly Thr Lys Leu Thr Val Leu Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  88
<211>  452
<212>  PRT
<213>  Artificial Sequence

<220>
<221>  CHAIN
<223>  ch1822 heavy chain


<400>  88
Glu Val Arg Leu Val Glu Ser Gly Gly Asp Phe Val Gln Pro Gly Arg
1               5                   10                  15
Ser Val Lys Leu Ser Cys Ala Ala Ser Gly Phe Thr Phe Ser Asn Tyr
            20                  25                  30
Tyr Met Ala Trp Val Arg Gln Ala Pro Thr Lys Gly Leu Glu Trp Val
        35                  40                  45

Gly Ser Ile Ser Thr Gly Gly Val Asn Thr Tyr Tyr Arg Asp Ser Val
    50                  55                  60
Lys Gly Arg Phe Thr Ile Ser Arg Asp Asn Ala Glu Ser Thr Leu Tyr
65                  70                  75                  80
Leu Gln Met Asp Ser Leu Arg Ser Glu Glu Thr Ala Thr Tyr Tyr Cys
                85                  90                  95
Ala Arg His Thr Thr Pro Asp Tyr His Tyr Gly Ile Tyr Phe Ala Met
            100                 105                 110
Asp Ala Trp Gly Gln Gly Thr Ser Val Thr Val Ser Ser Ala Ser Thr
        115                 120                 125
Lys Gly Pro Ser Val Phe Pro Leu Ala Pro Cys Ser Arg Ser Thr Ser
    130                 135                 140
Glu Ser Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser
            180                 185                 190
Val Val Thr Val Pro Ser Ser Ser Leu Gly Thr Lys Thr Tyr Thr Cys
        195                 200                 205
Asn Val Asp His Lys Pro Ser Asn Thr Lys Val Asp Lys Arg Val Glu
    210                 215                 220
Ser Lys Tyr Gly Pro Pro Cys Pro Pro Cys Pro Ala Pro Glu Ala Ala
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
Gln Glu Asp Pro Glu Val Gln Phe Asn Trp Tyr Val Asp Gly Val Glu
        275                 280                 285
Val His Asn Ala Lys Thr Lys Pro Arg Glu Glu Gln Phe Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Gly Leu Pro Ser Ser
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Gln Glu Glu Met Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Arg Leu Thr
                405                 410                 415
Val Asp Lys Ser Arg Trp Gln Glu Gly Asn Val Phe Ser Cys Ser Val
            420                 425                 430
Met His Glu Ala Leu His Asn His Tyr Thr Gln Lys Ser Leu Ser Leu
        435                 440                 445
Ser Leu Gly Lys
    450

<210>  89
<211>  218
<212>  PRT
<213>  Artificial Sequence

<220>
<221>  CHAIN
<223>  ch1822 light chain

<400>  89
Gln Val Thr Leu Thr Gln Pro Lys Ser Val Ser Gly Ser Leu Arg Ser
1               5                   10                  15
Thr Ile Thr Ile Pro Cys Glu Arg Ser Ser Gly Asp Ile Gly Glu Ser
            20                  25                  30
Tyr Val Asn Trp Tyr Gln Gln His Leu Gly Arg Pro Pro Ile Asn Val
        35                  40                  45
Ile Tyr Ala Asp Asp Gln Arg Pro Ser Glu Val Ser Asp Arg Phe Ser
    50                  55                  60
Gly Ser Ile Asp Ser Ser Ser Asn Ser Ala Ser Leu Thr Ile Thr Asn
65                  70                  75                  80
Leu Gln Val Asp Asp Glu Ala Asp Tyr Phe Cys Gln Ser Tyr Asp Ser
                85                  90                  95
Ser Ile Asp Ile Phe Phe Gly Gly Gly Thr Lys Leu Thr Val Leu Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  90
<211>  275
<212>  PRT
<213>  Artificial Sequence

<220>
<221>  PEPTIDE
<223>  Dulaglutide

<400>  90
His Gly Glu Gly Thr Phe Thr Ser Asp Val Ser Ser Tyr Leu Glu Glu
1               5                   10                  15
Gln Ala Ala Lys Glu Phe Ile Ala Trp Leu Val Lys Gly Gly Gly Gly
            20                  25                  30
Gly Gly Gly Ser Gly Gly Gly Gly Ser Gly Gly Gly Gly Ser Ala Glu
        35                  40                  45
Ser Lys Tyr Gly Pro Pro Cys Pro Pro Cys Pro Ala Pro Glu Ala Ala
    50                  55                  60
Gly Gly Pro Ser Val Phe Leu Phe Pro Pro Lys Pro Lys Asp Thr Leu
65                  70                  75                  80
Met Ile Ser Arg Thr Pro Glu Val Thr Cys Val Val Val Asp Val Ser
                85                  90                  95
Gln Glu Asp Pro Glu Val Gln Phe Asn Trp Tyr Val Asp Gly Val Glu
            100                 105                 110
Val His Asn Ala Lys Thr Lys Pro Arg Glu Glu Gln Phe Asn Ser Thr
        115                 120                 125
Tyr Arg Val Val Ser Val Leu Thr Val Leu His Gln Asp Trp Leu Asn
    130                 135                 140
Gly Lys Glu Tyr Lys Cys Lys Val Ser Asn Lys Gly Leu Pro Ser Ser
145                 150                 155                 160
Ile Glu Lys Thr Ile Ser Lys Ala Lys Gly Gln Pro Arg Glu Pro Gln
                165                 170                 175

Val Tyr Thr Leu Pro Pro Ser Gln Glu Glu Met Thr Lys Asn Gln Val
                180                 185                 190
Ser Leu Thr Cys Leu Val Lys Gly Phe Tyr Pro Ser Asp Ile Ala Val
            195                 200                 205
Glu Trp Glu Ser Asn Gly Gln Pro Glu Asn Asn Tyr Lys Thr Thr Pro
    210                 215                 220
Pro Val Leu Asp Ser Asp Gly Ser Phe Phe Leu Tyr Ser Arg Leu Thr
225                 230                 235                 240
Val Asp Lys Ser Arg Trp Gln Glu Gly Asn Val Phe Ser Cys Ser Val
                245                 250                 255
Met His Glu Ala Leu His Asn His Tyr Thr Gln Lys Ser Leu Ser Leu
                260                 265                 270
Ser Leu Gly
            275

<210>  91
<211>  31
<212>  PRT
<213>  Artificial Sequence

<220>
<221>  PEPTIDE
<223>  GLP-1A


<400>  91
His Gly Glu Gly Thr Phe Thr Ser Asp Val Ser Ser Tyr Leu Glu Glu
1               5                   10                  15
Gln Ala Ala Lys Glu Phe Ile Ala Trp Leu Val Lys Gly Gly Gly
                20                  25                  30

<210>  92
<211>  31
<212>  PRT
<213>  Artificial Sequence

<220>
<221>  PEPTIDE
<223>  GLP-1B


<400>  92
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Lys Gly Gly Gly
                20                  25                  30

<210>  93
<211>  31
<212>  PRT
<213>  Artificial Sequence

<220>
<221>  PEPTIDE
<223>  GLP-1C


<400>  93
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Lys Gly Arg Gly
                20                  25                  30

<210>  94
<211>  31
<212>  PRT
<213>  Artificial Sequence

<220>
<221>  PEPTIDE
<223>  GLP-1D

<400>  94
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly
            20                  25                  30

<210>  95
<211>  31
<212>  PRT
<213>  Artificial Sequence

<220>
<221>  PEPTIDE
<223>  GLP-1E

<400>  95
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Arg Gly
            20                  25                  30

<210>  96
<211>  31
<212>  PRT
<213>  Artificial Sequence

<220>
<221>  PEPTIDE
<223>  GLP-1F

<400>  96
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Arg Gly Gly Gly
            20                  25                  30

<210>  97
<211>  31
<212>  PRT
<213>  Artificial Sequence

<220>
<221>  PEPTIDE
<223>  GLP-1G

<400>  97
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Arg Gly Gly Gly
            20                  25                  30

<210>  98
<211>  31
<212>  PRT
<213>  Artificial Sequence

<220>
<221>  PEPTIDE
<223>  GLP-1H


<400>  98
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Arg Gly Arg Gly
            20                  25                  30

<210>  99
<211>  31
<212>  PRT
<213>  Artificial Sequence

<220>
<221>  PEPTIDE
<223>  GLP-1J


<400>  99
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Arg Gly Arg Gly
            20                  25                  30

<210>  100
<211>  495
<212>  PRT
<213>  Artificial Sequence

<220>
<221>  CHAIN
<223>  hu1803-9A containing heavy chain part


<400>  100
His Gly Glu Gly Thr Phe Thr Ser Asp Val Ser Ser Tyr Leu Glu Glu
1               5                   10                  15
Gln Ala Ala Lys Glu Phe Ile Ala Trp Leu Val Lys Gly Gly Gly Gly
            20                  25                  30
Gly Gly Gly Ser Gly Gly Gly Gly Ser Gly Gly Gly Gly Ser Glu Val
        35                  40                  45
Gln Leu Val Gln Ser Gly Ala Glu Val Lys Lys Pro Gly Ala Ser Val
    50                  55                  60
Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125
Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln

145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  101
<211>  495
<212>  PRT
<213>  Artificial Sequence

<220>
<221>  CHAIN
<223>  hu1803-9B containing heavy chain part


<400>  101
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Lys Gly Gly Gly Gly
            20                  25                  30
Gly Gly Gly Ser Gly Gly Gly Gly Ser Gly Gly Gly Gly Ser Glu Val
        35                  40                  45
Gln Leu Val Gln Ser Gly Ala Glu Val Lys Lys Pro Gly Ala Ser Val
    50                  55                  60

Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125
Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln
145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  102
<211>  496
<212>  PRT
<213>  Artificial Sequence

<220>
<221>  CHAIN
<223>  hu1803-9C containing heavy chain part

<400> 102
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Lys Gly Arg Gly Gly
            20                  25                  30
Gly Gly Gly Gly Ser Gly Gly Gly Gly Ser Gly Gly Gly Gly Ser Glu
        35                  40                  45
Val Gln Leu Val Gln Ser Gly Ala Glu Val Lys Lys Pro Gly Ala Ser
    50                  55                  60
Val Lys Val Ser Cys Lys Ala Ser Gly Tyr Thr Phe Thr Asp Tyr Trp
65                  70                  75                  80
Ile Glu Trp Val Arg Gln Ala Pro Gly Gln Gly Leu Glu Trp Met Gly
                85                  90                  95
Glu Ile Leu Pro Gly Ser Thr Tyr Thr Asn Tyr Asn Glu Lys Phe Lys
            100                 105                 110
Gly Arg Val Thr Met Thr Arg Asp Thr Ser Thr Ser Thr Val Tyr Met
        115                 120                 125
Glu Leu Ser Ser Leu Arg Ser Glu Asp Thr Ala Val Tyr Tyr Cys Ala
    130                 135                 140
Arg Gly Leu Ser Thr Leu Met Ala Val Asp Tyr Phe Asp Tyr Trp Gly
145                 150                 155                 160
Gln Gly Thr Thr Val Thr Val Ser Ser Ala Ser Thr Lys Gly Pro Ser
                165                 170                 175
Val Phe Pro Leu Ala Pro Cys Ser Arg Ser Thr Ser Glu Ser Thr Ala
            180                 185                 190
Ala Leu Gly Cys Leu Val Lys Asp Tyr Phe Pro Glu Pro Val Thr Val
        195                 200                 205
Ser Trp Asn Ser Gly Ala Leu Thr Ser Gly Val His Thr Phe Pro Ala
    210                 215                 220
Val Leu Gln Ser Ser Gly Leu Tyr Ser Leu Ser Ser Val Val Thr Val
225                 230                 235                 240
Pro Ser Ser Ser Leu Gly Thr Lys Thr Tyr Thr Cys Asn Val Asp His
                245                 250                 255
Lys Pro Ser Asn Thr Lys Val Asp Lys Arg Val Glu Ser Lys Tyr Gly
            260                 265                 270
Pro Pro Cys Pro Pro Cys Pro Ala Pro Glu Ala Ala Gly Gly Pro Ser
        275                 280                 285
Val Phe Leu Phe Pro Pro Lys Pro Lys Asp Thr Leu Met Ile Ser Arg
    290                 295                 300
Thr Pro Glu Val Thr Cys Val Val Val Asp Val Ser Gln Glu Asp Pro
305                 310                 315                 320
Glu Val Gln Phe Asn Trp Tyr Val Asp Gly Val Glu Val His Asn Ala
                325                 330                 335
Lys Thr Lys Pro Arg Glu Glu Gln Phe Asn Ser Thr Tyr Arg Val Val
            340                 345                 350
Ser Val Leu Thr Val Leu His Gln Asp Trp Leu Asn Gly Lys Glu Tyr
        355                 360                 365
Lys Cys Lys Val Ser Asn Lys Gly Leu Pro Ser Ser Ile Glu Lys Thr
    370                 375                 380
Ile Ser Lys Ala Lys Gly Gln Pro Arg Glu Pro Gln Val Tyr Thr Leu
385                 390                 395                 400
Pro Pro Ser Gln Glu Glu Met Thr Lys Asn Gln Val Ser Leu Thr Cys
                405                 410                 415
Leu Val Lys Gly Phe Tyr Pro Ser Asp Ile Ala Val Glu Trp Glu Ser
            420                 425                 430
Asn Gly Gln Pro Glu Asn Asn Tyr Lys Thr Thr Pro Pro Val Leu Asp
        435                 440                 445
Ser Asp Gly Ser Phe Phe Leu Tyr Ser Arg Leu Thr Val Asp Lys Ser
    450                 455                 460
Arg Trp Gln Glu Gly Asn Val Phe Ser Cys Ser Val Met His Glu Ala
465                 470                 475                 480

Leu His Asn His Tyr Thr Gln Lys Ser Leu Ser Leu Ser Leu Gly Lys
                485                 490                 495

<210>  103
<211>  495
<212>  PRT
<213>  Artificial Sequence

<220>
<221>  CHAIN
<223>  hu1803-9D containing heavy chain part


<400>  103
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly Gly
            20                  25                  30
Gly Gly Gly Ser Gly Gly Gly Gly Ser Gly Gly Gly Gly Ser Glu Val
        35                  40                  45
Gln Leu Val Gln Ser Gly Ala Glu Val Lys Lys Pro Gly Ala Ser Val
    50                  55                  60
Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125
Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln
145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro

385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  104
<211>  496
<212>  PRT
<213>  Artificial Sequence

<220>
<221>  CHAIN
<223>  hu1803-9E containing heavy chain part


<400>  104
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Arg Gly Gly
            20                  25                  30
Gly Gly Gly Gly Ser Gly Gly Gly Gly Ser Gly Gly Gly Gly Ser Glu
        35                  40                  45
Val Gln Leu Val Gln Ser Gly Ala Glu Val Lys Lys Pro Gly Ala Ser
    50                  55                  60
Val Lys Val Ser Cys Lys Ala Ser Gly Tyr Thr Phe Thr Asp Tyr Trp
65                  70                  75                  80
Ile Glu Trp Val Arg Gln Ala Pro Gly Gln Gly Leu Glu Trp Met Gly
                85                  90                  95
Glu Ile Leu Pro Gly Ser Thr Tyr Thr Asn Tyr Asn Glu Lys Phe Lys
            100                 105                 110
Gly Arg Val Thr Met Thr Arg Asp Thr Ser Thr Ser Thr Val Tyr Met
        115                 120                 125
Glu Leu Ser Ser Leu Arg Ser Glu Asp Thr Ala Val Tyr Tyr Cys Ala
    130                 135                 140
Arg Gly Leu Ser Thr Leu Met Ala Val Asp Tyr Phe Asp Tyr Trp Gly
145                 150                 155                 160
Gln Gly Thr Thr Val Thr Val Ser Ser Ala Ser Thr Lys Gly Pro Ser
                165                 170                 175
Val Phe Pro Leu Ala Pro Cys Ser Arg Ser Thr Ser Glu Ser Thr Ala
            180                 185                 190
Ala Leu Gly Cys Leu Val Lys Asp Tyr Phe Pro Glu Pro Val Thr Val
        195                 200                 205
Ser Trp Asn Ser Gly Ala Leu Thr Ser Gly Val His Thr Phe Pro Ala
    210                 215                 220
Val Leu Gln Ser Ser Gly Leu Tyr Ser Leu Ser Ser Val Val Thr Val
225                 230                 235                 240
Pro Ser Ser Ser Leu Gly Thr Lys Thr Tyr Thr Cys Asn Val Asp His
                245                 250                 255
Lys Pro Ser Asn Thr Lys Val Asp Lys Arg Val Glu Ser Lys Tyr Gly
            260                 265                 270
Pro Pro Cys Pro Pro Cys Pro Ala Pro Glu Ala Ala Gly Gly Pro Ser
        275                 280                 285
Val Phe Leu Phe Pro Pro Lys Pro Lys Asp Thr Leu Met Ile Ser Arg
    290                 295                 300

Thr Pro Glu Val Thr Cys Val Val Val Asp Val Ser Gln Glu Asp Pro
305                 310                 315                 320
Glu Val Gln Phe Asn Trp Tyr Val Asp Gly Val Glu Val His Asn Ala
                325                 330                 335
Lys Thr Lys Pro Arg Glu Glu Gln Phe Asn Ser Thr Tyr Arg Val Val
            340                 345                 350
Ser Val Leu Thr Val Leu His Gln Asp Trp Leu Asn Gly Lys Glu Tyr
        355                 360                 365
Lys Cys Lys Val Ser Asn Lys Gly Leu Pro Ser Ser Ile Glu Lys Thr
    370                 375                 380
Ile Ser Lys Ala Lys Gly Gln Pro Arg Glu Pro Gln Val Tyr Thr Leu
385                 390                 395                 400
Pro Pro Ser Gln Glu Glu Met Thr Lys Asn Gln Val Ser Leu Thr Cys
                405                 410                 415
Leu Val Lys Gly Phe Tyr Pro Ser Asp Ile Ala Val Glu Trp Glu Ser
            420                 425                 430
Asn Gly Gln Pro Glu Asn Asn Tyr Lys Thr Thr Pro Pro Val Leu Asp
        435                 440                 445
Ser Asp Gly Ser Phe Phe Leu Tyr Ser Arg Leu Thr Val Asp Lys Ser
    450                 455                 460
Arg Trp Gln Glu Gly Asn Val Phe Ser Cys Ser Val Met His Glu Ala
465                 470                 475                 480
Leu His Asn His Tyr Thr Gln Lys Ser Leu Ser Leu Ser Leu Gly Lys
                485                 490                 495

<210>  105
<211>  495
<212>  PRT
<213>  Artificial Sequence

<220>
<221>  CHAIN
<223>  hu1803-9F containing heavy chain part


<400>  105
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Arg Gly Gly Gly Gly
            20                  25                  30
Gly Gly Gly Ser Gly Gly Gly Gly Ser Gly Gly Gly Gly Ser Glu Val
        35                  40                  45
Gln Leu Val Gln Ser Gly Ala Glu Val Lys Lys Pro Gly Ala Ser Val
    50                  55                  60
Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125
Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln
145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val

210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  106
<211>  495
<212>  PRT
<213>  Artificial Sequence

<220>
<221>  CHAIN
<223>  hu1803-9G containing heavy chain part


<400>  106
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Arg Gly Gly Gly Gly
            20                  25                  30
Gly Gly Gly Ser Gly Gly Gly Gly Ser Gly Gly Gly Gly Ser Glu Val
        35                  40                  45
Gln Leu Val Gln Ser Gly Ala Glu Val Lys Lys Pro Gly Ala Ser Val
    50                  55                  60
Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125

Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln
145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210> 107
<211> 495
<212> PRT
<213> Artificial Sequence

<220>
<221> CHAIN
<223> hu1803-9H containing heavy chain part


<400> 107
His Gly Glu Gly Thr Phe Thr Ser Asp Val Ser Ser Tyr Leu Glu Glu
1               5                   10                  15
Glu Ala Ala Lys Glu Phe Ile Ala Trp Leu Val Arg Gly Arg Gly Gly
            20                  25                  30
Gly Gly Gly Ser Gly Gly Gly Gly Ser Gly Gly Gly Gly Ser Glu Val

35                  40                  45
Gln Leu Val Gln Ser Gly Ala Glu Val Lys Lys Pro Gly Ala Ser Val
    50                  55                  60
Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125
Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln
145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  108
<211>  495
<212>  PRT
<213>  Artificial Sequence

<220>
<221>  CHAIN
<223>  hu1803-9J containing heavy chain part


<400>  108
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Arg Gly Arg Gly Gly
            20                  25                  30
Gly Gly Gly Ser Gly Gly Gly Gly Ser Gly Gly Gly Gly Ser Glu Val
        35                  40                  45
Gln Leu Val Gln Ser Gly Ala Glu Val Lys Lys Pro Gly Ala Ser Val
    50                  55                  60
Lys Val Ser Cys Lys Ala Ser Gly Tyr Thr Phe Thr Asp Tyr Trp Ile
65                  70                  75                  80
Glu Trp Val Arg Gln Ala Pro Gly Gln Gly Leu Glu Trp Met Gly Glu
                85                  90                  95
Ile Leu Pro Gly Ser Thr Tyr Thr Asn Tyr Asn Glu Lys Phe Lys Gly
            100                 105                 110
Arg Val Thr Met Thr Arg Asp Thr Ser Thr Ser Thr Val Tyr Met Glu
        115                 120                 125
Leu Ser Ser Leu Arg Ser Glu Asp Thr Ala Val Tyr Tyr Cys Ala Arg
    130                 135                 140
Gly Leu Ser Thr Leu Met Ala Val Asp Tyr Phe Asp Tyr Trp Gly Gln
145                 150                 155                 160
Gly Thr Thr Val Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val
        275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg

450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  109
<211>  493
<212>  PRT
<213>  Artificial Sequence

<220>
<221>  CHAIN
<223>  ch1805-D containing heavy chain part


<400>  109
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly Gly
            20                  25                  30
Gly Gly Gly Ser Gly Gly Gly Gly Ser Gly Gly Gly Gly Ser Glu Val
        35                  40                  45
Gln Leu Gln Gln Ser Gly Pro Glu Leu Val Lys Pro Gly Ala Ser Val
    50                  55                  60
Lys Ile Pro Cys Lys Thr Ser Gly Tyr Thr Phe Thr Asp Tyr Asn Met
65                  70                  75                  80
Asp Trp Val Lys Gln Ser His Gly Arg Ser Leu Glu Trp Ile Gly Ser
                85                  90                  95
Ile Asp Pro Asp Asn Gly Gly Thr Ile Tyr Asn Gln Lys Phe Lys Gly
            100                 105                 110
Lys Ala Thr Leu Thr Val Asp Lys Ser Ser Ser Thr Ala Tyr Met Glu
        115                 120                 125
Leu Arg Ser Leu Thr Ser Glu Asp Thr Ala Val Tyr Tyr Cys Thr Arg
    130                 135                 140
Asp Tyr Tyr Gly Ser Ser Ser Trp Phe Ala Tyr Trp Gly Gln Gly Thr
145                 150                 155                 160
Leu Val Thr Val Ser Ala Ala Ser Thr Lys Gly Pro Ser Val Phe Pro
                165                 170                 175
Leu Ala Pro Cys Ser Arg Ser Thr Ser Glu Ser Thr Ala Ala Leu Gly
            180                 185                 190
Cys Leu Val Lys Asp Tyr Phe Pro Glu Pro Val Thr Val Ser Trp Asn
        195                 200                 205
Ser Gly Ala Leu Thr Ser Gly Val His Thr Phe Pro Ala Val Leu Gln
    210                 215                 220
Ser Ser Gly Leu Tyr Ser Leu Ser Ser Val Val Thr Val Pro Ser Ser
225                 230                 235                 240
Ser Leu Gly Thr Lys Thr Tyr Thr Cys Asn Val Asp His Lys Pro Ser
                245                 250                 255
Asn Thr Lys Val Asp Lys Arg Val Glu Ser Lys Tyr Gly Pro Pro Cys
            260                 265                 270
Pro Pro Cys Pro Ala Pro Glu Ala Ala Gly Gly Pro Ser Val Phe Leu
        275                 280                 285
Phe Pro Pro Lys Pro Lys Asp Thr Leu Met Ile Ser Arg Thr Pro Glu
    290                 295                 300
Val Thr Cys Val Val Val Asp Val Ser Gln Glu Asp Pro Glu Val Gln
305                 310                 315                 320
Phe Asn Trp Tyr Val Asp Gly Val Glu Val His Asn Ala Lys Thr Lys
                325                 330                 335
Pro Arg Glu Glu Gln Phe Asn Ser Thr Tyr Arg Val Val Ser Val Leu
            340                 345                 350
Thr Val Leu His Gln Asp Trp Leu Asn Gly Lys Glu Tyr Lys Cys Lys
        355                 360                 365

Val Ser Asn Lys Gly Leu Pro Ser Ser Ile Glu Lys Thr Ile Ser Lys
    370                 375                 380
Ala Lys Gly Gln Pro Arg Glu Pro Gln Val Tyr Thr Leu Pro Pro Ser
385                 390                 395                 400
Gln Glu Glu Met Thr Lys Asn Gln Val Ser Leu Thr Cys Leu Val Lys
                405                 410                 415
Gly Phe Tyr Pro Ser Asp Ile Ala Val Glu Trp Glu Ser Asn Gly Gln
            420                 425                 430
Pro Glu Asn Asn Tyr Lys Thr Thr Pro Pro Val Leu Asp Ser Asp Gly
        435                 440                 445
Ser Phe Phe Leu Tyr Ser Arg Leu Thr Val Asp Lys Ser Arg Trp Gln
    450                 455                 460
Glu Gly Asn Val Phe Ser Cys Ser Val Met His Glu Ala Leu His Asn
465                 470                 475                 480
His Tyr Thr Gln Lys Ser Leu Ser Leu Ser Leu Gly Lys
                485                 490

<210>  110
<211>  495
<212>  PRT
<213>  Artificial Sequence

<220>
<221>  CHAIN
<223>  ch1808-D containing heavy chain part


<400>  110
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly Gly
            20                  25                  30
Gly Gly Gly Ser Gly Gly Gly Gly Ser Gly Gly Gly Gly Ser Gln Val
        35                  40                  45
Gln Leu Gln Gln Ser Gly Ala Glu Leu Ala Arg Pro Gly Ala Ser Val
    50                  55                  60
Lys Leu Ser Cys Lys Ala Ser Gly Asp Thr Phe Thr Thr Asn Gly Ile
65                  70                  75                  80
Ser Trp Val Lys Gln Arg Ile Gly Gln Gly Leu Glu Trp Ile Gly Glu
                85                  90                  95
Ile Tyr Pro Arg Ser Gly Asn Thr Tyr Tyr Asn Glu Asn Phe Lys Gly
            100                 105                 110
Lys Ala Thr Leu Thr Ala Asp Lys Ser Ser Thr Thr Ala Tyr Met Glu
        115                 120                 125
Leu Arg Arg Leu Thr Ser Glu Asp Ser Ala Val Tyr Phe Cys Ala Arg
    130                 135                 140
Ser Ile Thr Ser Val Ile Gly Ala Asp Tyr Phe Asp Tyr Trp Gly Gln
145                 150                 155                 160
Gly Thr Thr Leu Thr Val Ser Ser Ala Ser Thr Lys Gly Pro Ser Val
                165                 170                 175
Phe Pro Leu Ala Pro Cys Ser Arg Ser Thr Ser Glu Ser Thr Ala Ala
            180                 185                 190
Leu Gly Cys Leu Val Lys Asp Tyr Phe Pro Glu Pro Val Thr Val Ser
        195                 200                 205
Trp Asn Ser Gly Ala Leu Thr Ser Gly Val His Thr Phe Pro Ala Val
    210                 215                 220
Leu Gln Ser Ser Gly Leu Tyr Ser Leu Ser Ser Val Val Thr Val Pro
225                 230                 235                 240
Ser Ser Ser Leu Gly Thr Lys Thr Tyr Thr Cys Asn Val Asp His Lys
                245                 250                 255
Pro Ser Asn Thr Lys Val Asp Lys Arg Val Glu Ser Lys Tyr Gly Pro
            260                 265                 270
Pro Cys Pro Pro Cys Pro Ala Pro Glu Ala Ala Gly Gly Pro Ser Val

275                 280                 285
Phe Leu Phe Pro Pro Lys Pro Lys Asp Thr Leu Met Ile Ser Arg Thr
    290                 295                 300
Pro Glu Val Thr Cys Val Val Val Asp Val Ser Gln Glu Asp Pro Glu
305                 310                 315                 320
Val Gln Phe Asn Trp Tyr Val Asp Gly Val Glu Val His Asn Ala Lys
                325                 330                 335
Thr Lys Pro Arg Glu Glu Gln Phe Asn Ser Thr Tyr Arg Val Val Ser
            340                 345                 350
Val Leu Thr Val Leu His Gln Asp Trp Leu Asn Gly Lys Glu Tyr Lys
        355                 360                 365
Cys Lys Val Ser Asn Lys Gly Leu Pro Ser Ser Ile Glu Lys Thr Ile
    370                 375                 380
Ser Lys Ala Lys Gly Gln Pro Arg Glu Pro Gln Val Tyr Thr Leu Pro
385                 390                 395                 400
Pro Ser Gln Glu Glu Met Thr Lys Asn Gln Val Ser Leu Thr Cys Leu
                405                 410                 415
Val Lys Gly Phe Tyr Pro Ser Asp Ile Ala Val Glu Trp Glu Ser Asn
            420                 425                 430
Gly Gln Pro Glu Asn Asn Tyr Lys Thr Thr Pro Pro Val Leu Asp Ser
        435                 440                 445
Asp Gly Ser Phe Phe Leu Tyr Ser Arg Leu Thr Val Asp Lys Ser Arg
    450                 455                 460
Trp Gln Glu Gly Asn Val Phe Ser Cys Ser Val Met His Glu Ala Leu
465                 470                 475                 480
His Asn His Tyr Thr Gln Lys Ser Leu Ser Leu Ser Leu Gly Lys
                485                 490                 495

<210>  111
<211>  496
<212>  PRT
<213>  Artificial Sequence

<220>
<221>  CHAIN
<223>  hu1810-12D containing heavy chain part


<400>  111
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly Gly
            20                  25                  30
Gly Gly Gly Ser Gly Gly Gly Gly Ser Gly Gly Gly Gly Ser Gln Val
        35                  40                  45
Gln Leu Val Gln Ser Gly Ala Glu Val Lys Lys Pro Gly Ser Ser Val
    50                  55                  60
Lys Val Ser Cys Lys Ala Ser Gly Gly Thr Phe Ser Asn Tyr Ala Ile
65                  70                  75                  80
Ser Trp Val Lys Gln Ala Pro Gly Gln Gly Leu Glu Trp Ile Gly Glu
                85                  90                  95
Ile Tyr Pro Thr Ser Gly Asn Thr Tyr Tyr Asn Glu Lys Phe Lys Gly
            100                 105                 110
Lys Ala Thr Leu Thr Ala Asp Arg Ser Thr Ser Thr Met Tyr Met Glu
        115                 120                 125
Leu Ser Ser Leu Arg Ser Glu Asp Thr Ala Val Tyr Tyr Cys Ala Ser
    130                 135                 140
Gly Val Ile Thr Thr Val Val Ser Thr Asp Tyr Phe Asp Tyr Trp Gly
145                 150                 155                 160
Gln Gly Thr Leu Val Thr Val Ser Ser Ala Ser Thr Lys Gly Pro Ser
                165                 170                 175
Val Phe Pro Leu Ala Pro Cys Ser Arg Ser Thr Ser Glu Ser Thr Ala
            180                 185                 190

Ala Leu Gly Cys Leu Val Lys Asp Tyr Phe Pro Glu Pro Val Thr Val
        195                 200                 205
Ser Trp Asn Ser Gly Ala Leu Thr Ser Gly Val His Thr Phe Pro Ala
    210                 215                 220
Val Leu Gln Ser Ser Gly Leu Tyr Ser Leu Ser Ser Val Val Thr Val
225                 230                 235                 240
Pro Ser Ser Ser Leu Gly Thr Lys Thr Tyr Thr Cys Asn Val Asp His
                245                 250                 255
Lys Pro Ser Asn Thr Lys Val Asp Lys Arg Val Glu Ser Lys Tyr Gly
            260                 265                 270
Pro Pro Cys Pro Pro Cys Pro Ala Pro Glu Ala Ala Gly Gly Pro Ser
        275                 280                 285
Val Phe Leu Phe Pro Pro Lys Pro Lys Asp Thr Leu Met Ile Ser Arg
    290                 295                 300
Thr Pro Glu Val Thr Cys Val Val Val Asp Val Ser Gln Glu Asp Pro
305                 310                 315                 320
Glu Val Gln Phe Asn Trp Tyr Val Asp Gly Val Glu Val His Asn Ala
                325                 330                 335
Lys Thr Lys Pro Arg Glu Glu Gln Phe Asn Ser Thr Tyr Arg Val Val
            340                 345                 350
Ser Val Leu Thr Val Leu His Gln Asp Trp Leu Asn Gly Lys Glu Tyr
        355                 360                 365
Lys Cys Lys Val Ser Asn Lys Gly Leu Pro Ser Ser Ile Glu Lys Thr
    370                 375                 380
Ile Ser Lys Ala Lys Gly Gln Pro Arg Glu Pro Gln Val Tyr Thr Leu
385                 390                 395                 400
Pro Pro Ser Gln Glu Glu Met Thr Lys Asn Gln Val Ser Leu Thr Cys
                405                 410                 415
Leu Val Lys Gly Phe Tyr Pro Ser Asp Ile Ala Val Glu Trp Glu Ser
            420                 425                 430
Asn Gly Gln Pro Glu Asn Asn Tyr Lys Thr Thr Pro Pro Val Leu Asp
        435                 440                 445
Ser Asp Gly Ser Phe Phe Leu Tyr Ser Arg Leu Thr Val Asp Lys Ser
    450                 455                 460
Arg Trp Gln Glu Gly Asn Val Phe Ser Cys Ser Val Met His Glu Ala
465                 470                 475                 480
Leu His Asn His Tyr Thr Gln Lys Ser Leu Ser Leu Ser Leu Gly Lys
                485                 490                 495

<210>  112
<211>  498
<212>  PRT
<213>  Artificial Sequence

<220>
<221>  CHAIN
<223>  ch1817-D containing heavy chain part


<400>  112
His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly Gly
            20                  25                  30
Gly Gly Gly Ser Gly Gly Gly Gly Ser Gly Gly Gly Gly Ser Glu Val
        35                  40                  45
Gln Leu Val Glu Ser Gly Gly Asp Leu Val Gln Pro Gly Arg Ser Met
    50                  55                  60
Lys Leu Ser Cys Ala Ala Ser Gly Phe Thr Phe Ser Asn Tyr Tyr Met
65                  70                  75                  80
Ala Trp Val Arg Gln Ala Pro Thr Lys Gly Leu Glu Trp Val Ala Ser
                85                  90                  95
Ile Ser Thr Gly Gly Val Asn Thr Tyr Tyr Arg Asp Ser Val Lys Gly

100                 105                 110
Arg Phe Thr Ile Ser Arg Asp Asn Ala Lys Asn Asn Leu Tyr Leu Gln
        115                 120                 125
Met Asp Ser Leu Arg Ser Glu Glu Thr Ala Thr Tyr Tyr Cys Ala Arg
    130                 135                 140
His Thr Thr Ala Asp Tyr Phe Tyr Gly Ile Tyr Phe Ala Leu Asp Ala
145                 150                 155                 160
Trp Gly Gln Gly Thr Ser Val Thr Val Ser Ser Ala Ser Thr Lys Gly
                165                 170                 175
Pro Ser Val Phe Pro Leu Ala Pro Cys Ser Arg Ser Thr Ser Glu Ser
            180                 185                 190
Thr Ala Ala Leu Gly Cys Leu Val Lys Asp Tyr Phe Pro Glu Pro Val
        195                 200                 205
Thr Val Ser Trp Asn Ser Gly Ala Leu Thr Ser Gly Val His Thr Phe
    210                 215                 220
Pro Ala Val Leu Gln Ser Ser Gly Leu Tyr Ser Leu Ser Ser Val Val
225                 230                 235                 240
Thr Val Pro Ser Ser Ser Leu Gly Thr Lys Thr Tyr Thr Cys Asn Val
                245                 250                 255
Asp His Lys Pro Ser Asn Thr Lys Val Asp Lys Arg Val Glu Ser Lys
            260                 265                 270
Tyr Gly Pro Pro Cys Pro Pro Cys Pro Ala Pro Glu Ala Ala Gly Gly
        275                 280                 285
Pro Ser Val Phe Leu Phe Pro Pro Lys Pro Lys Asp Thr Leu Met Ile
    290                 295                 300
Ser Arg Thr Pro Glu Val Thr Cys Val Val Val Asp Val Ser Gln Glu
305                 310                 315                 320
Asp Pro Glu Val Gln Phe Asn Trp Tyr Val Asp Gly Val Glu Val His
                325                 330                 335
Asn Ala Lys Thr Lys Pro Arg Glu Glu Gln Phe Asn Ser Thr Tyr Arg
            340                 345                 350
Val Val Ser Val Leu Thr Val Leu His Gln Asp Trp Leu Asn Gly Lys
        355                 360                 365
Glu Tyr Lys Cys Lys Val Ser Asn Lys Gly Leu Pro Ser Ser Ile Glu
    370                 375                 380
Lys Thr Ile Ser Lys Ala Lys Gly Gln Pro Arg Glu Pro Gln Val Tyr
385                 390                 395                 400
Thr Leu Pro Pro Ser Gln Glu Glu Met Thr Lys Asn Gln Val Ser Leu
                405                 410                 415
Thr Cys Leu Val Lys Gly Phe Tyr Pro Ser Asp Ile Ala Val Glu Trp
            420                 425                 430
Glu Ser Asn Gly Gln Pro Glu Asn Asn Tyr Lys Thr Thr Pro Pro Val
        435                 440                 445
Leu Asp Ser Asp Gly Ser Phe Phe Leu Tyr Ser Arg Leu Thr Val Asp
    450                 455                 460
Lys Ser Arg Trp Gln Glu Gly Asn Val Phe Ser Cys Ser Val Met His
465                 470                 475                 480
Glu Ala Leu His Asn His Tyr Thr Gln Lys Ser Leu Ser Leu Ser Leu
                485                 490                 495
Gly Lys


<210>  113
<211>  498
<212>  PRT
<213>  Artificial Sequence

<220>
<221>  CHAIN
<223>  ch1822-D containing heavy chain part


<400>  113

His Gly Glu Gly Thr Phe Thr Ser Asp Val Ser Ser Tyr Leu Glu Glu
1               5                   10                  15
Glu Ala Ala Lys Glu Phe Val Ala Trp Leu Val Lys Gly Gly Gly Gly
            20                  25                  30
Gly Gly Gly Ser Gly Gly Gly Gly Ser Gly Gly Gly Gly Ser Glu Val
        35                  40                  45
Gln Leu Val Glu Ser Gly Gly Asp Leu Val Gln Pro Gly Arg Ser Met
    50                  55                  60
Lys Leu Ser Cys Ala Ala Ser Gly Phe Thr Phe Ser Asn Tyr Tyr Met
65                  70                  75                  80
Ala Trp Val Arg Gln Ala Pro Thr Lys Gly Leu Glu Trp Val Ala Ser
                85                  90                  95
Ile Ser Thr Gly Gly Val Asn Thr Tyr Tyr Arg Asp Ser Val Lys Gly
            100                 105                 110
Arg Phe Thr Ile Ser Arg Asp Asn Ala Lys Asn Asn Leu Tyr Leu Gln
        115                 120                 125
Met Asp Ser Leu Arg Ser Glu Glu Thr Ala Thr Tyr Tyr Cys Ala Arg
    130                 135                 140
His Thr Thr Ala Asp Tyr Phe Tyr Gly Ile Tyr Phe Ala Leu Asp Ala
145                 150                 155                 160
Trp Gly Gln Gly Thr Ser Val Thr Val Ser Ser Ala Ser Thr Lys Gly
                165                 170                 175
Pro Ser Val Phe Pro Leu Ala Pro Cys Ser Arg Ser Thr Ser Glu Ser
            180                 185                 190
Thr Ala Ala Leu Gly Cys Leu Val Lys Asp Tyr Phe Pro Glu Pro Val
        195                 200                 205
Thr Val Ser Trp Asn Ser Gly Ala Leu Thr Ser Gly Val His Thr Phe
    210                 215                 220
Pro Ala Val Leu Gln Ser Ser Gly Leu Tyr Ser Leu Ser Ser Val Val
225                 230                 235                 240
Thr Val Pro Ser Ser Ser Leu Gly Thr Lys Thr Tyr Thr Cys Asn Val
                245                 250                 255
Asp His Lys Pro Ser Asn Thr Lys Val Asp Lys Arg Val Glu Ser Lys
            260                 265                 270
Tyr Gly Pro Pro Cys Pro Pro Cys Pro Ala Pro Glu Ala Ala Gly Gly
        275                 280                 285
Pro Ser Val Phe Leu Phe Pro Pro Lys Pro Lys Asp Thr Leu Met Ile
    290                 295                 300
Ser Arg Thr Pro Glu Val Thr Cys Val Val Val Asp Val Ser Gln Glu
305                 310                 315                 320
Asp Pro Glu Val Gln Phe Asn Trp Tyr Val Asp Gly Val Glu Val His
                325                 330                 335
Asn Ala Lys Thr Lys Pro Arg Glu Glu Gln Phe Asn Ser Thr Tyr Arg
            340                 345                 350
Val Val Ser Val Leu Thr Val Leu His Gln Asp Trp Leu Asn Gly Lys
        355                 360                 365
Glu Tyr Lys Cys Lys Val Ser Asn Lys Gly Leu Pro Ser Ser Ile Glu
    370                 375                 380
Lys Thr Ile Ser Lys Ala Lys Gly Gln Pro Arg Glu Pro Gln Val Tyr
385                 390                 395                 400
Thr Leu Pro Pro Ser Gln Glu Glu Met Thr Lys Asn Gln Val Ser Leu
                405                 410                 415
Thr Cys Leu Val Lys Gly Phe Tyr Pro Ser Asp Ile Ala Val Glu Trp
            420                 425                 430
Glu Ser Asn Gly Gln Pro Glu Asn Asn Tyr Lys Thr Thr Pro Pro Val
        435                 440                 445
Leu Asp Ser Asp Gly Ser Phe Phe Leu Tyr Ser Arg Leu Thr Val Asp
    450                 455                 460
Lys Ser Arg Trp Gln Glu Gly Asn Val Phe Ser Cys Ser Val Met His
465                 470                 475                 480
Glu Ala Leu His Asn His Tyr Thr Gln Lys Ser Leu Ser Leu Ser Leu
                485                 490                 495
Gly Lys

## Description

This application claims the priorities of the Patent Application No.201811573634.0 filed on December 21, 2018 and the Patent Application No.201811606887.3 filed on December 27, 2018; both are incorporated herein by reference.

### FIELD OF THE INVENTION

The present disclosure relates to human GCGR antibodies, GLP-1 peptides and mutants thereof, as well as bispecific proteins formed by the GCGR antibody fused to the GLP-1 peptide, and preparation methods and applications thereof.

### BACKGROUND OF THE INVENTION

The descriptions herein only provide background information about the present disclosure, and do not necessarily constitute prior art.

Diabetes (diabetes mellitus, DM) is a metabolic disease characterized by high level of blood glucose due to defect in insulin secretion and/or insulin dysfunction. The onset of the disease is mainly caused by the co-action of insulin and glucagon.

GLP-1 is one of the most important hormones affecting insulin secretion, and both GLP-1 and glucagon are derived from preproinsulin. Preproinsulin is composed of about 158 amino acids and can be cleaved into various peptide chains at different positions. The biologically active GLP-1 in the human body mainly includes two forms, GLP-1 (7-36) amide and GLP-1 (7-37). GLP-1 is secreted by small intestinal L cells, and it lowers the level of blood glucose in an organism by promoting insulin secretion mainly in a glucose concentration-dependent manner, protecting pancreatic islet β cells, and inhibiting glucagon secretion. At the same time, GLP-1 also has effects on inhibiting gastric emptying and reducing appetite. It is clinically useful for the treatment of type II diabetes and obesity. The natural active GLP-1 is easily to be degraded by enzyme DPPIV in an organism due to the very short half-life (less than 2 minutes) and has no clinical value.

The main direction of the research and development of GLP-1 drugs has always been prolonging the half-life. There are currently many commercially available GLP-1 agonists, such as Dulaglutide and Semaglutide. Although the efficacy of GLP-1 has been fully confirmed, though it has many side effects, mainly manifested as gastrointestinal symptoms, hypoglycemia, pancreatitis and kidney damage.

Glucagon has the opposite effect to insulin, and mainly plays a role in raising the level of blood glucose in the organism. Glucagon is a 29-amino acid peptide secreted by pancreatic islet α cells. Glucagon mainly accelerates the glycogenolysis, lipolysis and gluconeogenesis by activating the downstream cAMP/PKA pathway, and thereby increasing the level of blood glucose after binding to the receptor GCGR on the liver cell membrane.

The studies have found that GCGR-knockout mice exhibited a series of phenotypes such as increased GLP-1, decreased glycogen output, increased lipid metabolism, and decreased appetite. GCGR is one of the most popular targets for the treatment of diabetes, but currently the progress in the development of antagonistic drugs against GCGR has been slow. REMD-477, available from REMD Biotherapeutics, is currently the most cutting-edge GCGR monoclonal antibody drug, and is in clinical phase II.

GCGR antibodies have been disclosed in the prior arts, such as CN101589062A, CN101983208A, CN102482350A, CN103314011A, CN105189560A, CN107614695A, US20180273629A1 and WO2013059531A1. However, there is still a need to provide new and highly efficient GCGR antibodies and methods for the treatment of diabetes.

### SUMMARY OF THE INVENTION

The present disclosure provides an anti-GCGR monoclonal antibody or antigen-binding fragment thereof. The antibody or antigen-binding fragment thereof has an ability to bind to human GCGR (or an antigen epitope comprised therein).

In some embodiments, the anti-GCGR monoclonal antibody or antigen-binding fragment thereof comprises a combination of a heavy chain variable region and a light chain variable region selected from the following a) or b):
a) the heavy chain variable region comprising HCDR1, HCDR2 and HCDR3 regions as shown in SEQ ID NOs: 48, 49 and 50, respectively, and the light chain variable region comprising LCDR1, LCDR2 and LCDR3 regions as shown in SEQ ID NOs: 51, 52 and 53, respectively; or
b) the heavy chain variable region comprising HCDR1, HCDR2 and HCDR3 regions as shown in SEQ ID NOs: 38, 39 and 54, respectively, and the light chain variable region comprising LCDR1, LCDR2 and LCDR3 regions as shown in SEQ ID NOs: 55, 56 and 57, respectively.

In some embodiments, the anti-GCGR monoclonal antibody or antigen-binding fragment thereof comprises a combination of a heavy chain variable region and a light chain variable region selected from any one of the following i) to vi):
i) the heavy chain variable region comprising HCDR1, HCDR2 and HCDR3 regions as shown in SEQ ID NOs: 14, 15 and 16, respectively, and the light chain variable region comprising LCDR1, LCDR2 and LCDR3 regions as shown in SEQ ID NOs: 17, 18 and 19, respectively;
ii) the heavy chain variable region comprising HCDR1, HCDR2 and HCDR3 regions as shown in SEQ ID NOs: 20, 21 and 22, respectively, and the light chain variable region comprising LCDR1, LCDR2 and LCDR3 regions as shown in SEQ ID NOs: 23, 24 and 25, respectively;
iii) the heavy chain variable region comprising HCDR1, HCDR2 and HCDR3 regions as shown in SEQ ID NOs: 26, 27 and 28, respectively, and the light chain variable region comprising LCDR1, LCDR2 and LCDR3 regions as shown in SEQ ID NOs: 29, 30 and 31, respectively;
iv) the heavy chain variable region comprising HCDR1, HCDR2 and HCDR3 regions as shown in SEQ ID NOs: 32, 33 and 34, respectively, and the light chain variable region comprising LCDR1, LCDR2 and LCDR3 regions as shown in SEQ ID NOs: 35, 36 and 37, respectively;
v) the heavy chain variable region comprising HCDR1, HCDR2 and HCDR3 regions as shown in SEQ ID NOs: 38, 39 and 40, respectively, and the light chain variable region comprising LCDR1, LCDR2 and LCDR3 regions as shown in SEQ ID NOs: 41, 42 and 43, respectively; or
vi) the heavy chain variable region comprising HCDR1, HCDR2 and HCDR3 regions as shown in SEQ ID NOs: 38, 39 and 44, respectively, and the light chain variable region comprising LCDR1, LCDR2 and LCDR3 regions as shown in SEQ ID NOs: 45, 46 and 47, respectively.

In some embodiments, the anti-GCGR monoclonal antibody or antigen-binding fragment thereof is a murine antibody, chimeric antibody or humanized antibody or antigen-binding fragment thereof.

In some embodiments, the anti-GCGR monoclonal antibody or antigen-binding fragment thereof, the humanized antibody comprises framework region(s) derived from a human antibody or framework region variant thereof, and the framework region variant has at most 10 back mutations based on the light chain framework region of a human antibody, and/or the framework region variant has at most 10, at most 9, at most 8, at most 7, at most 6, at most 5, at most 4, at most 3, at most 2, at most 1 amino acid back mutaion based on the heavy chain framework region of a human antibody.

In some embodiments, the framework region variant comprises:
aa) one or more amino acid back mutation(s) of 42G, 44V, 71Y and 87F comprised in the light chain variable region, and/or one or more amino acid back mutation(s) of 38K, 481, 67A, 69F, 71A, 73P, 78A and 93 comprised in the heavy chain variable region; or
ab) one or more amino acid back mutation(s) of 38L, 44V, 59S, 70E and 71Y comprised in the light chain variable region, and/or one or more amino acid back mutation(s) of 38K, 481, 66K, 67A, 69L, 73R, 78M and 94S comprised in the heavy chain variable region. Further, the position of the back mutation site is determined according to the Kabat numbering criteria.

In some embodiments, the anti-GCGR monoclonal antibody or antigen-binding fragment thereof comprises a heavy chain variable region as shown in any one of SEQ ID NOs: 2, 61, 62, 63 and 64, or having at least 90%, 91%, 92%, 93%, 94%, 95%, 96%, 97%, 98% or 99% sequence identity to any one of SEQ ID NOs: 2, 61, 62, 63 and 64; and/or
a light chain variable region as shown in any one of SEQ ID NOs: 3, 58, 59 and 60, or having at least 90%, 91%, 92%, 93%, 94%, 95%, 96%, 97%, 98% or 99% sequence identity to any one of SEQ ID NOs: 3, 58, 59 and 60.

In some embodiments, the anti-GCGR monoclonal antibody or antigen-binding fragment thereof comprises a heavy chain variable region as shown in SEQ ID NO: 63 and a light chain variable region as shown in SEQ ID NO: 58.

In some embodiments, the anti-GCGR monoclonal antibody or antigen-binding fragment thereof comprises a heavy chain variable region as shown in SEQ ID NO: 4 or having at least 90%, 91%, 92%, 93%, 94%, 95%, 96%, 97%, 98% or 99% sequence identity to SEQ ID NO: 4; and/or
a light chain variable region as shown in SEQ ID NO: 5 or having at least 90%, 91%, 92%, 93%, 94%, 95%, 96%, 97%, 98% or 99% sequence identity to SEQ ID NO:5.

In some embodiments, the anti-GCGR monoclonal antibody or antigen-binding fragment thereof comprises a heavy chain variable region as shown in SEQ ID NO: 6 or having at least 90%, 91%, 92%, 93%, 94%, 95%, 96%, 97%, 98% or 99% sequence identity to SEQ ID NO: 6; and/or
a light chain variable region as shown in SEQ ID NO: 7 or having at least 90%, 91%, 92%, 93%, 94%, 95%, 96%, 97%, 98% or 99% sequence identity to SEQ ID NO:7.

In some embodiments, the anti-GCGR monoclonal antibody or antigen-binding fragment thereof comprises a heavy chain variable region as shown in any one of SEQ ID NOs: 8, 68, 69, 70 and 71 or having at least 90%, 91%, 92%, 93%, 94%, 95%, 96%, 97%, 98% or 99% sequence identity to any one of SEQ ID NOs: 8, 68, 69, 70 and 71; and/or
a light chain variable region as shown in any one of SEQ ID NOs: 9, 65, 66 and 67 or having at least 90%, 91%, 92%, 93%, 94%, 95%, 96%, 97%, 98% or 99% sequence identity to any one of SEQ ID NOs: 9, 65, 66 and 67.

In some embodiments, the anti-GCGR monoclonal antibody or antigen-binding fragment thereof comprises a heavy chain variable region as shown in SEQ ID NO: 71 and a light chain variable region as shown in SEQ ID NO: 67.

In some embodiments, the anti-GCGR monoclonal antibody or antigen-binding fragment thereof comprises a heavy chain variable region as shown in SEQ ID NO: 10 or having at least 90%, 91%, 92%, 93%, 94%, 95%, 96%, 97%, 98% or 99% sequence identity to SEQ ID NO: 10; and/or
a light chain variable region as shown in SEQ ID NO: 11 or having at least 90%, 91%, 92%, 93%, 94%, 95%, 96%, 97%, 98% or 99% sequence identity to SEQ ID NO:11.

In some embodiments, the anti-GCGR monoclonal antibody or antigen-binding fragment thereof comprises a heavy chain variable region as shown in SEQ ID NO: 12 or having at least 90%, 91%, 92%, 93%, 94%, 95%, 96%, 97%, 98% or 99% sequence identity to SEQ ID NO: 12; and/or
a light chain variable region as shown in SEQ ID NO: 13 or having at least 90%, 91%, 92%, 93%, 94%, 95%, 96%, 97%, 98% or 99% sequence identity to SEQ ID NO:13.

In some embodiments of the anti-GCGR monoclonal antibody or antigen-binding fragment thereof, the antibody is a full-length antibody, further comprising antibody constant region(s), specifically, the heavy chain constant region of the antibody constant regions is selected from human IgG1, IgG2, IgG3 and IgG4 constant regions and the conventional variants thereof, and the light chain constant region of the antibody constant regions is selected from human antibody κ and λ chain constant regions and the conventional variants thereof, and more preferably comprising a human antibody heavy chain constant region as shown in SEQ ID NO: 72 and a human light chain constant region as shown in SEQ ID NO:73.

In some embodiments, the anti-GCGR monoclonal antibody or antigen-binding fragment thereof comprises a heavy chain and a light chain, wherein:
the heavy chain is as shown in SEQ ID NO: 74, 76 or 78 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 75 or has at least 85%, 86%, 87%, 88%, 89 %, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 74, 76 or 78 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 77 or has at least 85%, 86%, 87%, 88%, 89%, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 74, 76 or 78 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 79 or has at least 85%, 86%, 87%, 88%, 89%, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 80 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 81 or has at least 85%, 86%, 87%, 88%, 89 %, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 82 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 83 or has at least 85%, 86%, 87%, 88%, 89 %, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 84 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 85 or has at least 85%, 86%, 87%, 88%, 89 %, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 86 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 87 or has at least 85%, 86%, 87%, 88%, 89 %, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain and a light chain, wherein:
the heavy chain is as shown in SEQ ID NO: 88 or has at least 85%, 86%, 87%, 88%, 89%, 90%, 91%, 92%, 93%, 94%, 95%, 96%, 97%, 98% or 99% sequence identity thereto, and the light chain is as shown in SEQ ID NO: 89 or has at least 85%, 86%, 87%, 88%, 89 %, 90%, 91%, 92%, 93%, 94%, 95%, 96%, 97%, 98% or 99% sequence identity thereto.

In some embodiments, the anti-GCGR monoclonal antibody or antigen-binding fragment thereof comprises a heavy chain as shown in SEQ ID NO: 78 and a light chain as shown in SEQ ID NO: 79.

In some embodiments, the anti-GCGR monoclonal antibody or antigen-binding fragment thereof comprises a heavy chain as shown in SEQ ID NO: 84 and a light chain as shown in SEQ ID NO: 85.

In some embodiments, the anti-GCGR monoclonal antibody or antigen-binding fragment thereof comprises a combination of a heavy chain variable region and a light chain variable region selected from any one of the following ac) to ah):
ac) the heavy chain variable region comprising the same HCDR1, HCDR2 and HCDR3 regions as those of the heavy chain variable region as shown in SEQ ID NO: 2, and the light chain variable region comprising the same LCDR1, LCDR2 and LCDR3 regions as those of the light chain variable region as shown in SEQ ID NO: 3;
ad) the heavy chain variable region comprising the same HCDR1, HCDR2 and HCDR3 regions as those of the heavy chain variable region as shown in SEQ ID NO: 4, and the light chain variable region comprising the same LCDR1, LCDR2 and LCDR3 regions as those of the light chain variable region as shown in SEQ ID NO: 5;
ae) the heavy chain variable region comprising the same HCDR1, HCDR2 and HCDR3 regions as those of the heavy chain variable region as shown in SEQ ID NO: 6, and the light chain variable region comprising the same LCDR1, LCDR2 and LCDR3 regions as those of the light chain variable region as shown in SEQ ID NO: 7;
af) the heavy chain variable region comprising the same HCDR1, HCDR2 and HCDR3 regions as those of the heavy chain variable region as shown in SEQ ID NO: 8, and the light chain variable region comprising the same LCDR1, LCDR2 and LCDR3 regions as those of the light chain variable region as shown in SEQ ID NO: 9;
ag) the heavy chain variable region comprising the same HCDR1, HCDR2 and HCDR3 regions as those of the heavy chain variable region as shown in SEQ ID NO: 10, and the light chain variable region comprising the same LCDR1, LCDR2 and LCDR3 regions as those of the light chain variable region as shown in SEQ ID NO: 11; or
ah) the heavy chain variable region comprising the same HCDR1, HCDR2 and HCDR3 regions as those of the heavy chain variable region as shown in SEQ ID NO: 12, and the light chain variable region comprising the same LCDR1, LCDR2 and LCDR3 regios as those of the light chain variable region as shown in SEQ ID NO: 13.

In some embodiments of the anti-GCGR monoclonal antibody or antigen-binding fragment thereof, wherein the antigen-binding fragment is selected from the group consisting of Fab, Fab', F(ab')2, single-chain antibody, dimerized V region (diabody) and disulfide-stabilized V region (dsFv).

In some embodiments, the anti-GCGR monoclonal antibody or antigen-binding fragment thereof is characterized in that it competes with the monoclonal antibody or antigen-binding fragment thereof mentioned above for binding to human GCGR (or its epitope).

In some embodiments, the anti-GCGR monoclonal antibody or antigen-binding fragment thereof has at least one of the following features:
i. Antagonistic activity of blocking human GCGR to bind to human glucagon at an IC50 value of less than 500nM, less than 450nM, less than 400nM, less than 350nM, or less than 300nM (preferably less than 300nM);
ii. Antagonistic activity of blocking cynomolgus monkey or rhesus monkey GCGR to bind to cynomolgus monkey or rhesus monkey glucagon;
iii. Inhibition of the increase of human blood glucose concentration; and
iv. Antagonistic activity of blocking mouse GCGR to bind to mouse glucagon.

In some embodiments of the anti-GCGR monoclonal antibody or antigen-binding fragment thereof, wherein the monoclonal antibody or antigen-binding fragment thereof binds to the same antigen epitope as that of the monoclonal antibody or antigen-binding fragment thereof mentioned above.

In another aspect, the present disclosure provides a bispecific protein. In some embodiments, the bispecific protein provided herein comprises a GLP-1 peptide and a GCGR antibody, and the GLP-1 peptide is covalently linked to the polypeptide chain of the GCGR antibody via a peptide bond or a linker.

In some embodiments of the bispecific protein, wherein the carboxyl terminus of the GLP-1 peptide is linked to the amino terminus of the heavy chain variable region of the GCGR antibody via a peptide bond or a linker, or the carboxyl terminus of the GLP-1 peptide is linked to the amino terminus of the light chain variable region of the GCGR antibody via a peptide bond or a linker.

In some embodiments of the bispecific protein, the carboxyl terminus of the GLP-1 peptide is linked to the amino terminus of the heavy chain variable region of the GCGR antibody via a peptide bond or a linker.

In some embodiments of the bispecific protein, the carboxyl terminus of the GLP-1 peptide is linked to the amino terminus of the light chain variable region of the GCGR antibody via a peptide bond or a linker.

In some embodiments of the bispecific protein, the carboxyl terminus of the GLP-1 peptide is linked to the amino terminus of the heavy chain of the full-length GCGR antibody via a peptide bond or a linker.

In some embodiments of the bispecific protein, the carboxyl terminus of the GLP-1 peptide is linked to the amino terminus of the light chain of the full-length GCGR antibody via a peptide bond or a linker.

In some embodiments of the bispecific protein, the GCGR antibody is selected from the anti-GCGR monoclonal antibodies or antigen-binding fragments thereof mentioned above.

In some embodiments of the bispecific protein, the GLP-1 peptide is GLP-1A as shown in SEQ ID NO: 91, or the GLP-1 peptide is a GLP-1A peptide variant having one or more amino acid substitutions of Q17E, I23 V K28R and G30R based on GLP-1A.

In some embodiments of the bispecific protein, the GLP-1 peptide is GLP-1A as shown in SEQ ID NO: 91, or the GLP-1 peptide is a GLP-1A peptide variant having Q17E based on GLP-1A.

In some embodiments of the bispecific protein, the GLP-1 peptide is GLP-1A as shown in SEQ ID NO: 91, or the GLP-1 peptide is a GLP-1A peptide variant having Q17E and one or more amino acid substitutions of I23V, K28R and G30R based on GLP-1A.

In some embodiments of the bispecific protein, the GLP-1 peptide is GLP-1A as shown in SEQ ID NO: 91, or the GLP-1 peptide is a GLP-1A peptide variant having Q17E or both Q17E and I23V based on GLP-1A.

In some embodiments of the bispecific protein, the GLP-1A peptide variant comprises or consists of the sequence as shown in SEQ ID NO: 92, 93, 94, 95, 96, 97, 98 or 99.

In some embodiments of the bispecific protein, the GCGR antibody is selected from any one of the anti-GCGR monoclonal antibodies or antigen-binding fragments thereof mentioned above, and the GLP-1A peptide or GLP-1A peptide variant is any one as described above.

In some embodiments, the bispecific protein comprises a first polypeptide chain and a second polypeptide chain, wherein the first polypeptide chain is a polypeptide selected from the group consisting of SEQ ID NO: 100, 101, 102, 103, 104, 105, 106, 107 and 108, and the second polypeptide chain is a polypeptide as shown in SEQ ID NO: 79.

In some embodiments, the bispecific protein comprises a first polypeptide chain comprising the GCGR antibody heavy chain and a second polypeptide chain comprising the GCGR antibody light chain, wherein: the first polypeptide chain is a polypeptide as shown in SEQ ID NO: 109, and the second polypeptide chain is a polypeptide as shown in SEQ ID NO: 81;

In some embodiments, the bispecific protein comprises a first polypeptide chain comprising the GCGR antibody heavy chain and a second polypeptide chain comprising the GCGR antibody light chain, wherein: the first polypeptide chain is a polypeptide as shown in SEQ ID NO: 110, and the second polypeptide chain is a polypeptide as shown in SEQ ID NO: 83;

In some embodiments, the bispecific protein comprises a first polypeptide chain comprising the GCGR antibody heavy chain and a second polypeptide chain comprising the GCGR antibody light chain, wherein: the first polypeptide chain is a polypeptide as shown in SEQ ID NO: 111, and the second polypeptide chain is a polypeptide as shown in SEQ ID NO: 85;

In some embodiments, the bispecific protein comprises a first polypeptide chain comprising the GCGR antibody heavy chain and a second polypeptide chain comprising the GCGR antibody light chain, wherein: the first polypeptide chain is a polypeptide as shown in SEQ ID NO: 112, and the second polypeptide chain is a polypeptide as shown in SEQ ID NO: 87; or

In some embodiments, the bispecific protein comprises a first polypeptide chain comprising the GCGR antibody heavy chain and a second polypeptide chain comprising the GCGR antibody light chain, wherein: the first polypeptide chain is a polypeptide as shown in SEQ ID NO: 113, and the second polypeptide chain is a polypeptide as shown in SEQ ID NO: 89.

In another aspect, the present disclosure also provides a GLP-1 peptide variant. In some embodiments, the GLP-1 peptide variant is a mutant having one or more amino acid mutation(s) of Q17E, I23V, K28R and G30R based on GLP-1A as shown in SEQ ID NO:91.

In some embodiments, the GLP-1 peptide is GLP-1A as shown in SEQ ID NO: 91, or the GLP-1 peptide is a GLP-1A peptide variant having Q17E or both Q17E and 123 V based on GLP-1A.

In some embodiments, the GLP-1 peptide variant has a sequence as shown in SEQ ID NO: 92, 93, 94, 95, 96, 97, 98 or 99.

The present disclosure also provides a pharmaceutical composition, comprising a therapeutically effective amount of the anti-GCGR monoclonal antibody or antigen-binding fragment thereof as described above, or the bispecific protein as described above, or the GLP-1 peptide variant as described above, and one or more pharmaceutically acceptable carriers, diluents, buffers or excipients.

The present disclosure also provides an isolated nucleic acid molecule, which encodes the anti-GCGR monoclonal antibody or antigen-binding fragment thereof as described above, or the bispecific protein as described above, or the GLP-1 peptide variant as described above.

The present disclosure provides a recombinant vector comprising the isolated nucleic acid molecule as described above.

The present disclosure provides a host cell transformed with the recombinant vector as described above, said host cell being selected from prokaryotic cells and eukaryotic cells, preferably eukaryotic cells, more preferably mammalian cells or insect cells.

The present disclosure provides a method for producing the anti-GCGR monoclonal antibody or antigen-binding fragment thereof as described above, or the bispecific protein as described above, or the GLP-1 peptide variant as described above, the method comprising culturing the host cell as described above in a culture medium to generate and accumulate the anti-GCGR monoclonal antibody or antigen-binding fragment thereof as described above, or the bispecific protein as described above, or the GLP-1 peptide variant as described above, and recovering the monoclonal antibody or antigen-binding fragment thereof or the bispecific protein or the GLP-1 peptide variant from the culture.

The present disclosure provides a method for detecting or measuring human GCGR *in vitro,* the method comprising using the monoclonal antibody or antigen-binding fragment thereof as described above.

A kit for detecting human GCGR, comprises the monoclonal antibody or antigen-binding fragment thereof as described above.

According to some embodiments, it also provides use of the anti-GCGR monoclonal antibody or antigen-binding fragment thereof as described above in the preparation of medical devices (such as kits, arrays, test papers, multi-well plates, magnetic beads, coated particles), the medical device comprising the monoclonal antibody or antigen-binding fragment thereof as described above. As an example, the kit includes a multi-well plate coated with the anti-GCGR monoclonal antibody or antigen-binding fragment thereof as described above.

The present disclosure provides use of the monoclonal antibody or antigen-binding fragment thereof as described above in the preparation of a reagent for detecting or measuring human GCGR.

The present disclosure provides a method for lowering the blood glucose concentration in a subject, the method comprising administering to the subject a therapeutically effective amount of the anti-GCGR monoclonal antibody or antigen-binding fragment thereof as described above, or the bispecific protein as described above, or the GLP-1 peptide variant as described above, or the pharmaceutical composition as described above.

Preferably, the therapeutically effective amount is 0.1 to 3000mg of the anti-GCGR monoclonal antibody or antigen-binding fragment thereof as described above, or the bispecific protein as described above, or the GLP-1 peptide variant as described above in a unit dose of the composition.

The present disclosure provides a method for the treatment of a metabolic disorder, the method comprising administering to a subject the anti-GCGR monoclonal antibody or antigen-binding fragment thereof as described above, or the bispecific protein as described above, or the GLP-1 peptide variant as described above, or the pharmaceutical composition as described above; preferably, the metabolic disorder is metabolic syndrome, obesity, impaired glucose tolerance, diabetes, diabetic ketoacidosis, hyperglycemia, hyperglycemic hyperosmolar syndrome, perioperative hyperglycemia, hyperinsulinemia, insulin resistance syndrome, impaired fasting glucose, dyslipidemia, atherosclerosis or prediabetic condition.

The present disclosure also provides a use of the anti-GCGR monoclonal antibody or antigen-binding fragment thereof as described above, or the bispecific protein as described above, or the GLP-1 peptide variant as described above, or the pharmaceutical composition as described above in the preparation of a medicament for the treatment of a metabolic disorder or for lowering the blood glucose concentration in a subject;

Preferably, the metabolic disorder is metabolic syndrome, obesity, impaired glucose tolerance, diabetes, diabetic ketoacidosis, hyperglycemia, hyperglycemic hyperosmolar syndrome, perioperative hyperglycemia, hyperinsulinemia, insulin resistance syndrome, impaired fasting glucose, dyslipidemia, atherosclerosis or prediabetic condition.

The present disclosure also provides the anti-GCGR monoclonal antibody or antigen-binding fragment thereof as described above, or the bispecific protein as described above, or the GLP-1 peptide variant as described above, or the pharmaceutical composition as described above, for use as a medicament, preferably for use as a medicament for the treatment of a metabolic disorder or for lowering the blood glucose concentration in a subject.

More preferably, the metabolic disorder is metabolic syndrome, obesity, impaired glucose tolerance, diabetes, diabetic ketoacidosis, hyperglycemia, hyperglycemic hyperosmolar syndrome, perioperative hyperglycemia, hyperinsulinemia, insulin resistance syndrome, impaired fasting glucose, dyslipidemia, atherosclerosis or prediabetic condition.

### DESCRIPTION OF THE DRAWINGS

Figure 1: Schematic diagram of the structure of the bispecific protein (GLP-1/GCGR antibody) of the present disclosure.
Figure 2: Antagonistic activity of the bispecific protein and GCGR antibody against GCGR.
Figure 3: Activating activity of the bispecific protein and Dulaglutide for GLP-1R.
Figure 4: Effect of long-term administration on random blood glucose concentrations in ob/ob mice, Vehicle (empty vector) is a control model injected with phosphate buffered saline (PBS), hu1803-9D-3mpk, hu1803-9-2.84mpk and Dulaglutide-1.16mpk refer to the same molarity of the substance.
Figure 5: Effect of long-term administration on fasting blood glucose concentrations in ob/ob mice. All the experimental groups can significantly reduce the fasting blood glucose concentration in mice, among the groups, hu1803-9D-3mpk and hu1803-9-2.84mpk have the most powerful ability in lowering the blood glucose concentration, and hu1803-9D-3mpk exhibits a superior ability in lowering the blood glucose than that of hu1803-9-2.84mpk.

### DETAILED DESCRIPTION OF THE INVENTION

### Terminology

Three-letter codes and one-letter codes for amino acids used in the present disclosure are as described in J. biol. chem, 243, p3558(1968).

The term "bispecific protein" refers to a protein molecule capable of binding to two target proteins or target antigens. The bispecific protein used in the present disclosure specifically refers to a protein that is capable of binding to GCGR and GLP-1R (GLP-1 receptor), and is formed by a GLP-1 peptide fused to a polypeptide chain of a GCGR antibody (or antigen-binding fragment thereof).

"GLP-1 peptide" refers to a peptide capable of binding to and activating the GLP-1 receptor. Peptides in the prior art are described in Patent Applications WO2008/071972, WO2008/101017, WO2009/155258, WO2010/096052, WO2010/096142, WO2011/075393, WO2008/152403, WO2010/070251, WO2010/070252, WO2010/070253, WO2010/070255, WO2011/160630, WO2011/006497, WO2011/117415, WO2011/117416, WO2006/134340, WO1997046584, WO2007124461, WO2017100107, WO2007039140, CN1935261A, CN1935846A, WO2006036834, WO2005058958, WO2002046227, WO1999043705, WO1999043708, WO1999043341, CN102949730, CN104293834, CN104327187, WO2015067716, WO2015049651, WO2014096145, WO2014096148, WO2014096150, WO2014096149, the contents of which are all incorporated herein by reference. Some specific GLP-1 peptides, including GLP-1, GLP-1 analogs and GLP-1 receptor peptide agonists, are, for example, Lixisenatide/AVE0010/ZP10/Lyxumia, Exenatide/Exendin-4/Byetta/Bydureon/ITCA 650/AC-2993, liraglutide/Victoza, Semaglutide, Taspoglutide), SyncriaAlbiglutide, Dulaglutide, rExendin-4, CJC-1134-PC, PB-1023, TTP-054, Langlenatide/HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034, MOD-6030, CAM-2036, DA-15864, ARI-2651, ARI-2255, Exenatide-XTEN and Glucagon-Xten. In addition to the above GLP-1 peptides, GLP-1A as shown in SEQ ID NO: 91 and mutants thereof (as shown in SEQ ID NOs: 92-99) of the present disclosure are also included.

GPCR, i.e., G Protein-Coupled Receptor, is a type of transmembrane protein expressed on the cytoplasmic membrane. GPCR is composed of more than 800 members and is the largest membrane protein family in the mammalian genome currently known. In the human body, GPCR proteins are widely distributed in the organs and tissues such as central nervous system, immune system, heart and blood vessels and retina, and are involved in the body development and normal functioning.

The main body of the GPCR protein is composed of seven segments of a trans-cytoplasmic membrane alpha helix structure. The N-terminus and three loops are located outside the cell and are involved in the interaction between the protein and its receptor; the C-terminus and three loops are located within the cell, of which the C-terminus and the third loop plays an important role in the process of intracellular signal transduction mediated by the interaction between the GPCR protein and the downstream G protein.

"GCGR" is a glucagon receptor and a member of the GPCR family. Glucagon mainly accelerates the glycogenolysis, lipolysis and/or gluconeogenesis by activating the downstream pathway upon binding to GCGR, and thereby increasing the level of blood glucose.

The term "antibody (Ab)" includes at least one complementary determining region antigen-binding molecule (or molecule complex) that specifically binds to or interacts with (e.g., recognizes and/or binds to) a specific antigen (or epitope) (e.g., GCGR).

The term "antibody" includes immunoglobulin molecules comprising four polypeptide chains, two heavy (H) chains and two light (L) chains interconnected by disulfide bond(s), and multimers thereof (for example, IgM). Each heavy chain includes a heavy chain variable region (hereinafter abbreviated as HCVR or VH) and a heavy chain constant region (CH). This heavy chain constant region comprises three regions (domains), CH1, CH2, and CH3. Each light chain includes a light chain variable region (hereinafter abbreviated as LCVR or VL) and a light chain constant region (CL). The VH and VL regions can be further subdivided into hypervariable regions, named as complementarity determining regions (CDRs), among which interspersed with more conservative regions, named as framework regions (FRs, also named as frameworks). Each VH and VL is composed of three CDRs and four FRs, arranged from the amino terminus to the carboxyl terminus in the following order: FR1, CDR1, FR2, CDR2, FR3, CDR3, FR4.

In various embodiments of the present disclosure, the FRs of the anti-GCGR antibody (or antigen-binding fragment thereof) can be the same as those of the human germline sequence, or can be modified naturally or artificially. The antibodies can be different subclasses of antibodies, for example, IgG (e.g., IgG1, IgG2, IgG3, or IgG4 subclass), IgA1, IgA2, IgD, IgE or IgM antibody.

The term "antibody" also encompasses antigen-binding fragments of a full-length antibody molecule.

The terms "antigen-binding portion", "antigen-binding domain", "antigen-binding fragment" of an antibody and the like, as used herein, include any naturally occurring, enzymatically produced, synthetically or genetically engineered polypeptide or glycoprotein that specifically binds to an antigen to form a complex. Antigen-binding fragments of an antibody can be derived from, for example, a complete antibody molecule by using any suitable standard techniques, such as proteolytic digestion or recombinant genetic engineering technique involving manipulation and expression of DNAs encoding the antibody variable regions and (optionally) constant regions. The DNAs are known and/or can be easily obtained from, for example, commercially available sources, DNA database (including, for example, phage-antibody database), or can be synthesized. The DNAs can be sequenced and manipulated chemically or by molecular biotechnology, for example, arranging one or more variable and/or constant regions into a suitable configuration, introducing codons, generating cysteine residues, modifying, adding or deleting amino acids, etc.

Non-limiting examples of antigen-binding fragments include: (i) Fab fragment; (ii) F(ab') 2 fragments; (iii) Fd fragments; (iv) Fv fragments; (v) single-chain Fv (scFv) molecules; and (vi) dAb fragments. Other engineered molecules, such as region-specific antibodies, single-domain antibodies, region-deleted antibodies, chimeric antibodies, diabodies, tribodies, tetrabodies, minibodies, nanobodies (e.g. monovalent nanobodies, bivalent nanobodies, etc.), Small Modular Immunopharmaceuticals (SMIP) and Shark Variable IgNAR Regions are also included in the term "antigen-binding fragment" as used herein.

The antigen-binding fragment will typically contain at least one variable region. The variable region can be a region of any size or amino acid composition and will generally comprise one or more CDRs adjacent to or within the framework sequences.

In some embodiments, the antigen-binding fragment of an antibody can be in any configuration of variable region and constant region, the variable region and the constant region can be connected to each other directly or through a complete or partial hinge or a linker region. The hinge region can be composed of at least 2 (for example, 5, 10, 15, 20, 40, 60 or more) amino acids, which allows a flexible and semi-flexible connection generated between the adjacent variable and/or constant region within a single polypeptide molecule.

"Murine antibody" as used herein refers to mouse- or rat-derived monoclonal antibodies prepared according to the knowledge and skills in the art. During the preparation, test subjects are injected with an antigen, and then a hybridoma expressing the antibody which possesses desired sequence or functional characteristics is isolated. When the injected test subjects are mice, the resulting antibody is a mouse-derived antibody, and when the injected test subjects are rats, the resulting antibody is a rat-derived antibody.

A "chimeric antibody" is an antibody formed by fusing antibody variable region(s) of a first species (such as mouse) to antibody constant region(s) of a second species (such as human). To establish a chimeric antibody, it is necessary to establish a hybridoma that secretes a monoclonal antibody of the first species (such as mouse), to clone the variable region(s) gene from the hybridoma cell, and then to clone the constant region gene(s) of an antibody of the second species (such as human) as needed. The variable region gene(s) of the first species is (are) connected to the constant region gene(s) of the second species to form a chimeric gene, which is then inserted into an expression vector, and finally the chimeric antibody molecule is expressed in a eukaryotic or a prokaryotic system.

In a preferable embodiment of the present disclosure, the antibody light chain of the chimeric antibody further comprises a light chain constant region of a human kappa, lambda chain or a variant thereof. The antibody heavy chain of the chimeric antibody further comprises a heavy chain constant region of human IgG1, IgG2, IgG3, IgG4 or a variant thereof, preferably comprises a heavy chain constant region of human IgG1, IgG2 or IgG4, or comprises a heavy chain constant region variant of human IgG1, IgG2 or IgG4 with amino acid mutation(s) (such as YTE mutation or back mutation, L234A and/or L235A mutation, or S228P mutation).

The term "humanized antibody", including CDR-grafted antibody, refers to an antibody generated by grafting animal-derived antibody, e.g., murine antibody CDR sequences into human antibody variable region frameworks (i.e., framework regions). Humanized antibodies can avoid heterologous responses induced by chimeric antibodies which carry a large number of heterologous protein components. Such framework sequences can be obtained from public DNA database covering germline antibody gene sequences or published references. For example, germline DNA sequences of human heavy and light chain variable region genes can be found in "VBase" human germline sequence database (available on http://www.vbase2.org/), as well as in Kabat, E.A., et al. 1991 Sequences of Proteins of Immunological Interest, 5th Ed.

To avoid a decrease in activity caused by the decreased immunogenicity, the framework sequences in human antibody variable region can be subjected to minimal reverse mutations or back mutations to maintain the activity. The humanized antibodies of the present disclosure also comprise humanized antibodies on which CDR affinity maturation is performed by phage display.

Due to the residues contacted with an antigen, the grafting of CDR can result in a decreased affinity of an antibody or antigen binding fragment thereof to the antigen due to the framework residues contacted with the antigen. Such interactions can be resulted from highly somatic mutations. Therefore, it may still be necessary to graft the donor framework amino acids onto the humanized antibody frameworks. The amino acid residues involved in antigen binding and derived from non-human antibody or antigen binding fragment thereof can be identified by checking the sequence and structure of animal monoclonal antibody variable region. The donor CDR framework amino acid residues which are different from the germ lines can be considered as being related. If it is not possible to determine the most closely related germ line, the sequence can be compared to the common sequence shared by subtypes or the animal antibody sequence with high similarity percentage. Rare framework residues are thought to be the result of a high mutation in somatic cells, and play an important role in binding.

In an embodiment of the present disclosure, the antibody or antigen-binding fragment thereof may further comprise a light chain constant region of human or murine κ, λ chain or variant thereof, or further comprises a heavy chain constant region of human or murine IgG1, IgG2, IgG3, IgG4 or variant thereof.

The "conventional variants" of the human antibody heavy chain constant region and the human antibody light chain constant region refer to the human heavy or light chain constant region variants disclosed in the prior art which do not change the structure and function of the antibody variable regions. Exemplary variants include IgG1, IgG2, IgG3 or IgG4 heavy chain constant region variants by site-directed modification and amino acid substitutions on the heavy chain constant region. The specific substitutions are, for example, YTE mutation, L234A and/or L235A mutations, S228P mutation, or mutations resulting in a knob-into-hole structure (making the antibody heavy chain form a combination of knob-Fc and hole-Fc), etc. These mutations have been proven to confer the antibody with new properties, without changing the function of the antibody variable region.

"Human antibody" and "antibody derived from human" can be used interchangeably, and can be antibodies derived from human or antibodies obtained from a transgenic organism which has been "engineered" and produced by any method known in the art to produce specific human antibodies in response to antigen stimulation. In some technologies, elements of human heavy and light chain loci are introduced into organism cell strains derived from embryonic stem cell lines, in which the endogenous heavy and light chain loci are targeted for disruption. Transgenic organisms can synthesize human antibodies specific for human antigens, and the organisms can be used to produce hybridomas that secrete human antibodies. A human antibody can also be such antibody in which the heavy and light chains are encoded by nucleotide sequences derived from one or more human DNA sources. Fully human antibodies can also be constructed by gene or chromosome transfection methods and phage display technology, or constructed from B cells activated *in vitro,* all of which are known in the art.

"Monoclonal antibody" refers to an antibody obtained from a population of substantially homogeneous antibodies, that is, the individual antibodies constituting the population are identical and/or bind to the same epitope, except for possible variant antibodies (for example, variants having naturally occurring mutations or mutations produced during the manufacture of monoclonal antibody preparations, and the mutations are usually present in minimal amounts). Unlike polyclonal antibody preparations that usually contain different antibodies directed against different determinants (epitopes), each monoclonal antibody of a monoclonal antibody preparation (formulation) is directed against a single determinant on the antigen. Therefore, the modifier "monoclonal" indicates the characteristics of the antibody obtained from a substantially homogeneous antibody population, and should not be interpreted as requiring any specific method to manufacture the antibody. For example, monoclonal antibodies used in accordance with the present disclosure can be prepared by various techniques, including but not limited to hybridoma methods, recombinant DNA methods, phage display methods, and methods by using transgenic animals having all or part of human immunoglobulin loci. Such methods, and other exemplary methods for preparing monoclonal antibodies are described herein. Monoclonal antibodies comprised in the term "monoclonal antibody or antigen-binding fragment thereof" refer to full-length antibodies.

The terms "full-length antibody", "full antibody", "whole antibody" and "complete antibody" are used interchangeably herein and refer to an antibody in a substantially complete form, as distinguished from antigen-binding fragments defined below. The term specifically refers to an antibody in which the heavy chain includes the VH region, the CH1 region, the hinge region and the Fc region in the order from the amino terminus to the carboxyl terminus, and the light chain includes the VL region and the CL region in the order from the amino terminus to the carboxyl terminus.

In addition, the VL domain and VH domain of the Fv fragment are encoded by two separate genes, however, they can be linked by a synthetic linker by using recombinant methods, to generate a single protein chain in which a monovalent molecular is formed by pairing the VL and VH domain (referred to as single chain Fv (scFv); see, e.g., Bird et al. (1988): 423-426; Science 242 and Huston et al (1988) Proc. Natl. Acad. Sci USA85:5879-5883). Such single chain antibodies are also intended to be included in the term of "antigen binding fragment" of an antibody. Such antibodies are obtained using conventional techniques known in the field, and are screened for functional fragments by using the same method as that for an intact antibody. Antigen binding portions can be produced by recombinant DNA technology or by enzymatic or chemical disruption of an intact immunoglobulin.

Antigen-binding fragments can also be incorporated into a single-chain molecule comprising a pair of tandem Fv fragments (VH-CH1-VH-CH1), and the pair of tandem Fv fragments form a pair of antigen-binding regions together with complementary light chain polypeptides (Zapata et al., 1995 Protein Eng. 8(10): 1057-1062; and US Patent No. 5,641,870).

Fab is an antibody fragment obtained by treating an IgG antibody molecule with a papain (which cleaves the amino acid residue at position 224 of the H chain), and the antibody fragment has a molecular weight of about 50,000 and has antigen binding activity, in which about a half of the N-terminal side of H chain and the entire L chain are bound together through disulfide bond(s).

F (ab')2 is an antibody fragment having molecular weight of about 100,000Da and having antigen binding activity and comprising two Fab regions which are bound at the hinge position, it can be produced by digesting the part downstream of the two disulfide bonds in the IgG hinge region with pepsin.

Fab' is an antibody fragment having a molecular weight of about 50,000 Da and having antigen binding activity, which is obtained by cleaving the disulfide bonds at the hinge region of the above-mentioned F (ab')2. Fab' can be produced by treating F(ab')2 that specifically recognizes and binds to an antigen with a reducing agent such as dithiothreitol.

Further, the Fab' can be produced by inserting DNA encoding Fab' of the antibody into a prokaryotic expression vector or eukaryotic expression vector and introducing the vector into a prokaryote or eukaryote to express the Fab'.

The term "single chain antibody", "single chain Fv" or "scFv" refers to a molecule comprising antibody heavy chain variable domain (or region; VH) connected to antibody light chain variable domain (or region; VL) by a linker. Such scFv molecules have general structure of NH2-VL-linker-VH-COOH or NH2-VH-linker-VL-COOH. Suitable linkers in the prior art consist of repeated GGGGS amino acid sequence or a variant thereof, for example, a variant with 1-4 (including 1, 2, 3 or 4) repeats (Holliger et al. (1993), Proc Natl Acad Sci USA. 90: 6444-6448). Other linkers that can be used in the present disclosure are described by Alfthan et al.,(1995), Protein Eng. 8:725-731, Choi et al., (2001), Eur J Immuno.31:94-106, Hu et al., (1996), Cancer Res.56:3055-3061, Kipriyanov et al., (1999), J Mol Biol. 293:41-56 and Roovers et al., (2001), Cancer Immunol Immunother. 50:51-59.

"Linker" refers to a connecting peptide sequence used to connect protein domains, usually with a certain degree of flexibility, and the use of linkers will not cause the protein domain to lose its original functions.

Diabody is an antibody fragment wherein the scFv is dimerized, and it is an antibody fragment having bivalent antigen binding activity. In the bivalent antigen binding activity, the two antigens can be the same or different.

dsFv is obtained by substituting one amino acid residue in each of VH and VL with a cysteine residue, and then connecting the substituted polypeptides via a disulfide bond between the two cysteine residues. The amino acid residues to be substituted with a cysteine residue can be selected based on three-dimensional structure prediction of the antibody in accordance with known methods (Protein Engineering, 7, 697 (1994)).

In some embodiments of the present disclosure, the antigen-binding fragment can be produced by the following steps: obtaining cDNAs encoding the monoclonal antibody VH and/or VL of the present disclosure that specifically recognizes and binds to the antigen, and cDNAs encoding the other domains as required; constructing DNA encoding the antigen-binding fragment; inserting the DNA into a prokaryotic or eukaryotic expression vector, and then introducing the expression vector into a prokaryote or eukaryote to express the antigen-binding fragment.

"Fc region" can be a naturally occurring sequence or a variant Fc region. The boundaries of the Fc region of an immunoglobulin heavy chain are likely to vary, however, the Fc region of a human IgG heavy chain is usually defined as being extending from the amino acid residue at position Cys226 or from Pro230 to its carboxyl terminus. The numbering of residues in the Fc region is according to the EU index numbering in Kabat. Kabat et al., Sequences of Proteins of Immunological Interest, 5th Edition Public Health Service, National Institutes of Health, Bethesda, Md., 1991. The Fc region of immunoglobulin usually has two constant domains, CH2 and CH3 .

"Knob-Fc" refers to a knob-like spatial structure formed by incorporating a point mutation T366W in the Fc region of an antibody. Correspondingly, "hole-Fc" refers to a hole-like spatial structure formed by incorporating point mutations T366S, L368A, and Y407V in the Fc region of an antibody. Knob-Fc and hole-Fc are more likely to form heterodimers due to steric hindrance. In order to further promote the formation of heterodimers, point mutations S354C and Y349C can be introduced into knob-Fc and hole-Fc, respectively, to further promote the formation of heterodimers via disulfide bonds. Meanwhile, in order to eliminate or alleviate the ADCC effect caused by antibody Fc, substitution mutations of 234A and 235A can also be introduced into Fc. In a bispecific antibody, knob-Fc or hole-Fc can be used as either the Fc region of the first polypeptide chain or the Fc region of the second polypeptide chain. For a single bispecific antibody, Fc regions of the first and the second polypeptide chain can not both be knob-Fc or hole-Fc.

The term "amino acid difference" or "amino acid mutation" refers to the amino acid changes or mutations in a protein or polypeptide variant compared to the original protein or polypeptide, comprising one or more amino acid insertions, deletions or substitutions on the basis of the original protein or polypeptide.

"Variable region" of an antibody refers to an antibody light chain variable region (VL) or antibody heavy chain variable region (VH), alone or in combination. As known in the field, each of the heavy and light chain variable regions consists of three complementarity determining regions (CDRs) (also named as hypervariable regions) connected to four framework regions (FRs). The CDRs in each chain are held tightly together by FRs and contribute to the formation of an antigen binding site of the antibody together with the CDRs from the other chain. There are at least two techniques for determining CDRs: (1) a method based on cross-species sequence variability (i.e., Kabat et al. Sequences of Proteins of Immunological Interest, (5th edition, 1991, National Institutes of Health, Bethesda MD)); and (2) a method based on the crystallographic study of antigen-antibody complexes (Al-Lazikani et al., J. Molec. Biol. 273:927-948 (1997)). As used herein, CDRs may refer to those determined by either of or the combination of the two methods.

The term "antibody framework" or "FR region" refers to a part of the variable domain, either VL or VH, which serves as a scaffold for the antigen binding loops (CDRs) of this variable domain. Essentially, it is a variable domain without CDRs.

The terms "complementary determining region" and "CDR" refer to one of the six hypervariable regions present in the antibody variable domain that mainly contribute to antigen binding. Generally, there are three CDRs (HCDR1, HCDR2, HCDR3) in each heavy chain variable region, and three CDRs (LCDR1, LCDR2, LCDR3) in each light chain variable region. The amino acid sequence boundaries of CDRs can be determined by any of a variety of well-known schemes, including the "Kabat" numbering criteria (see Kabat et al. (1991), "Sequences of Proteins of Immunological Interest", 5th edition, Public Health Service, National Institutes of Health, Bethesda, MD), "Chothia" numbering criteria (Al-Lazikani et al., (1997) JMB 273:927-948) and ImmunoGenTics (IMGT) numbering criteria (Lefranc MP, Immunologist, 7, 132- 136 (1999); Lefranc, MP, etc., Dev. Comp. Immunol., 27,55-77 (2003), and the like. For example, for the classical format, following the Kabat criteria, the CDR amino acid residues in the heavy chain variable domain (VH) are numbered as 31-35 (HCDR1), 50-65 (HCDR2) and 95-102 (HCDR3); and the CDR amino acid residues in the light chain variable domain (VL) are numbered as 24-34 (LCDR1), 50-56 (LCDR2), and 89-97 (LCDR3). Following the Chothia criteria, the CDR amino acid residues in VH are numbered as 26-32 (HCDR1), 52-56 (HCDR2) and 95-102 (HCDR3); and the amino acid residues in VL are numbered as 26-32 (LCDR1), 50-52 (LCDR2) and 91-96 (LCDR3). By combining both Kabat and Chothia to define CDRs, the CDRs are composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. Following IMGT criteria, the CDR amino acid residues in VH are roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). Following IMGT criteria, the CDR regions of an antibody can be determined by using IMGT/DomainGap Align Program.

"Any CDR variant thereof" in "HCDR1, HCDR2 and HCDR3 regions or any variant thereof " refers to a variant obtained by subjecting any one, or two, or three of the HCDR1, HCDR2 and HCDR3 regions to amino acid mutations.

"Antibody constant region domain" refers to the domains derived from the antibody light and heavy chain constant regions, comprising CL and CH1, CH2, CH3 and CH4 domains derived from different types of antibodies.

"Epitope" or "antigenic determinant" refers to a site on an antigen to which an immunoglobulin or antibody specifically binds. Epitopes usually include at least 3, 4, 5, 6, 7, 8, 9, 10, 11, 12, 13, 14, or 15 consecutive or non-contiguous amino acids in a unique spatial conformation. See, for example, Epitope Mapping Protocols in Methods in Molecular Biology, Vol. 66, G.E. Morris, Ed. (1996).

The term "specifically bind to", "selectively bind to", "selectively binds to" or "specifically binds to" refers to the binding of an antibody to a predetermined epitope on an antigen. Typically, the antibody binds with an affinity (KD) of less than about 10⁻⁸M, for example, less than about 10⁻⁹ M, 10⁻¹⁰ M or 10⁻¹¹ M or even less.

When the term "competition" is used in the context of antigen binding proteins (e.g., neutralizing antigen binding proteins or neutralizing antibodies) that compete for the same epitope, it means that competition occurs between the antigen binding proteins, which is determined by the assays wherein an antigen binding protein to be tested (e.g., an antibody or antigen-binding fragment thereof) prevents or inhibits (e.g., reduces) the specific binding of a reference antigen binding protein (e.g., a ligand or reference antibody) to a common antigen. Numerous types of competitive binding assays are available to determine whether an antigen binding protein competes with another. These assays are, for example, solid phase direct or indirect radioimmunoassay (RIA), solid phase direct or indirect enzyme immunoassay (EIA), Sandwich competition assay (see, e.g., Stahli et al, 1983, Methods in Enzymology 9: 242-253); solid phase direct biotin-avidin EIA (see, e.g., Kirkland et al, 1986, J. Immunol. 137: 3614-3619), solid phase direct labeling assay, solid phase direct labeling sandwich assay (see, e.g., Harlow and Lane, 1988, Antibodies, A Laboratory Manual, Cold Spring Harbor Press); solid phase direct labeling RIA with 1-125 label (see, e.g., Morel et al, 1988, Molec. Immunol. 25: 7-15); solid phase direct biotin-avidin EIA (see, e.g., Cheung, et al, 1990, Virology 176: 546-552); and direct labeling RIA (Moldenhauer et al, 1990, Scand. J. Immunol. 32: 77-82). Typically, the assay involves the use of a purified antigen capable of binding to both an unlabeled test antigen binding protein and a labeled reference antigen binding protein (the antigen is located on a solid surface or cell surface). Competitive inhibition is determined by measuring the amount of a label bound to the solid surface or to the cell surface in the presence of the test antigen binding protein. Usually, the test antigen binding protein is present in excess. Antigen binding proteins identified by competitive assay (competing with the antigen binding protein) includes: antigen binding proteins that bind to the same epitope as the reference antigen binding protein; and antigen binding proteins that bind to an epitope that is sufficiently close to the epitope to which the reference antigen binding protein binds, where the two epitopes spatially interfere with each other to hinder the binding. Additional details regarding methods for determining competitive binding are provided in the Examples herein. Typically, when a competing antigen binding protein is present in excess, it will inhibit (e.g., reduce) at least 40-45%, 45-50%, 50-55%, 55-60%, 60-65%, 65-70%, 70-75% or 75% or even more of the specific binding of the reference antigen binding protein to the common antigen. In some cases, the binding is inhibited by at least 80-85%, 85-90%, 90-95%, 95-97%, or 97% or even more.

The term "affinity" refers to the strength of the interaction between an antibody and an antigen at a single epitope. Within each antigenic site, the variable region of the antibody "arm" interacts with the antigen at multiple amino acid sites via weak non-covalent forces; the greater the interaction is, the stronger the affinity is. As used herein, the term "high affinity" of an antibody or antigen-binding fragment thereof (e.g., Fab fragment) generally refers to an antibody or antigen-binding fragment with K_{D} of 1E⁻⁹M or less (e.g., K_{D} of 1E⁻¹⁰M or less, K_{D} of 1E⁻¹¹M or less, K_{D} of 1E⁻¹²M or less, K_{D} of 1E⁻¹³M or less, K_{D} of 1E⁻¹⁴ M or less, etc.).

The term "KD" or "K_{D}" refers to a dissociation equilibrium constant for particular antibody-antigen interaction. Typically, the antibody binds to an antigen with a dissociation equilibrium constant (KD) of less than about 1E⁻⁸M, for example, less than about 1E⁻⁹M, 1E⁻¹⁰M or 1E⁻¹¹M or even less, for example, as determined by Surface Plasma Resonance (SPR) technology in Biacore instrument. The smaller the KD valuen is, the greater the affinity is.

The term "nucleic acid molecule" refers to DNA molecules and RNA molecules. The nucleic acid molecule can be single-stranded or double-stranded, but preferably is double-stranded DNA. A nucleic acid is "operably linked" when it is placed into a functional relationship with another nucleic acid sequence. For instance, a promoter or enhancer is operably linked to a coding sequence if it affects the transcription of the sequence.

The term "vector" means a construct capable of delivering one or more target genes or sequences, and preferably, expressing them in a host cell. Examples of vectors include, but are not limited to, viral vectors, naked DNA or RNA expression vectors, plasmids, cosmids or phage vectors, DNA or RNA expression vectors associated with cationic coagulants, DNA or RNA expression vectors encapsulated in liposomes, and certain eukaryotic cells such as producer cells.

Methods for producing and purifying antibodies and antigen-binding fragments are well known in the art, for example, A Laboratory Manual for Antibodies, Cold Spring Harbor Laboratory Press, Cold Spring Harbor, New York, chapters 5-8 and 15. For example, mice can be immunized with antigen or fragment thereof, and the resulting antibodies can then be renatured, purified, and sequenced for amino acid sequences by using conventional methods. Antigen-binding fragments can also be prepared by conventional methods. The antibodies or antigen binding fragments of the present disclosure are engineered to incorporate one or more human framework regions onto the CDR regions derived from non-human antibody. Human FR germline sequences can be obtained from website http://imgt.cines.fr, or from The Immunoglobulin Facts Book, 2001, ISBN 012441351, by aligning against IMGT human antibody variable germline gene database and MOE software.

The term "host cell" refers to a cell into which an expression vector has been introduced. Host cells may include bacterial, microbial, plant or animal cells. Bacteria suitable for transformation include members of enterobacteriaceae, such as Escherichia coli or Salmonella strains; Bacillaceae such as Bacillus subtilis; Pneumococcus; Streptococcus and Haemophilus influenzae. Suitable microorganisms include Saccharomyces cerevisiae and Pichia pastoris. Suitable animal host cell lines include CHO (Chinese Hamster Ovary cell line), HEK293 cells (non-limiting examples such as HEK293E cells), and NS0 cells.

The engineered antibodies or antigen-binding fragments can be prepared and purified by conventional methods. For example, the cDNA sequences encoding the heavy and light chains can be cloned and recombined into a GS expression vector. The recombinant immunoglobulin expression vector can be stably transfected into CHO cells. As an alternative prior art, mammalian expression systems can lead to glycosylation of antibodies, especially in the highly conserved N-terminal sites of the Fc region. Stable clones were obtained by expressing an antibody specifically binding to an antigen. Positive clones can be expanded in serum-free culture medium in bioreactors for antibody production. Culture medium, into which an antibody has been secreted, can be purified by conventional techniques. For example, purification can be performed on Protein A or Protein G Sepharose FF column comprising modified buffer. The nonspecific binding components are washed out. The bound antibody is eluted by pH gradient and antibody fragments are detected by SDS-PAGE, and then pooled. The antibodies can be filtered and concentrated using common techniques. Soluble mixtures and aggregates can be effectively removed by common techniques, such as size exclusion or ion exchange. The resulting product is needed to be frozen immediately, such as at -70°C, or lyophilized.

"Administration", "administer", "dosing" and "treatment", when applied to animals, humans, experimental subjects, cells, tissues, organs, or biological fluids, refer to administer exogenous pharmaceutical agents, therapeutic agents, diagnostic agents, compositions or artificial manipulations (for example, "euthanasia" as used in the examples) to the animals, humans, subjects, cells, tissues, organs, or biological fluids. "Administration" and "treatment" can refer, e.g., to therapeutic, pharmacokinetic, diagnostic, research, and experimental methods. The treatment of a cell encompasses contacting a reagent with the cell, as well as contacting a reagent with a fluid, where the fluid is in contact with the cell. "Administration" or "treatment" also means *in vitro* or *ex vivo* treatments, e.g., of a cell, with a reagent, diagnostic, binding compound, or with another cell. "Treatment", as it applies to a human, veterinary, or research subject, refers to therapeutic treatment, prophylactic or preventative measures, research and diagnostic applications.

"Treat" means to administer a therapeutic agent, such as a composition comprising any of the compounds of the present disclosure, internally or externally to a subject having (or suspected to have or being susceptible to) one or more disease symptoms for which the agent has known therapeutic activity. Typically, the agent is administered in an amount effectively to alleviate one or more disease symptoms in the subject or population to be treated, by inducing the regression of or inhibiting the progression of such symptom(s) by any clinically measurable degree. The amount of a therapeutic agent that is effective to alleviate any particular disease symptom (also referred to as the "therapeutically effective amount") may vary according to various factors such as the disease state, age, and body weight of the subject, and the ability of the drug to elicit a desired response in the subject. Whether a disease symptom has been alleviated can be assessed by any clinical measurement typically used by physicians or other skilled healthcare providers to assess the severity or progression status of that symptom. While the embodiment of the present disclosure (e.g., a treatment method or article of manufacture) is not effective in alleviating the target disease symptom(s) in every subject, it shall alleviate the target disease symptom(s) in a statistically significant number of subjects as determined by any statistical test known in the art such as Student's t-test, chi-square test, U-test according to Mann and Whitney, Kruskal-Wallis test (H-test), Jonckheere-Terpstra-test and Wilcoxon-test.

"Amino acid conservative modification" or "amino acid conservative substitution" means that the amino acids in a protein or polypeptide are substituted by other amino acids with similar characteristics (such as charge, side chain size, hydrophobicity/hydrophilicity, backbone conformation and rigidity, etc.), such that the changes can frequently be performed without altering the biological activity or other required characteristics (such as affinity and/or specificity to an antigen) of the protein or polypeptide. Those skilled in the art recognize that, in general, a single amino acid substitution in non-essential regions of a polypeptide does not substantially alter the biological activity (see, e.g., Watson et al. (1987) Molecular Biology of the Gene, The Benjamin/Cummings Pub. Co., p. 224 (4th Ed.)). In addition, substitutions with structurally or functionally similar amino acids are less likely to disrupt biological activity. Exemplary Amino Acid Conservative Substitutions are as follows:

| Original residue | Conservative substitution |
|---|---|
| Ala (A) | Gly; Ser |
| Arg(R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu. |

"Effective amount" or "effective dose" refers to the amount of a medicament, compound, or pharmaceutical composition necessary to obtain any one or more beneficial or desired results. For prophylactic applications, beneficial or desired results include elimination or reduction of risk, reduction of severity, or delay of the onset of the disease, including the biochemical, histological, and behavioral manifestations of the condition, its complications, and intermediate pathological phenotypes during the development of the condition. For therapeutic applications, beneficial or desired results include clinical results, such as reduction of the incidence of various conditions associated with target antigen of the present disclosure or improvement of one or more symptoms of the condition, reduction of the dosage of other agents required to treat the condition, enhancement of the efficacy of another agent, and/or delay of the progression of the condition associated with the target antigen of the present disclosure in subjects.

"Exogenous" refers to substances produced outside organisms, cells, or humans according to circumstances.

"Endogenous" refers to substances produced in cells, organisms, or human bodies according to circumstances.

"Homology" and "identity" are interchangeable herein and refer to the sequence similarity between two polynucleotide sequences or between two polypeptide sequences. When a position in both of the two sequences to be compared is occupied by the same base or amino acid monomer subunit, e.g., if a position in each of two DNA molecules is occupied by adenine, then the molecules are homologous at that position. The percentage of homology between two sequences is a function of the number of matching or homologous positions shared by the two sequences divided by the number of positions to be compared and then multiplied by 100. For example, when two sequences are optimally aligned, if 6 out of 10 positions in the two sequences are matched or homologous, then the two sequences are 60% homologous; if 95 out of 100 positions in the two sequences are matched or homologous, then the two sequences are 95% homologous. Generally, when two sequences are aligned, comparison is performed to give the maximum homology percentage. For example, the comparison can be performed by BLAST algorithm, in which the parameters of the algorithm are selected to give the maximum match between each sequence over the entire length of each reference sequence.

The following references relate to the BLAST algorithm frequently used for sequence analysis: BLAST algorithm (BLAST ALGORITHMS): Altschul, SF et al., (1990) J. Mol. Biol. 215:403-410; Gish, W. et al., (1993) Nature Genet. 3:266-272; Madden, T.L. et al., (1996) Meth. Enzymol. 266:131-141; Altschul, S.F. et al., (1997) Nucleic Acids Res. 25:3389-3402; Zhang, J. et al. (1997) Genome Res. 7:649-656. Other conventional BLAST algorithms such as those available from NCBI BLAST are also well known to those skilled in the art.

"Isolated" refers to a state of "being separated from its original environment", and in this case means that the indicated molecule is essentially free of other non-target biomolecules. In general, the term "isolated" is not intended to mean the complete absence of these materials or the absence of water, buffers or salts, unless they are present in an amount that significantly interferes with the experimental or therapeutic use of the compound as described herein.

"Optional" or "optionally" means that the event or circumstance that follows may but does not necessarily occur, and the description includes the instances in which the event or circumstance does or does not occur.

"Pharmaceutical composition" refers to a mixture comprising one or more compounds according to the present disclosure or a physiologically/pharmaceutically acceptable salt or produg thereof and other chemical components, such as physiologically/pharmaceutically acceptable carriers and excipients. The pharmaceutical composition aims at promoting the administration to an organism, facilitating the absorption of the active ingredient and thereby exerting a biological effect.

The term "pharmaceutically acceptable carrier" refers to any inactive substance suitable for use in a formulation for the delivery of antibodies or antigen-binding fragments. The carrier can be an anti-adhesive agent, adhesive agent, coating agent, disintegrating agent, filler or diluent, preservative (such as antioxidant, antibacterial or antifungal agent), sweetener, absorption delaying agent, wetting agent, emulsifier, buffer, and the like. Examples of suitable pharmaceutically acceptable carriers include water, ethanol, polyol (such as glycerol, propylene glycol, polyethylene glycol, and the like) dextrose, vegetable oil (such as olive oil), saline, buffer, buffered saline, and isotonic agent, such as sugars, polyol, sorbitol and sodium chloride.

Specific examples of the term "metabolic disorder" are metabolic syndrome, obesity, impaired glucose tolerance, diabetes, diabetic ketoacidosis, hyperglycemia, hyperglycemic hyperosmolar syndrome, perioperative hyperglycemia, hyperinsulinemia, insulin resistance syndrome, impaired fasting glucose, dyslipidemia, atherosclerosis, and prediabetic condition.

In addition, another aspect of the present disclosure relates to methods for immunodetection or determination of target antigens, reagents for immunodetection or determination of target antigens, methods for immunodetection or determination of cells expressing target antigens, and the diagnostic agents for diagnosing diseases associated with target antigen-positive cells, comprising the monoclonal antibodies or antibody fragments of the present disclosure that specifically recognize and bind to the target antigen as active ingredients.

In the present disclosure, the method for detecting or measuring the amount of the target antigen can be any known method. For example, it includes immunoassay or immunodetection method.

The immunoassay or immunodetection method is a method of detecting or measuring the amount of an antibody or antigen with a labeled antigen or antibody. Examples of immunoassay or immunodetection methods include radioactive substance-labeled immunoantibody method (RIA), enzyme immunoassay (EIA or ELISA), fluorescence immunoassay (FIA), luminescence immunoassay, western blotting, physicochemical method, and the like.

The above-mentioned diseases related to the target antigen-positive cells can be diagnosed by detecting or measuring the target antigen-expressing cells using the antibodies or antibody fragments of the present disclosure.

Cells expressing the polypeptide can be detected by the known immunodetection methods, preferably by immunoprecipitation, fluorescent cell staining, immunotissue staining, and the like. In addition, the method such as fluorescent antibody staining method with the FMAT8100HTS system (Applied Biosystem) can be used.

In the present disclosure, living samples to be detected or measured for the target antigen are not particularly limited, as long as they are possible to contain cells expressing the target antigen, for example, tissue cells, blood, plasma, serum, pancreatic juice, urine, faeces, tissue fluid or culture medium.

Dependent on the required diagnostic method, the diagnostic agent comprising the monoclonal antibody or antibody fragment thereof of the present disclosure may also contain reagents for performing an antigen-antibody reaction or reagents for detecting the reaction. The reagents for performing an antigen-antibody reaction include buffers, salts and the like. The reagents for detection include agents commonly used in immunoassay or immunodetection methods, for example, a labeled secondary antibody that recognizes the monoclonal antibody, antibody fragment or conjugate thereof, and a substrate corresponding to the label.

The details of one or more embodiments of the present disclosure are set forth in the above specification. The preferred methods and materials are described below, although any method and material similar or identical to those described herein can be used in the practice or testing of the present disclosure. Through the specification and claims, other features, purposes and advantages of the present disclosure will become apparent. In the specification and claims, the singular forms include plural aspects unless the context clearly indicates otherwise. Unless otherwise defined explicitly herein, all technical and scientific terms used herein have the meaning commonly understood by those skilled in the art to which this disclosure belongs. All patents and publications cited in the specification are incorporated by reference. The following examples are presented to further illustrate the preferred embodiments of the present disclosure. These examples should not be construed as limiting the scope of the present disclosure in any way, and the scope of the present disclosure is defined by the claims.

### EXAMPLES

### Example 1: Preparation of GCGR antigen and antibody

### 1.1 Construction and screening of antigens

The human GCGR cDNA encoding the full-length glucagon receptor of 477 amino acids was subcloned into an expression vector (such as pcDNA3.1) and was transfected into CHO-K1 cells. After screening and single cell cloning, single clones were selected for the study of the characteristics of antigen expression and receptor-based expression on cell surface. The following GCGR antigens refer to human GCGR, unless specifically indicated.

Full-length GCGR: used for the construction of GCGR-overexpressing cell lines, or used as antigens for the immunization and for subsequent detection:

MPPCQPQRPLLLLLLLLACQPQVPSAQVMDFLFEKWKLYGDQCHHNLSLLPPPT
ELVCNRTFDKYSCWPDTPANTTANISCPWYLPWHHKVQHRFVFKRCGPDGQW
VRGPRGQPWRDASQCQMDGEEIEVQKEVAKMYSSFQVMYTVGYSLSLGALLL
ALAILGGLSKLHCTRNAIHANLFASFVLKASSVLVIDGLLRTRYSQKIGDDLSVS
TWLSDGAVAGCRVAAVFMQYGIVANYCWLLVEGLYLHNLLGLATLPERSFFSLY
LGIGWGAPMLFVVPWAVVKCLFENVQCWTSNDNMGFWWILRFPVFLAILINFFI
FVRIVQLLVAKLRARQMHHTDYKFRLAKSTLTLIPLLGVHEVVFAFVTDEHAQG
TLRSAKLFFDLFLSSFQGLLVAVLYCFLNKEVQSELRRRWHRWRLGKVLWEERN
TSNHRASSSPGHGPPSKELQFGRGGGSQDSSAETPLAGGLPRLAESPF
(SEQ ID NO: 1)

### 1.2 Purification of GCGR hybridomas and recombinant antibodies

### (1) Isolation and purification of hybridoma supernatant/Protein G affinity chromatography:

For the purification of mouse hybridoma supernatant, Protein G affinity chromatography performed was preferable. The harvested hybridoma culture was centrifuged and the supernatant was taken, and based on the volume of the supernatant, 10-15% volume of 1M Tris-HCl (pH 8.0-8.5) was added to adjust pH of the supernatant. The Protein G column was washed with 3-5 × column volume of 6M guanidine hydrochloride, and then washed with 3-5×column volume of pure water; the column was equilibrated with 3-5×column volume of equilibrium buffer such as 1×PBS (pH7.4) buffer system; the cell supernatant was loaded at a low flow rate for binding, and the flow rate was controlled so that the retention time was about 1 min or longer; the column was washed with 3-5×column volume of 1×PBS (pH 7.4) until the UV absorption dropped to the baseline; The samples were eluted with 0.1M acetic acid/sodium acetate (pH3.0) buffer, the elution peaks were pooled according to UV detection. The eluted product was temporarily preserved after the pH was adjusted to 5-6 with 1M Tris-HCl (pH8.0). The eluted product can be subjected to solution replacement according to methods well-known to those skilled in the art, for example, replacing the solution with a desired buffer system by ultrafiltration-concentration with an ultrafiltration tube, or replacing the solution with a desired buffer system by using molecular exclusion such as G-25 desalting, or removing the aggregation components from the eluted product to improve the purity of the sample by using high-resolution molecular exclusion column such as Superdex 200.

### (2) Extraction of Fc tagged bispecific proteins or antibodies by Protein A affinity chromatography:

First, the cell culture supernatant expressing containing the Fc bispecific protein or the antibody was centrifuged at a high speed to collect the supernatant. The Protein A affinity column was washed with 3-5×column volume of 6M guanidine hydrochloride, and then washed with 3-5× column volume of pure water. The column was equilibrated with 3-5×column volume of 1×PBS (pH7.4) buffer system as equilibrium buffer. The cell supernatant was loaded at a low flow rate for binding, and the flow rate was controlled so that the retention time was about 1 min or longer; after the binding was finished, the column was washed with 3-5×column volume of 1×PBS (pH 7.4) until the UV absorption dropped to the baseline The samples were eluted with 0.1M acetic acid/sodium acetate (pH3.0-3.5) buffer, the elution peak was pooled according to UV detection. The eluted product was temporarily preserved after the pH was adjusted to 5-6 with 1M Tris-HCl (pH8.0). The eluted product can be subjected to solution replacement according to methods well-known to those skilled in the art, for example, replacing the solution with a desired buffer system by ultrafiltration-concentration with an ultrafiltration tube, or replacing the solution with a desired buffer system by using molecular exclusion such as G-25 desalting, or removing the aggregation components from the eluted product to improve the purity of the sample by using high-resolution molecular exclusion column such as Superdex 200.

### Example 2: Preparation of anti-human GCGR monoclonal antibody

### 2.1 Immunization

(1) Mouse immunization: The anti-human GCGR monoclonal antibodies were produced by immunizing mice. SJL white mice, female, 6-8 weeks old were used for experiment (Beijing Charles River Experimental Animal Technology Co., Ltd., animal production license number: SCXK (Jing) 2012-0001). Feeding environment: SPF level. After the mice were purchased, they were adapted to the laboratory environment for 1 week, 12/12 hours light/dark cycle, at temperature of 20-25 °C; humidity of 40-60 %. Then, the mice that had been adapted to the environment were immunized according to the following schemes. The antigen for immunization was CHO-K1 cells stably transfected with GCGR.
   Immunization scheme: Mice were pre-immunized with the adjuvant, TiterMax® Gold Adjuvant (Sigma Cat No. T2684), by intraperitoneal injection (i.p.), 0.1ml/mouse (first immunization). 15min later, mice were intraperitoneally injected (i.p.) with GCGR CHO-K1 stably transfected cells, 1E7 cells per mouse. The vaccination times were on days 0, 14, 28, 42, 56, 70, 84, 98 and 112. Blood samples were collected on days 35, 63 and 91, the antibody titers in mouse serum were determined by ELISA method. After 6 to 9 immunizations, mice with a high serum antibody titer that was tending to the plateau were selected for splenocyte fusion. Three days before the splenocyte fusion, GCGR CHO-K1 stably transfected cells were injected intraperitoneally (i.p.) at 1E7 cells per mouse for booster immunization.
(2) Rat immunization: 6-8 weeks old SD rats were immunized with DNA (encoding full-length hGCGR, the coding sequence can be found in Genbank accession number: NM_000160), and the antibody titers in rat serum were determined by FACS method. After 3 to 4 immunizations, rats with a high serum antibody titer that was tending to the plateau were selected for splenocyte fusion. Booster immunization was performed 3 days before the splenocyte fusion.

### 2.2 Splenocyte fusion

Hybridoma cells were obtained by fusing splenic lymphocytes with myeloma Sp2/0 cells (ATCC® CRL-8287™) by using an optimized PEG-mediated fusion procedure. The fused hybridoma cells were resuspended in complete medium (IMEM medium comprising 20% FBS, 1×HAT, 1×OPI) at a density of 0.5-1E6/ml, seeded in a 96-well plate with 100 µl/well, incubated at 37°C and 5% CO₂ for 3-4 days, supplemented with 100µl/well of HAT complete medium, and continued to be cultured for 3-4 days until clones formed. The supernatant was removed, 200µl/well of HT complete medium (IMDM medium comprising 20% FBS, 1×HT and 1×OPI) was added to each well, incubated at 37°C, 5% CO₂ for 3 days and then subjected to ELISA detection.

### 2.3 Screening of Hybridoma cells

According to the growth density of hybridoma cells, the hybridoma culture supernatant was detected by cell-binding ELISA method. Cells in the wells that tested with positive were timely expanded, cryopreserved, and subcloned 2 to 3 times until a single cell clone was obtained.

Cells after each subcloning were also tested by GCGR cell binding ELISA. The hybridoma clones were screened by the assay. The antibodies were further prepared by serum-free cell culture method. The antibodies were purified according to the example of purification described in the test examples.

### 2.4 Sequencing of the positive hybridoma clones

The procedures of cloning the sequences from the positive hybridoma were as follows. Hybridoma cells in logarithmic growth phase were collected. RNAs were extracted with Trizol (Invitrogen, Cat No.15596 -018) according to the kit's instruction and were reversely transcribed with PrimeScript™ Reverse Transcription kit (Takara, Cat No. 2680A). The cDNAs resulting from reverse transcription were amplified by PCR using mouse Ig-Primer Set (Novagen, TB326 Rev. B 0503), and were sequenced after PCR amplification.

The positive clones were obtained from the resulting DNA sequences, and the antibodies were prepared by the serum-free cell culture method. The antibodies were purified according to the purification example, and were subjected to several rounds of screening by using the cell-based GCGR binding blocking assay, thereby the murine hybridoma clones 1803, 1805, 1808, and 1810, and rat hybridoma clones 1817 and 1822 were obtained.

**Table 1: The light chain and heavy chain variable region sequences of GCGR antibody**

| Antibo dy name | Heavy chain variable region (SEQ ID NO) | Light chain variable region (SEQ ID NO) |
|---|---|---|
| m1803 | QFQLHQSGAELVKPGASVKLSCKAT GYTFTDYWIEWVKQRPGHGLEWIG EILPGSTYTNYNEKFKGRATFTAEPSS SSAYMQLSGLTTEDSAIYYCSRGLST LMAVDYFDYWGQGTTLTVSS (SEQ ID NO: 2) | DIQMTQTTSSLSASLGDRVTIN CRASQDISNYLNWYQQKPDGT VKLLIYYSSTLHSGVPSRFSGSG SGTDYSLTISHLEQEDIATYFCQ QTNIFPWTFGGGTKLEI (SEQ ID NO: 3) |
| m1805 | EVQLQQSGPELVKPGASVKIPCKTSG YTFTDYNMDWVKQSHGRSLEWIGSI DPDNGGTIYNQKFKGKATLTVDKSS STAYMELRSLTSEDTAVYYCTRDYY GSSSWFAYWGQGTLVTVSA (SEQ ID NO: 4) | DVVMTQSPATLSVTPGDRVSLS CRASQSISDYLHWYQQKSHESP RLLIKYASQSISGIPSRFSGSGSG SDFTLSINSVEPEDVGVYYCQN GHSFPYTFGGGTKLEIK (SEQ ID NO: 5) |
| m1808 | QVQLQQSGAELARPGASVKLSCKAS GDTFTTNGISWVKQRIGQGLEWIGEI YPRSGNTYYNENFKGKATLTADKSS TTAYMELRRLTSEDSAVYFCARSITS VIGADYFDYWGQGTTLTVSS (SEQ ID NO: 6) | DIQMTQTTSSLSASLGDRVTISC RASQDISNYLNWYQKKPDGTV KLLIYYSSTLHSGVPSRFSGSGS GTDYSLTISNLEQEDIATYFCQQ GNTFPWTFGGGTKLEIK (SEQ ID NO: 7) |
| m1810 | QVQLQQSGTELARPGTSVTLSCKAS GYTFTNYAISWVKQRTGQGLEWIGE IYPTSGNTYYNEKFKGKATLTADRSS KTMYMELRSLTSVDSAVYFCASGVI TTVVSTDYFDYWGQGSPLTVSS (SEQ ID NO: 8) | DIQMTQIPSSLSVSLGDRVTISC RASLDISNYLNWYQLKPDGTV KLLIYYTSTLHSGVSSRFSGSGS GTEYSLTISNLEQEDIATYFCQQ GNMVPYTFGGGTKLEIK (SEQ ID NO: 9) |
| rat1817 | EVQLVESGGDLVQPGRSMKLSCAAS GFTFSNYYMAWVRQAPTKGLEWVA SISTGGVNTYYRDSVKGRFTISRDNA KNNLYLQMDSLRSEETATYYCARHT TADYFYGIYFALDAWGQGTSVTVSS (SEQ ID NO: 10) | QFTLTQPKSVSGSLRSTITIPCER SSGDIGDSYVNWYQQHLGRPP LNVIYADVQRPSEVSDRFSGSID SSSNSASLTITNLQMDDEADYF CQSYDTNIDIIFGGGTKLTVL (SEQ ID NO: 11) |
| rat1822 | EVRLVESGGDFVQPGRSVKLSCAAS GFTFSNYYMAWVRQAPTKGLEWVG SISTGGVNTYYRDSVKGRFTISRDNA ESTLYLQMDSLRSEETATYYCARHTT PDYHYGIYFAMDAWGQGTSVTVSS (SEQ ID NO: 12) | QVTLTQPKSVSGSLRSTITIPCE RSSGDIGESYVNWYQQHLGRP PINVIYADDQRPSEVSDRFSGSI DSSSNSASLTITNLQVDDEADY FCQSYDSSIDIFFGGGTKLTVL (SEQ ID NO: 13) |

**Table 2. CDR region sequences according to Kabat Numbering**

| Antibody Name | Heavy chain CDR | | Light chain CDR | |
|---|---|---|---|---|
| m1803 | HCDR1 | DYWIE (SEQ ID NO: 14) | LCDR1 | RASQDISNYLN (SEQ ID NO: 17) |
| | HCDR2 | EILPGSTYTNYNEKFKG (SEQ ID NO: 15) | LCDR2 | YSSTLHS (SEQ ID NO: 18) |
| | HCDR3 | GLSTLMAVDYFDY (SEQ ID NO: 16) | LCDR3 | QQTNIFPWT (SEQ ID NO: 19) |
| m1805 | HCDR1 | DYNMD (SEQ ID NO: 20) | LCDR1 | RASQSISDYLH (SEQ ID NO: 23) |
| | HCDR2 | SIDPDNGGTIYNQKFKG (SEQ ID NO: 21) | LCDR2 | YASQSIS (SEQ ID NO: 24) |
| | HCDR3 | DYYGSSSWFAY (SEQ ID NO: 22) | LCDR3 | QNGHSFPYT (SEQ ID NO: 25) |
| m1808 | HCDR1 | TNGIS (SEQ ID NO: 26) | LCDR1 | RASQDISNYLN (SEQ ID NO: 29) |
| | HCDR2 | EIYPRSGNTYYNENFKG (SEQ ID NO: 27) | LCDR2 | YSSTLHS (SEQ ID NO: 30) |
| | HCDR3 | SITSVIGADYFDY (SEQ ID NO: 28) | LCDR3 | QQGNTFPWT (SEQ ID NO: 31) |
| m1810 | HCDR1 | NYAIS (SEQ ID NO: 32) | LCDR1 | RASLDISNYLN (SEQ ID NO: 35) |
| | HCDR2 | EIYPTSGNTYYNEKFKG (SEQ ID NO: 33) | LCDR2 | YTSTLHS (SEQ ID NO: 36) |
| | HCDR3 | GVITTVVSTDYFDY (SEQ ID NO: 34) | LCDR3 | QQGNMVPYT (SEQ ID NO: 37) |
| rat1817 | HCDR1 | NYYMA (SEQ ID NO: 38) | LCDR1 | ERSSGDIGDSYVN (SEQ ID NO: 41) |
| | HCDR2 | SISTGGVNTYYRDSVKG (SEQ ID NO: 39) | LCDR2 | ADVQRPS (SEQ ID NO: 42) |
| | HCDR3 | HTTADYFYGIYFALDA (SEQ ID NO: 40) | LCDR3 | QSYDTNIDII (SEQ ID NO: 43) |
| rat1822 | HCDR1 | NYYMA (SEQ ID NO: 38) | LCDR1 | ERSSGDIGESYVN (SEQ ID NO: 45) |
| | HCDR2 | SISTGGVNTYYRDSVKG (SEQ ID NO: 39) | LCDR2 | ADDQRPS (SEQ ID NO: 46) |
| | HCDR3 | HTTPDYHYGIYFAMDA (SEQ ID NO: 44) | LCDR3 | QSYDSSIDIF (SEQ ID NO: 47) |

By sequence aligning and computer simulation, it was found that the light and heavy chain CDR sequences of the murine antibodies m1803, m1805, m1808, and m1810 have higher homology, and the light and heavy chain CDR sequences of rat antibodies rat 1817 and rat1822 have higher homology. The consensus sequences thereof are shown in the following table:

**Table 3. The consensus sequences of heavy and light chain CDR regions**

| | Heavy chain CDR | | Light chain CDR | |
|---|---|---|---|---|
| Murine antibody | HCDR1 | X₁X₂X₃IX₄, wherein X₁X₂X₃ is selected from DYW, TNG or NYA, and X₄ is selected from E/S (SEQ ID NO: 48) | LCDR1 | RASX₁₇DISNYLN, wherein X₁₇ is selected from L/Q, (SEQ ID NO: 51) |
| | HCDR2 | EIX₅PX₆SX₇X₈TX₉YNEX₃₁FKG, wherein X₅ is selected from L/Y, X₆ is selected from G/R/T, X₇ is selected from T/G, X₈ is selected from N/Y, X₉ is selected from N/Y, X₃₁ is selected from K or N (SEQ ID NO: 49) | LCDR2 | YX₁₈STLHS, wherein X₁₈ is selected from S/T (SEQ ID NO: 52) |
| | HCDR3 | X₃₁X₁₀X₁₁X₁₂X₁₃X₁₄X₁₅X₁₆DYFDY, wherein X₁₀ is selected from I/L/VI, X₁₁ is selected from S/T, X₁₂ is selected from S/T, and X₁₃ is selected from L/V, X₁₄ is selected from M/V/I, X₁₅ is selected from S/G/A, X₁₆ is selected from T/A/V, and X₃₁ is selected from G or S. (SEQ ID NO: 50) | LCDR3 | QQX₁₉NX₂₀X₂₁PX₂₂T, wherein X₁₉ is selected from T/G, X₂₀ is selected from M/T/I, X₂₁ is selected from F/V, and X₂₂ is selected from W/Y (SEQ ID NO: 53) |
| Rat antibody | HCDR1 | NYYMA (SEQ ID NO: 38) | LCDR1 | ERSSGDIGX₂₆SYVN, wherein X₂₆ is selected from E/D (SEQ ID NO: 55) |
| | HCDR2 | SISTGGVNTYYRDSVKG (SEQ ID NO: 39) | LCDR2 | ADX₂₇QRPS, wherein X₂₇ is selected from D/V (SEQ ID NO: 56) |
| | HCDR3 | HTTX₂₃DYX₂₄YGIYFAX₂₅DA, wherein X₂₃ is selected from P/A, X₂₄ is selected from F/H, and X₂₅ is selected from L/M (SEQ ID NO: 54) | LCDR3 | QSYDX₂₈X₂₉IDIX₃₀, wherein X₂₈ is selected from S/T, X₂₉ is selected from S/N, and X₃₀ is selected from F/I (SEQ ID NO: 57) |

### Example 3. Humanization of murine anti-human GCGR antibody

The heavy and light chain variable region germline genes with high homology to murine antibodies were selected as templates by aligning against the IMGT human antibody heavy and light chain variable region germline gene database by MOE software analysis. The CDRs of the murine antibodies were grafted onto the corresponding human template to form a variable region sequence in the order of FR1-CDR1-FR2-CDR2-FR3-CDR3-FR4. As needed, some of the amino acids in the framework sequence were mutated back to the amino acids corresponding to the murine antibody to obtain the humanized anti-GCGR monoclonal antibody. The amino acid residues in the CDR regions were determined and annotated by the Kabat numbering system.

The above murine light and heavy chain variable regions were connected to the human light and heavy chain constant regions respectively to form a chimeric antibody. The chimeric antibody corresponding to clone 1803 was named as ch1803, and other antibodies were named in a similar way.

### 3.1 Humanization of hybridoma clone 1803

(1) Selection of frameworks for humanizing hybridoma clone 1803:
For the murine antibody m1803, the humanization light chain templates were IGKV1-39*01 and hjk4.1, and the humanization heavy chain templates were IGHV1-3*01 and hjh6.1. The humanized variable region sequences are as follows:
hu1803VH-CDR grafting: (SEQ ID NO: 61) 
EVQLVQSGAEVKKPGASVKVSCKASGYTFTDYWIEWVRQAPGQGLEWMGE
ILPGSTYTNYNEKFKGRVTMTRDTSTSTVYMELSSLRSEDTAVYYCARGLSTLMAVD
YFDYWGQGTTVTVSS;
hu1803VL-CDR grafting: (SEQ ID NO: 58)

DIQMTQSPSSLSASVGDRVTITCRASQDISNYLNWYQQKPGKAPKLLIYYSST
LHSGVPSRFSGSGSGTDFTLTISSLQPEDFATYYCQQTNIFPWTFGGGTKVEIK;

Note: Arranged in the order of FR1-CDR1-FR2-CDR2-FR3-CDR3-FR4, the italic sequences represent FRs, and the underlined sequences represent CDRs.
(2) Selection of humanization templates and design of back-mutations for the hybridoma clone 1803 are shown in the Table below:

**Table 4. Template selection and back mutation**

| hu1803 VL | | hu1803 VH | |
|---|---|---|---|
| hu1803 VL.1 | grafting | hu1803 VH.1 | grafting |
| hul803_VL.1A | K42G, P44V, F71Y, Y87F | hu1803_VH.1A | M69F, R71A, V78A |
| hu1803_VL.1B | P44V, F71Y | hu1803_VH.1B | M48I, V67A, M69F, R71A, V78A, A93 S |
| | | hu1803_VH.1C | R38K, M48I, V67A, M69F, R71A, T73P, V78A, A93S |

Note: For example, P44V indicates that P at position 44 according to the Kabat numbering system is mutated back to V. Grafting represents that the murine antibody CDR is directly implanted into the human germline FR region sequence.
(3) The specific humanized sequences of the hybridoma clone 1803 are as follows:
> hu1803_VL.1 (the same as Hu1803VL-CDR grafting): (SEQ ID NO: 58) 
DIQMTQSPSSLSASVGDRVTITCRASQDISNYLNWYQQKPGKAPKLLIYYSSTL
HSGVPSRFSGSGSGTDFTLTISSLQPEDFATYYCQQTNIFPWTFGGGTKVEIK
> hu1803_VL.1A: (SEQ ID NO: 59) 
DIQMTQSPSSLSASVGDRVTITCRASQDISNYLNWYQQKPGGAVKLLIYYSSTL
HSGVPSRFSGSGSGTDYTLTISSLQPEDFATYFCQQTNIFPWTFGGGTKVEIK
> hu1803_VL.1B: (SEQ ID NO: 60) 
DIQMTQSPSSLSASVGDRVTITCRASQDISNYLNWYQQKPGKAVKLLIYYSSTL
HSGVPSRFSGSGSGTDYTLTISSLQPEDFATYYCQQTNIFPWTFGGGTKVEIK
> hu1803_VH.1 (the same as Hu1803VH-CDR grafting): (SEQ ID NO: 61) 
EVQLVQSGAEVKKPGASVKVSCKASGYTFTDYWIEWVRQAPGQGLEWMGEIL
PGSTYTNYNEKFKGRVTMTRDTSTSTVYMELSSLRSEDTAVYYCARGLSTLMAVDY
FDYWGQGTTVTVSS
> hu1803_VH.1A: (SEQ ID NO: 62) 
EVQLVQSGAEVKKPGASVKVSCKASGYTFTDYWIEWVRQAPGQGLEWMGEIL
PGSTYTNYNEKFKGRVTFTADTSTSTAYMELSSLRSEDTAVYYCARGLSTLMAVDY
FDYWGQGTTVTVSS
> hu1803_VH.1B: (SEQ ID NO: 63) 
EVQLVQSGAEVKKPGASVKVSCKASGYTFTDYWIEWVRQAPGQGLEWIGEILP
GSTYTNYNEKFKGRATFTADTSTSTAYMELSSLRSEDTAVYYCSRGLSTLMAVDYF
DYWGQGTTVTVSS
> hu1803_VH.1C: (SEQ ID NO: 64)

EVQLVQSGAEVKKPGASVKVSCKASGYTFTDYWIEWVKQAPGQGLEWIGEILP
GSTYTNYNEKFKGRATFTADPSTSTAYMELSSLRSEDTAVYYCSRGLSTLMAVDYF
DYWGQGTTVTVSS.

(4) The sequence combinations of the humanized antibody light chain variable region and heavy chain variable region of the antibody derived from hybridoma clone 1803 are as follows:

**Table 5: Combinations of the light and heavy chain variable region of various humanized antibodies**

| | hu1803_VL.1 | hu1803_VL.1A | hu1803_VL.1B |
|---|---|---|---|
| hu1803_VH.1 | hu1803-1 | hu1803-5 | hu1803-9 |
| hu1803_VH.1A | hu1803-2 | hu1803-6 | hu1803-10 |
| hu1803_VH.1B | hu1803-3 | hu1803-7 | hu1803-11 |
| hu1803_VH.1C | hu1803-4 | hu1803-8 | hu1803-12 |

The light and heavy chain variable regions of the antibodies indicated in the above table can be connected to the corresponding antibody light and heavy chain constant region to form full-length antibodies. Unless otherwise specified in the present disclosure, for a full-length antibody, the antibody light chain is formed by the light chain variable region connected to the Kappa chain constant region as shown in SEQ ID NO: 73, and the antibody heavy chain is formed by the heavy chain variable region connected to IgG4-AA as shown in SEQ ID NO: 72.

### 3.2 Humanization of hybridoma clone 1810

(1) Selection of frameworks for humanizing hybridoma clone 1810:
For the murine antibody m1810, the humanization light chain templates were IGKV1-39*01 and hJK4.1, and the humanization heavy chain templates were IGHV1-69*02 and hJH4.1. The humanized variable region sequences are as follows:
Hu1810VH-CDR grafting: (SEQ ID NO: 68) 
QVQLVQSGAEVKKPGSSVKVSCKASGGTFSNYAISWVRQAPGQGLEWMGEI
YPTSGNTYYNEKFKGRVTITADKSTSTAYMELSSLRSEDTAVYYCARGVITTVVSTD
YFDYWGQGTLVTVSS
Hu1810VL-CDR grafting: (SEQ ID NO: 65)

DIQMTQSPSSLSASVGDRVTITCRASLDISNYLNWYQQKPGKAPKLLIYYTST
LHSGVPSRFSGSGSGTDFTLTISSLQPEDFATYYCQQGNMVPYTFGGGTKVEIK

Note: Arranged in the order of FR1-CDR1-FR2-CDR2-FR3-CDR3-FR4, the italic sequences represent FRs, and the underlined sequences represent CDRs.
(2) Selection of humanization templates and design of back-mutations for the hybridoma clone 1810 are shown in the Table below:

**Table 6. Template selection and back mutation**

| hu1810_VL | | hu1810_VH | |
|---|---|---|---|
| hul810_VL.1 | grafting | hu1810_VH.1 | grafting |
| hu1810_VL.1A | P44V, P59S, F71Y | hu1810_VH.1A | K73R, A78M, R94S |
| hu1810_VL.1B | Q38L, P44V, P59S, D70E, F71Y | hu1810_VH.1B | M48I, V67A, I69L, K73R, A78M, R94S |
| | | hu1810_VH.1C | R38K, M48I, R66K, V67A, I69L, K73R, A78M, R94S |

Note: For example, P44V indicates that P at position 44 according to the Kabat numbering system is mutated back to V. Grafting represents that the murine antibody CDR is directly implanted into the human germline FR region sequence.
(3) The specific humanized sequences of the hybridoma clone 1810 are as follows:
> hul810_VL.1 (the same as Hu1810VL-CDR grafting): (SEQ ID NO: 65) 
DIQMTQSPSSLSASVGDRVTITCRASLDISNYLNWYQQKPGKAPKLLIYYTST
LHSGVPSRFSGSGSGTDFTLTISSLQPEDFATYYCQQGNMVPYTFGGGTKVEIK
> hu1810_VL.1A: (SEQ ID NO: 66) 
DIQMTQSPSSLSASVGDRVTITCRASLDISNYLNWYQQKPGKAVKLLIYYTST
LHSGVSSRFSGSGSGTDYTLTISSLQPEDFATYYCQQGNMVPYTFGGGTKVEIK
> hu1810_VL.1B: (SEQ ID NO: 67) 
DIQMTQSPSSLSASVGDRVTITCRASLDISNYLNWYQLKPGKAVKLLIYYTST
LHSGVSSRFSGSGSGTEYTLTISSLQPEDFATYYCQQGNMVPYTFGGGTKVEIK
> hu1810_VH.1 (the same as Hu1810VH-CDR grafting): (SEQ ID NO: 68) 
QVQLVQSGAEVKKPGSSVKVSCKASGGTFSNYAISWVRQAPGQGLEWMGEI
YPTSGNTYYNEKFKGRVTITADKSTSTAYMELSSLRSEDTAVYYCARGVITTVVSTD
YFDYWGQGTLVTVSS
> hu1810_VH.1A: (SEQ ID NO: 69) 
QVQLVQSGAEVKKPGSSVKVSCKASGGTFSNYAISWVRQAPGQGLEWMGEI
YPTSGNTYYNEKFKGRVTITADRSTSTMYMELSSLRSEDTAVYYCASGVITTVVSTD
YFDYWGQGTLVTVSS
> hu1810_VH.1B: (SEQ ID NO: 70) 
QVQLVQSGAEVKKPGSSVKVSCKASGGTFSNYAISWVRQAPGQGLEWIGEIY
PTSGNTYYNEKFKGRATLTADRSTSTMYMELSSLRSEDTAVYYCASGVITTVVSTDY
FDYWGQGTLVTVSS
> hu1810_VH.1C: (SEQ ID NO: 71)

QVQLVQSGAEVKKPGSSVKVSCKASGGTFSNYAISWVKQAPGQGLEWIGEI
YPTSGNTYYNEKFKGKATLTADRSTSTMYMELSSLRSEDTAVYYCASGVITTVVSTD
YFDYWGQGTLVTVSS.

(4) The sequence combinations of the humanized antibody light and heavy chain variable region of the hybridoma clone 1810 are as follows:

**Table 7: Combinations of the light and heavy chain variable region of various humanized antibodies**

| | hul810_VL.1 | hu1810_VL.1A | hu1810_VL.1B |
|---|---|---|---|
| hu1810_VH.1 | hu1810-1 | hu1810-5 | hu1810-9 |
| hu1810_VH.1A | hu1810-2 | hu1810-6 | hu1810-10 |
| hu1810_VH.1B | hu1810-3 | hu1810-7 | hu1810-11 |
| hu1810_VH.1C | hu1810-4 | hu1810-8 | hu1810-12 |

The light and heavy chain variable regions of the antibodies indicated in the above table can be connected to the corresponding antibody light and heavy chain constant region to form full-length antibodies. Unless otherwise specified in the present disclosure, for a full-length antibody, the antibody light chain is formed by the light chain variable region connected to the Kappa chain constant region as shown in SEQ ID NO: 73, and the antibody heavy chain is formed by the heavy chain variable region connected to IgG4-AA as shown in SEQ ID NO: 72.

### Example 4: Construction and expression of IgG4-AA format of GCGR chimeric/humanized antibody

Primers were designed, VH/VK gene fragment of each chimeric/humanized antibody was amplified by PCR and then inserted into the expression vector pHr (with a signal peptide and constant region gene (CH1-FC/CL) fragment) via homologous recombination to construct an expression vector for a full-length antibody VH-CH1-FC-pHr/VK-CL-pHr. The IgG4-AA antibody format can be obtained from IgG4 antibody format by simple point mutations. IgG4-AA represents mutations F234A, L235A and S228P. The mutations of F234A and L235A can reduce the binding ability of IgG4-Fc to FcγR, and further reduce ADCC/CDC. S228P indicates that the amino acid S at position 228 in the hinge region of wild-type IgG4 is mutated to P. The mutation at this position can prevent natural IgG4 antibody from mismatches caused by Fab-exchange occurring *in vivo.*

ch1803, ch1805, ch1808, ch1810, ch1817 and ch1822 are chimeric antibodies formed by connecting the animal-derived light and heavy chain variable regions with the human antibody kappa chain and the human antibody IgG4-AA heavy chain constant region, respectively.

The heavy chain constant region sequence of IgG4-AA is as follows: (SEQ ID NO: 72)

ASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVESKYGPP
CPPCPAPEAAGGPSVFLFPPKPKDTLMISRTPEVTCVVVDVSQEDPEVQFNWYV

DGVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNGQ
PENNYKTTPPVLDSDGSFFLYSRLTVDKSRWQEGNVFSCSVMHEALHNHYTQK
SLSLSLGK;

The light chain (kappa chain) constant region sequence of the antibody is as follows: (SEQ ID NO: 73)



;

Exemplary sequences of the constructed antibodies are as follows:
ch1803: antibody format IgG4AA
ch1803 heavy chain sequence: (SEQ ID NO: 74) 
QFQLHQSGAELVKPGASVKLSCKATGYTFTDYWIEWVKQRPGHGLEWI
GEILPGSTYTNYNEKFKGRATFTAEPSSSSAYMQLSGLTTEDSAIYYCSRGLSTL
MAVDYFDYWGQGTTLTVSSASTKGPSVFPLAPCSRSTSESTAALGCLVKDYFPE
PVTVSWNSGALTSGVHTFPAVLQSSGLYSLSSVVTVPSSSLGTKTYTCNVDHKPS
NTKVDKRVESKYGPPCPPCPAPEAAGGPSVFLFPPKPKDTLMISRTPEVTCVVVD
VSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCS
VMHEALHNHYTQKSLSLSLGK;
ch1803 light chain sequence: (SEQ ID NO: 75) 
DIQMTQTTSSLSASLGDRVTINCRASQDISNYLNWYQQKPDGTVKLLIYY
SSTLHSGVPSRFSGSGSGTDYSLTISHLEQEDIATYFCQQTNIFPWTFGGGTKLEIR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC;
hu1803-1: antibody format IgG4AA
hu1803-1: heavy chain sequence: (SEQ ID NO: 76) 
EVQLVQSGAEVKKPGASVKVSCKASGYTFTDYWIEWVRQAPGQGLEW
MGEILPGSTYTNYNEKFKGRVTMTRDTSTSTVYMELSSLRSEDTAVYYCARGLS
TLMAVDYFDYWGQGTTVTVSSASTKGPSVFPLAPCSRSTSESTAALGCLVKDYF
PEPVTVSWNSGALTSGVHTFPAVLQSSGLYSLSSVVTVPSSSLGTKTYTCNVDHK
PSNTKVDKRVESKYGPPCPPCPAPEAAGGPSVFLFPPKPKDTLMISRTPEVTCVV
 
VDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVF
SCSVMHEALHNHYTQKSLSLSLGK;
hu1803-1 light chain sequence: (SEQ ID NO: 77) 
DIQMTQSPSSLSASVGDRVTITCRASQDISNYLNWYQQKPGKAPKLLIYY
SSTLHSGVPSRFSGSGSGTDFTLTISSLQPEDFATYYCQQTNIFPWTFGGGTKVEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC;
hu1803-9 heavy chain sequence: (SEQ ID NO: 78) 
EVQLVQSGAEVKKPGASVKVSCKASGYTFTDYWIEWVRQAPGQGLEW
MGEILPGSTYTNYNEKFKGRVTMTRDTSTSTVYMELSSLRSEDTAVYYCARGLS
TLMAVDYFDYWGQGTTVTVSSASTKGPSVFPLAPCSRSTSESTAALGCLVKDYF
PEPVTVSWNSGALTSGVHTFPAVLQSSGLYSLSSVVTVPSSSLGTKTYTCNVDHK
PSNTKVDKRVESKYGPPCPPCPAPEAAGGPSVFLFPPKPKDTLMISRTPEVTCVV
VDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVF
SCSVMHEALHNHYTQKSLSLSLGK;
hu1803-9 light chain sequence: (SEQ ID NO: 79) 
DIQMTQSPSSLSASVGDRVTITCRASQDISNYLNWYQQKPGKAVKLLIYY
SSTLHSGVPSRFSGSGSGTDYTLTISSLQPEDFATYYCQQTNIFPWTFGGGTKVEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC;
ch1805 heavy chain sequence: (SEQ ID NO: 80) 
EVQLQQSGPELVKPGASVKIPCKTSGYTFTDYNMDWVKQSHGRSLEWI
GSIDPDNGGTIYNQKFKGKATLTVDKSSSTAYMELRSLTSEDTAVYYCTRDYYG
SSSWFAYWGQGTLVTVSAASTKGPSVFPLAPCSRSTSESTAALGCLVKDYFPEPV
TVSWNSGALTSGVHTFPAVLQSSGLYSLSSVVTVPSSSLGTKTYTCNVDHKPSNT
KVDKRVESKYGPPCPPCPAPEAAGGPSVFLFPPKPKDTLMISRTPEVTCVVVDVS
QEDPEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVFSCSVM
 HEALHNHYTQKSLSLSLGK;
ch1805 light chain sequence: (SEQ ID NO: 81) 
DVVMTQSPATLSVTPGDRVSLSCRASQSISDYLHWYQQKSHESPRLLIKY
ASQSISGIPSRFSGSGSGSDFTLSINSVEPEDVGVYYCQNGHSFPYTFGGGTKLEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC;
ch1808 heavy chain sequence: (SEQ ID NO: 82) 
QVQLQQSGAELARPGASVKLSCKASGDTFTTNGISWVKQRIGQGLEWIG
EIYPRSGNTYYNENFKGKATLTADKSSTTAYMELRRLTSEDSAVYFCARSITSVIG
ADYFDYWGQGTTLTVSSASTKGPSVFPLAPCSRSTSESTAALGCLVKDYFPEPVT
VSWNSGALTSGVHTFPAVLQSSGLYSLSSVVTVPSSSLGTKTYTCNVDHKPSNT
KVDKRVESKYGPPCPPCPAPEAAGGPSVFLFPPKPKDTLMISRTPEVTCVVVDVS
QEDPEVQFNWYVDGVEVHNAKTKPREEQFNSTYRVVSVLTVLHQDWLNGKEY
KCKVSNKGLPSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVFSCSVM
HEALHNHYTQKSLSLSLGK;
ch1808 light chain sequence: (SEQ ID NO: 83) 
DIQMTQTTSSLSASLGDRVTISCRASQDISNYLNWYQKKPDGTVKLLIYY
SSTLHSGVPSRFSGSGSGTDYSLTISNLEQEDIATYFCQQGNTFPWTFGGGTKLEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC;
hu1810-12 heavy chain sequence: (SEQ ID NO: 84) 
QVQLVQSGAEVKKPGSSVKVSCKASGGTFSNYAISWVKQAPGQGLEWIGEI
YPTSGNTYYNEKFKGKATLTADRSTSTMYMELSSLRSEDTAVYYCASGVITTVVSTD
YFDYWGQGTLVTVSSASTKGPSVFPLAPCSRSTSESTAALGCLVKDYFPEPVTVS
WNSGALTSGVHTFPAVLQSSGLYSLSSVVTVPSSSLGTKTYTCNVDHKPSNTKV
DKRVESKYGPPCPPCPAPEAAGGPSVFLFPPKPKDTLMISRTPEVTCVVVDVSQE
DPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCSVMH
EALHNHYTQKSLSLSLGK;
hu1810-12 light chain sequence: (SEQ ID NO: 85) DIQMTQSPSSLSASVGDRVTITCRASLDISNYLNWYQLKPGKAVKLLIYYTST 
LHSGVSSRFSGSGSGTEYTLTISSLQPEDFATYYCQQGNMVPYTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC;
ch1817 heavy chain sequence: (SEQ ID NO: 86) 
EVQLVESGGDLVQPGRSMKLSCAASGFTFSNYYMAWVRQAPTKGLEW
VASISTGGVNTYYRDSVKGRFTISRDNAKNNLYLQMDSLRSEETATYYCARHTT
ADYFYGIYFALDAWGQGTSVTVSSASTKGPSVFPLAPCSRSTSESTAALGCLVK
DYFPEPVTVSWNSGALTSGVHTFPAVLQSSGLYSLSSVVTVPSSSLGTKTYTCNV
DHKPSNTKVDKRVESKYGPPCPPCPAPEAAGGPSVFLFPPKPKDTLMISRTPEVT
CVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLT
CLVKGFYPSDIAVEWESNGQPENNYKTTPPVLDSDGSFFLYSRLTVDKSRWQEG
NVFSCSVMHEALHNHYTQKSLSLSLGK;
ch1817 light chain sequence: (SEQ ID NO: 87) 
QFTLTQPKSVSGSLRSTITIPCERSSGDIGDSYVNWYQQHLGRPPLNVIYA
DVQRPSEVSDRFSGSIDSSSNSASLTITNLQMDDEADYFCQSYDTNIDIIFGGGTK
LTVLRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC;
ch1822 heavy chain sequence: (SEQ ID NO: 88) 
EVRLVESGGDFVQPGRSVKLSCAASGFTFSNYYMAWVRQAPTKGLEWV
GSISTGGVNTYYRDSVKGRFTISRDNAESTLYLQMDSLRSEETATYYCARHTTPD
YHYGIYFAMDAWGQGTSVTVSSASTKGPSVFPLAPCSRSTSESTAALGCLVKDY
FPEPVTVSWNSGALTSGVHTFPAVLQSSGLYSLSSVVTVPSSSLGTKTYTCNVDH
KPSNTKVDKRVESKYGPPCPPCPAPEAAGGPSVFLFPPKPKDTLMISRTPEVTCV
VVDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTCL
VKGFYPSDIAVEWESNGQPENNYKTTPPVLDSDGSFFLYSRLTVDKSRWQEGNV
FSCSVMHEALHNHYTQKSLSLSLGK;
ch1822 light chain sequence: (SEQ ID NO: 89) 
QVTLTQPKSVSGSLRSTITIPCERSSGDIGESYVNWYQQHLGRPPINVIYA
DDQRPSEVSDRFSGSIDSSSNSASLTITNLQVDDEADYFCQSYDSSIDIFFGGGTK
LTVLRTVAAPSVFIFPPSDEQLKSGTASVVCLLNNFYPREAKVQWKVDNALQSG
 
NSQESVTEQDSKDSTYSLSSTLTLSKADYEKHKVYACEVTHQGLSSPVTKSFNR
GEC;
Positive control: Dulaglutide is in double-chain form, and the single-chain is GLP-1/hIgG4 Fc (SEQ ID NO: 90)

HGEGTFTSDVSSYLEEQAAKEFIAWLVKGGGGGGGSGGGGSGGGGSAE
SKYGPPCPPCPAPEAAGGPSVFLFPPKPKDTLMISRTPEVTCVVVDVSQEDPEVQ
FNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLG.

### Example 5: Cloning and expression of antibody bispecific protein

Human GLP-1 peptide was used as the GLP-1 receptor agonist part of the bispecific protein, and the GCGR antibody was used as the GCGR antagonist part of the bispecific protein, to form the GLP-1/GCGR antibody bispecific protein.

Studies have found that the new GLP-1/GCGR antibody bispecific protein with amino acid mutation(s) at specific position(s) of GLP-1 (such as Q17E, I23V, K28R or G30R) has an increased *in vitro* stability, wherein the stability is the highest when Q at position 17 of GLP-1A is mutated to E and I at position 23 of GLP-1A is mutated to V(GLP-IC). Non-limiting exemplary sequences of GLP-1 and mutant forms thereof of the present disclosure are as follows:

**Table 8. GLP-1A polypeptide and variant sequences thereof**

| GLP-1 peptide name (relative to the mutation site of GLP-1A) | Sequence | SEQ ID NO |
|---|---|---|
| GLP-1A | HGEGTFTSDVSSYLEEQAAKEFIAWLVKGGG | 91 |
| GLP-1B (Q17E) | HGEGTFTSDVSSYLEEEAAKEFIAWLVKGGG | 92 |
| GLP-1C (Q17E G30R) | HGEGTFTSDVSSYLEEEAAKEFIAWLVKGRG | 93 |
| GLP-1D (Q17E I23V) | HGEGTFTSDVSSYLEEEAAKEFVAWLVKGGG | 94 |
| GLP-1E (Q17E I23V G30R) | HGEGTFTSDVSSYLEEEAAKEFVAWLVKGRG | 95 |
| GLP-1F (Q17E K28R) | HGEGTFTSDVSSYLEEEAAKEFIAWLVRGGG | 96 |
| GLP-1G (Q17E I23V K28R) | HGEGTFTSDVSSYLEEEAAKEFVAWLVRGGG | 97 |
| GLP-1H (Q17E K28R G30R) | HGEGTFTSDVSSYLEEEAAKEFIAWLVRGRG | 98 |
| GLP-1J (Q17E I23V K28R G30R) | HGEGTFTSDVSSYLEEEAAKEFVAWLVRGRG | 99 |

The C-terminal amino acid of the GLP-1 peptide of the present disclosure was connected to the N-terminal amino acid of the GCGR antibody heavy chain through a peptide bond or a linker by using homologous recombination technology. Expression was carried out using the 293 expression system, and the structural models of the bispecific protein as shown in Table 9 were obtained:

**Table 9. Structural models of GLP-1/GCGR bispecific protein**

| Name of the structural model of the bispecific protein | Structural model GLP-1-linker-Ab* |
|---|---|
| Bispecific protein 1 | GLP-1A -linker-Ab |
| Bispecific protein 2 | GLP-1B -linker-Ab |
| Bispecific protein 3 | GLP-1C -linker-Ab |
| Bispecific protein 4 | GLP-1D -linker-Ab |
| Bispecific protein 5 | GLP-1E -linker-Ab |
| Bispecific protein 6 | GLP-1F -linker-Ab |
| Bispecific protein 7 | GLP-1G -linker-Ab |
| Bispecific protein 8 | GLP-1H -linker-Ab |
| Bispecific protein 9 | GLP-1-I -linker-Ab |

| | |
|---|---|
| *Note: Ab is the GCGR antibody as described in this disclosure. The GLP-1 peptide can be linked to the amino terminus of the heavy chain variable region of the GCGR antibody or to the amino terminus of the light chain variable region of the GCGR antibody. It has been verified by experiments that the bispecific protein has a more favorable stability when the GLP-1 peptide is linked to the amino terminus of the GCGR antibody heavy chain variable region, instead of to the amino terminus of the GCGR antibody light chain variable region. A schematic diagram of the bispecific protein structure in some embodiments where the GLP-1 peptide of the present disclosure is linked to the heavy chain variable region of the GCGR full-length antibody is shown in Figure 1. | |

The following proteins are formed by connecting different GLP-1 peptides to the heavy chain amino acids of different antibodies via linkers (e.g.(GGGGS)₃):

**Table 10. Bispecific protein sequences**

| Name of the Bispecific protein | The first chain (comprising the heavy chain part (SEQ ID NO)): GLP-1 peptide-linker-antibody heavy chain (from N to C) | The second chain (light chain part) |
|---|---|---|
| hu1803-9A | HGEGTFTSDVSSYLEEQAAKEFIAWLVKGGGGGG GSGGGGSGGGGSEVQLVQSGAEVKKPGASVKVSC KASGYTFTDYWIEWVRQAPGQGLEWMGEILPGST YTNYNEKFKGRVTMTRDTSTSTVYMELSSLRSEDT AVYYCARGLSTLMAVDYFDYWGQGTTVTVSSAST KGPSVFPLAPCSRSTSESTAALGCLVKDYFPEPVTV SWNSGALTSGVHTFPAVLQSSGLYSLSSVVTVPSSS LGTKTYTCNVDHKPSNTKVDKRVESKYGPPCPPCP APEAAGGPSVFLFPPKPKDTLMISRTPEVTCVVVD VSQEDPEVQFNWYVDGVEVHNAKTKPREEQFNST YRVVSVLTVLHQDWLNGKEYKCKVSNKGLPSSIE KTISKAKGQPREPQVYTLPPSQEEMTKNQVSLTCL VKGFYPSDIAVEWESNGQPENNYKTTPPVLDSDGS FFLYSRLTVDKSRWQEGNVFSCSVMHEALHNHYT QKSLSLSLGK(SEQ ID NO: 100) | DIQMTQSP SSLSASVG DRVTITCR ASQDISNY LNWYQQK PGKAVKLL IYYSSTLHS GVPSRFSG SGSGTDYT LTISSLQPE DFATYYCQ QTNIFPWT FGGGTKVE IKRTVAAPS VFIFPPSDE QLKSGTAS VVCLLNNF YPREAKV QWKVDNA LQSGNSQE SVTEQDSK DSTYSLSS TLTLSKAD YEKHKVY ACEVTHQ GLSSPVTK SFNRGEC (SEQ ID NO: 79) |
| hu1803-9B | HGEGTFTSDVSSYLEEEAAKEFIAWLVKGGGGGGG SGGGGSGGGGSEVQLVQSGAEVKKPGASVKVSCK ASGYTFTDYWIEWVRQAPGQGLEWMGEILPGSTY TNYNEKFKGRVTMTRDTSTSTVYMELSSLRSEDTA VYYCARGLSTLMAVDYFDYWGQGTTVTVSSASTK GPSVFPLAPCSRSTSESTAALGCLVKDYFPEPVTVS WNSGALTSGVHTFPAVLQSSGLYSLSSVVTVPSSSL GTKTYTCNVDHKPSNTKVDKRVESKYGPPCPPCPA PEAAGGPSVFLFPPKPKDTLMISRTPEVTCVVVDVS QEDPEVQFNWYVDGVEVHNAKTKPREEQFNSTYR VVSVLTVLHQDWLNGKEYKCKVSNKGLPSSIEKTI SKAKGQPREPQVYTLPPSQEEMTKNQVSLTCLVKG FYPSDIAVEWESNGQPENNYKTTPPVLDSDGSFFLY SRLTVDKSRWQEGNVFSCSVMHEALHNHYTQKSL SLSLGK(SEQ ID NO: 101) | |
| hu1803-9C | HGEGTFTSDVSSYLEEEAAKEFIAWLVKGRGGGGG GSGGGGSGGGGSEVQLVQSGAEVKKPGASVKVSC | |
| | KASGYTFTDYWIEWVRQAPGQGLEWMGEILPGST YTNYNEKFKGRVTMTRDTSTSTVYMELSSLRSEDT AVYYCARGLSTLMAVDYFDYWGQGTTVTVSSAST KGPSVFPLAPCSRSTSESTAALGCLVKDYFPEPVTV SWNSGALTSGVHTFPAVLQSSGLYSLSSVVTVPSSS LGTKTYTCNVDHKPSNTKVDKRVESKYGPPCPPCP APEAAGGPSVFLFPPKPKDTLMISRTPEVTCVVVD VSQEDPEVQFNWYVDGVEVHNAKTKPREEQFNST YRVVSVLTVLHQDWLNGKEYKCKVSNKGLPSSIE KTISKAKGQPREPQVYTLPPSQEEMTKNQVSLTCL VKGFYPSDIAVEWESNGQPENNYKTTPPVLDSDGS FFLYSRLTVDKSRWQEGNVFSCSVMHEALHNHYT QKSLSLSLGK(SEQ ID NO: 102) | |
| hu1803-9D | HGEGTFTSDVSSYLEEEAAKEFVAWLVKGGGGGG GSGGGGSGGGGSEVQLVQSGAEVKKPGASVKVSC KASGYTFTDYWIEWVRQAPGQGLEWMGEILPGST YTNYNEKFKGRVTMTRDTSTSTVYMELSSLRSEDT AVYYCARGLSTLMAVDYFDYWGQGTTVTVSSAST KGPSVFPLAPCSRSTSESTAALGCLVKDYFPEPVTV SWNSGALTSGVHTFPAVLQSSGLYSLSSVVTVPSSS LGTKTYTCNVDHKPSNTKVDKRVESKYGPPCPPCP APEAAGGPSVFLFPPKPKDTLMISRTPEVTCVVVD VSQEDPEVQFNWYVDGVEVHNAKTKPREEQFNST YRVVSVLTVLHQDWLNGKEYKCKVSNKGLPSSIE KTISKAKGQPREPQVYTLPPSQEEMTKNQVSLTCL VKGFYPSDIAVEWESNGQPENNYKTTPPVLDSDGS FFLYSRLTVDKSRWQEGNVFSCSVMHEALHNHYT QKSLSLSLGK(SEQ ID NO: 103) | |
| hu1803-9E | HGEGTFTSDVSSYLEEEAAKEFVAWLVKGRGGGG GGSGGGGSGGGGSEVQLVQSGAEVKKPGASVKVS CKASGYTFTDYWIEWVRQAPGQGLEWMGEILPGS TYTNYNEKFKGRVTMTRDTSTSTVYMELSSLRSED TAVYYCARGLSTLMAVDYFDYWGQGTTVTVSSAS TKGPSVFPLAPCSRSTSESTAALGCLVKDYFPEPVT VSWNSGALTSGVHTFPAVLQSSGLYSLSSVVTVPSS SLGTKTYTCNVDHKPSNTKVDKRVESKYGPPCPPC PAPEAAGGPSVFLFPPKPKDTLMISRTPEVTCVVVD VSQEDPEVQFNWYVDGVEVHNAKTKPREEQFNST YRVVSVLTVLHQDWLNGKEYKCKVSNKGLPSSIE KTISKAKGQPREPQVYTLPPSQEEMTKNQVSLTCL | |
| | VKGFYPSDIAVEWESNGQPENNYKTTPPVLDSDGS FFLYSRLTVDKSRWQEGNVFSCSVMHEALHNHYT QKSLSLSLGK(SEQ ID NO: 104) | |
| hu1803-9F | HGEGTFTSDVSSYLEEEAAKEFIAWLVRGGGGGGG SGGGGSGGGGSEVQLVQSGAEVKKPGASVKVSCK ASGYTFTDYWIEWVRQAPGQGLEWMGEILPGSTY TNYNEKFKGRVTMTRDTSTSTVYMELSSLRSEDTA VYYCARGLSTLMAVDYFDYWGQGTTVTVSSASTK GPSVFPLAPCSRSTSESTAALGCLVKDYFPEPVTVS WNSGALTSGVHTFPAVLQSSGLYSLSSVVTVPSSSL GTKTYTCNVDHKPSNTKVDKRVESKYGPPCPPCPA PEAAGGPSVFLFPPKPKDTLMISRTPEVTCVVVDVS QEDPEVQFNWYVDGVEVHNAKTKPREEQFNSTYR VVSVLTVLHQDWLNGKEYKCKVSNKGLPSSIEKTI SKAKGQPREPQVYTLPPSQEEMTKNQVSLTCLVKG FYPSDIAVEWESNGQPENNYKTTPPVLDSDGSFFLY SRLTVDKSRWQEGNVFSCSVMHEALHNHYTQKSL SLSLGK(SEQ ID NO: 105) | |
| hu1803-9G | HGEGTFTSDVSSYLEEEAAKEFVAWLVRGGGGGG GSGGGGSGGGGSEVQLVQSGAEVKKPGASVKVSC KASGYTFTDYWIEWVRQAPGQGLEWMGEILPGST YTNYNEKFKGRVTMTRDTSTSTVYMELSSLRSEDT AVYYCARGLSTLMAVDYFDYWGQGTTVTVSSAST KGPSVFPLAPCSRSTSESTAALGCLVKDYFPEPVTV SWNSGALTSGVHTFPAVLQSSGLYSLSSVVTVPSSS LGTKTYTCNVDHKPSNTKVDKRVESKYGPPCPPCP APEAAGGPSVFLFPPKPKDTLMISRTPEVTCVVVD VSQEDPEVQFNWYVDGVEVHNAKTKPREEQFNST YRVVSVLTVLHQDWLNGKEYKCKVSNKGLPSSIE KTISKAKGQPREPQVYTLPPSQEEMTKNQVSLTCL VKGFYPSDIAVEWESNGQPENNYKTTPPVLDSDGS FFLYSRLTVDKSRWQEGNVFSCSVMHEALHNHYT QKSLSLSLGK(SEQ ID NO: 106) | |
| hu1803-9H | HGEGTFTSDVSSYLEEEAAKEFIAWLVRGRGGGGG SGGGGSGGGGSEVQLVQSGAEVKKPGASVKVSCK ASGYTFTDYWIEWVRQAPGQGLEWMGEILPGSTY TNYNEKFKGRVTMTRDTSTSTVYMELSSLRSEDTA VYYCARGLSTLMAVDYFDYWGQGTTVTVSSASTK GPSVFPLAPCSRSTSESTAALGCLVKDYFPEPVTVS WNSGALTSGVHTFPAVLQSSGLYSLSSVVTVPSSSL | |
| | GTKTYTCNVDHKPSNTKVDKRVESKYGPPCPPCPA PEAAGGPSVFLFPPKPKDTLMISRTPEVTCVVVDVS QEDPEVQFNWYVDGVEVHNAKTKPREEQFNSTYR VVSVLTVLHQDWLNGKEYKCKVSNKGLPSSIEKTI SKAKGQPREPQVYTLPPSQEEMTKNQVSLTCLVKG FYPSDIAVEWESNGQPENNYKTTPPVLDSDGSFFLY SRLTVDKSRWQEGNVFSCSVMHEALHNHYTQKSL SLSLGK(SEQ ID NO: 107) | |
| hu1803-9J | HGEGTFTSDVSSYLEEEAAKEFVAWLVRGRGGGG GSGGGGSGGGGSEVQLVQSGAEVKKPGASVKVSC KASGYTFTDYWIEWVRQAPGQGLEWMGEILPGST YTNYNEKFKGRVTMTRDTSTSTVYMELSSLRSEDT AVYYCARGLSTLMAVDYFDYWGQGTTVTVSSAST KGPSVFPLAPCSRSTSESTAALGCLVKDYFPEPVTV SWNSGALTSGVHTFPAVLQSSGLYSLSSVVTVPSSS LGTKTYTCNVDHKPSNTKVDKRVESKYGPPCPPCP APEAAGGPSVFLFPPKPKDTLMISRTPEVTCVVVD VSQEDPEVQFNWYVDGVEVHNAKTKPREEQFNST YRVVSVLTVLHQDWLNGKEYKCKVSNKGLPSSIE KTISKAKGQPREPQVYTLPPSQEEMTKNQVSLTCL VKGFYPSDIAVEWESNGQPENNYKTTPPVLDSDGS FFLYSRLTVDKSRWQEGNVFSCSVMHEALHNHYT QKSLSLSLGK(SEQ ID NO: 108) | |
| ch1805-D | HGEGTFTSDVSSYLEEEAAKEFVAWLVKGGGGGG GSGGGGSGGGGSEVQLQQSGPELVKPGASVKIPCK TSGYTFTDYNMDWVKQSHGRSLEWIGSIDPDNGG TIYNQKFKGKATLTVDKSSSTAYMELRSLTSEDTAV YYCTRDYYGSSSWFAYWGQGTLVTVSAASTKGPS VFPLAPCSRSTSESTAALGCLVKDYFPEPVTVSWNS GALTSGVHTFPAVLQSSGLYSLSSVVTVPSSSLGTK TYTCNVDHKPSNTKVDKRVESKYGPPCPPCPAPEA AGGPSVFLFPPKPKDTLMISRTPEVTCVVVDVSQE DPEVQFNWYVDGVEVHNAKTKPREEQFNSTYRVV SVLTVLHQDWLNGKEYKCKVSNKGLPSSIEKTISK AKGQPREPQVYTLPPSQEEMTKNQVSLTCLVKGFY PSDIAVEWESNGQPENNYKTTPPVLDSDGSFFLYSR LTVDKSRWQEGNVFSCSVMHEALHNHYTQKSLSL SLGK(SEQ ID NO: 109) | ch1805 light chain sequence: (SEQ ID NO: 81) |
| ch1808-D | HGEGTFTSDVSSYLEEEAAKEFVAWLVKGGGGGG GSGGGGSGGGGSQVQLQQSGAELARPGASVKLSC | ch1808 light chain |
| | KASGDTFTTNGISWVKQRIGQGLEWIGEIYPRSGN TYYNENFKGKATLTADKSSTTAYMELRRLTSEDSA VYFCARSITSVIGADYFDYWGQGTTLTVSSASTKG PSVFPLAPCSRSTSESTAALGCLVKDYFPEPVTVSW NSGALTSGVHTFPAVLQSSGLYSLSSVVTVPSSSLG TKTYTCNVDHKPSNTKVDKRVESKYGPPCPPCPAP EAAGGPSVFLFPPKPKDTLMISRTPEVTCVVVDVS QEDPEVQFNWYVDGVEVHNAKTKPREEQFNSTYR VVSVLTVLHQDWLNGKEYKCKVSNKGLPSSIEKTI SKAKGQPREPQVYTLPPSQEEMTKNQVSLTCLVKG FYPSDIAVEWESNGQPENNYKTTPPVLDSDGSFFLY SRLTVDKSRWQEGNVFSCSVMHEALHNHYTQKSL SLSLGK(SEQ ID NO: 110) | sequence: (SEQ ID NO: 83) |
| hu1810-12D | HGEGTFTSDVSSYLEEEAAKEFVAWLVKGGGGGG GSGGGGSGGGGSQVQLVQSGAEVKKPGSSVKVSCKA SGGTFSNYAISWVKQAPGQGLEWIGEIYPTSGNTYY NEKFKGKATLTADRSTSTMYMELSSLRSEDTAVYYCAS GVITTVVSTDYFDYWGQGTLVTVSSASTKGPSVFPL APCSRSTSESTAALGCLVKDYFPEPVTVSWNSGALT SGVHTFPAVLQSSGLYSLSSVVTVPSSSLGTKTYTC NVDHKPSNTKVDKRVESKYGPPCPPCPAPEAAGGP SVFLFPPKPKDTLMISRTPEVTCVVVDVSQEDPEVQ FNWYVDGVEVHNAKTKPREEQFNSTYRVVSVLTV LHQDWLNGKEYKCKVSNKGLPSSIEKTISKAKGQP REPQVYTLPPSQEEMTKNQVSLTCLVKGFYPSDIAV EWESNGQPENNYKTTPPVLDSDGSFFLYSRLTVDK SRWQEGNVFSCSVMHEALHNHYTQKSLSLSLGK(S EQ ID NO: 111) | hu1810-12 light chain sequence: (SEQ ID NO: 85) |
| ch1817-D | HGEGTFTSDVSSYLEEEAAKEFVAWLVKGGGGGG GSGGGGSGGGGSEVQLVESGGDLVQPGRSMKLSC AASGFTFSNYYMAWVRQAPTKGLEWVASISTGGV NTYYRDSVKGRFTISRDNAKNNLYLQMDSLRSEET ATYYCARHTTADYFYGIYFALDAWGQGTSVTVSS ASTKGPSVFPLAPCSRSTSESTAALGCLVKDYFPEP VTVSWNSGALTSGVHTFPAVLQSSGLYSLSSVVTVP SSSLGTKTYTCNVDHKPSNTKVDKRVESKYGPPCP PCPAPEAAGGPSVFLFPPKPKDTLMISRTPEVTCVV VDVSQEDPEVQFNWYVDGVEVHNAKTKPREEQF NSTYRVVSVLTVLHQDWLNGKEYKCKVSNKGLPS SIEKTISKAKGQPREPQVYTLPPSQEEMTKNQVSLT | ch1817 light chain sequence: (SEQ ID NO: 87) |
| | CLVKGFYPSDIAVEWESNGQPENNYKTTPPVLDSD GSFFLYSRLTVDKSRWQEGNVFSCSVMHEALHNH YTQKSLSLSLGK(SEQ ID NO: 112) | |
| ch1822-D | HGEGTFTSDVSSYLEEEAAKEFVAWLVKGGGGGG GSGGGGSGGGGSEVRLVESGGDFVQPGRSVKLSC AASGFTFSNYYMAWVRQAPTKGLEWVGSISTGGV NTYYRDSVKGRFTISRDNAESTLYLQMDSLRSEET ATYYCARHTTPDYHYGIYFAMDAWGQGTSVTVSS ASTKGPSVFPLAPCSRSTSESTAALGCLVKDYFPEP VTVSWNSGALTSGVHTFPAVLQSSGLYSLSSVVTVP SSSLGTKTYTCNVDHKPSNTKVDKRVESKYGPPCP PCPAPEAAGGPSVFLFPPKPKDTLMISRTPEVTCVV VDVSQEDPEVQFNWYVDGVEVHNAKTKPREEQF NSTYRVVSVLTVLHQDWLNGKEYKCKVSNKGLPS SIEKTISKAKGQPREPQVYTLPPSQEEMTKNQVSLT CLVKGFYPSDIAVEWESNGQPENNYKTTPPVLDSD GSFFLYSRLTVDKSRWQEGNVFSCSVMHEALHNH YTQKSLSLSLGK(SEQ ID NO: 113) | ch1822 light chain sequence: (SEQ ID NO: 89) |

Herein, preferably the linker (GGGGS)₃ can be used as a linker in the bispecific protein. In other embodiments, a peptide bond or other linkers conventionally used for connection of polypeptides can also be used. The linkers for the bispecific protein of the present disclosure are not limited to the use of (GGGGS)₃. Nucleotide sequences encoding the GLP-1, GCGR antibodies, and linker protein fragment ((GGGGS)₃) were obtained by conventional technical means in the art. The C-terminal nucleotide of GLP-1 was linked to the N-terminal nucleotide of the GCGR antibody via a linker protein by homologous recombination technology and cloned into Phr-BsmbI vector. The recombinant GLP-1/GCGR antibody bispecific protein was expressed in 293 cells and purified by the method of Example 6. The purified protein can be used in each of the following examples.

### Example 6: Purification of antibody bispecific protein

The cell culture medium was centrifuged at high speed, the supernatant was collected, and was subjected to affinity chromatography as the first step of purification. The chromatographic medium was Protein A that interacts with Fc or a derivative filler, such as Mabselect, GE. The equilibration buffer was 1×PBS (137mmol/L NaCl, 2.7mmol/L KCl, 10mmol/L Na₂HPO₄, 2mmol/L KH₂PO₄, pH 7.4). After equilibrating the column with 5 × column volume, the cell culture supernatant was loaded for binding, and the flow rate was controlled so that the retention time of the sample on the column is ≥1min. After loading the sample, the column was washed with 1×PBS (pH 7.4) until the A280 UV absorption dropped to the baseline. Then the column was washed with 0.1M glycine (pH3.0) elution buffer, the elution peak was collected based on the A280 UV absorption peak, and the collected elution sample was neutralized with 1M Tris (pH8.5).

The neutralized elution sample was ultrafiltered and concentrated, and then subjected to size exclusion chromatography, wherein the buffer was 1×PBS, the chromatography column was XK26/60 Superdex200 (GE), the flow rate was controlled at 4ml/min, and the loading volume was less than 5ml. The target protein peak was pooled based on A280 UV absorption. The collected protein was identified by SEC-HPLC and the purity was greater than 95%. After LC-MS identification, the desired protein was aliquoted for use. The GLP-1/GCGR antibody bispecific proteins were obtained.

### Test Examples

### Test Example 1: ELISA assay of the binding of GCGR chimeric antibody to human, mouse and cynomolgus monkey GCGR

The binding ability of anti-GCGR antibodies was tested by binding assay of the antibody to CHO cells overexpressing GCGR. The human, mouse, and cynomolgus monkey GCGR full-length plasmids were transferred into CHO cells by transfection method, respectively. The expression levels of GCGR were detected after two weeks of pressure screening, After the GCGR-overexpressing cells were fixed to the bottom of 96-well plate, the antibodies were added and the signal strength was used to determine the binding activity of the antibody to GCGR overexpressing CHO cells. The binding abilities of the antibody to the three species of GCGRs were detected with the same binding assay. As an example, the binding of the GCGR antibodies to human GCGR was detected by an assay specifically described as follows:

The cells were seeded in a 96-well plate at a density of 0.9-1.0× 10⁶/ml, 100 µl/well and cultured overnight. The supernatant was discarded, the plate was washed three times with PBS, 100µl/well of cell immune fixed solution (Beyotime, Cat No. P0098) was added and fixed for an hour at room temperature, and washed four times with PBS. The liquid was discarded, 200µl/well blocking solution (5% skim milk (BD skim milk, Cat Wo.232100) diluted with PBS) was added, and incubated in an incubator at 37°C for 3 hours for blocking. After the blocking was finished, the blocking solution was discarded, the plate was washed 3 times with PBST buffer (PBS comprising 0.05% tweenW-20, pH7.4), 50µl/well of various concentrations of each of the test antibodies (antibodies purified from hybridoma, chimeric antibodies or humanized antibodies) diluted with sample dilution solution were added, and incubated in an incubator at 37°C for 2 hours. After the incubation was finished, the plate was washed 3 times with PBST, 50µl/well of HRP-labeled goat anti-mouse secondary antibody (JacksoW ImmuWo Research, Cat No. 115-035-003) or goat anti-human secondary antibody (JacksoW ImmuWo Research, Cat Wo. 109-035-003) diluted with sample dilution solution was added, and incubated at 37°C for 1 hour. The plate was washed 3 times with PBST, 50µl/well of TMB chromogenic substrate (KPL, Cat Wo. 52-00-03) was added, incubated at room temperature for 10 min, and 50µl/well of 1M H₂SO4 was added to stop the reaction. The absorbance value was read by a microplate reader (Thermo scientific Multiskan MK3) at a wavelength of 450 nm, and the data was analyzed with GraphPad Prism 5. The binding of GCGR chimeric antibodies to GCGR-overepressing CHO cells was calculated as EC50 value.

**Table 11. The binding activity of chimeric antibody**

| Antibody EC50 (nM) | ch1803 | ch1805 | ch1808 | ch1810 | ch1817 | ch1822 |
|---|---|---|---|---|---|---|
| Human GCGR | 0.1167 | 0.2969 | 0.6769 | 0.1168 | 0.1926 | 0.3834 |
| Mouse GCGR | 0.7183 | 0.3428 | 1.019 | 0.4415 | 0.1638 | 0.5681 |
| Cynomolgus monkey GCGR | 0.2128 | 0.3585 | 0.5356 | 0.2480 | 0.2968 | 0.4280 |

The results show that the chimeric antibodies ch1803, ch1805, ch1808, ch1810, ch1817 and ch1822 all have favorable binding activity to human GCGR on cell surface, and also have favorable cross-affinity activity with mouse and cynomolgus monkey GCGR.

### Test Example 2: Assay of GCGR chimeric antibody blocking the binding of GCGR ligand to GCGR

### 1. Purpose:

The antagonistic activities of the GCGR chimeric antibodies were evaluated by an assay of GCGR chimeric antibody blocking the binding of GCGR ligand (glucagon) to GCGR.

### 2. Test principle:

The expression of luciferase gene (luciferase) downstream of CRE can be induced by the binding of cAMP to CRE. The change in luorescence signal emitted by the binding of luciferase to its substrate can reflect the inhibition efficiency. CRE was cloned upstream of the luciferase gene, and was co-transfected into CHO-K1 cells with a plasmid comprising the GCGR gene. Monoclonal cells expressing both CRE and GCGR were selected. GLP-1/GCGR antibody bispecific protein can compete with glucagon to bind to GCGR, block the signal transduction downstream of the GCGR, and affect the expression of downstream cAMP. The antagonistic activities of GLP-1/GCGR antibody bispecific proteins against GCGR can be evaluated by measuring the changes in fluorescence signal.

### 3. Test samples:

Chimeric antibodies ch1803, ch1805, ch1808, ch1810, ch1817 and ch1822.

### 4. Experimental procedures

a. Cell suspension was prepared with fresh cell culture medium, and was added into a 96-well cell culture plate of 80µl culture system, 20,000 cells/well, and incubated at 5% CO2, 37°C for 16 hours.
b. 10µl of each of the prepared proteins to be tested was added into each well, 10µl of the prepared glucagon was then added, and incubated at 5% CO2, 37°C for 5 hours.
c. 100µl of detection solution ONE Glo (Promega) was added to each well, and placed in the dark at room temperature for 7 minutes.
d. The fluorescence was detected on the microplate reader Victor3, and the IC50 value and the blocking efficiency Imax of the GCGR chimeric antibody fusion to block the binding of GCGR ligand (glucagon) to human, mouse and cynomolgus GCGRs were calculated.

**Table 12. The antagonistic activity of chimeric antibody**

| Antibody | Human GCGR | | Mouse GCGR | |
|---|---|---|---|---|
| | IC50 (nM) | Imax | IC50 (nM) | Imax (%) |
| ch1803 | 79.3 | 100% | 24.7 | 93% |
| ch1805 | 95.03 | 91% | 18 | 100% |
| ch1808 | 105.2 | 98% | 26.6 | 100% |
| ch1810 | 72.46 | 100% | Not detected | Not detected |
| ch1817 | 59.8 | 83% | 12.8 | 96% |
| ch1822 | 62.48 | 59% | 15.96 | 87% |

### Test Example 3: Blocking assay of GCGR humanized antibody against the binding of GCGR ligand to GCGR

The antagonistic activities of the GCGR antibodies were evaluated by assay of anti-GCGR antibody blocking the binding of GCGR ligand (glucagon) to GCGR. The experimental principle and steps are the same as Test Example 2, and the results are shown in the following table:

**Table 13. Antagonistic activity of humanized antibody**

| Antibody | Human GCGR | | Antibody | Human GCGR | |
|---|---|---|---|---|---|
| | IC50 (nM) | Imax | | IC50 (nM) | Imax |
| hu1803-1 | 191.1 | 91.8% | hu1810-1 | 189.1 | 83.2% |
| hu1803-2 | 137.12 | 98.5% | hu1810-2 | 105.9 | 100% |
| hu1803-3 | 109.55 | 96.7% | hu1810-3 | 192.2 | 95.42% |
| hu1803-4 | 139.75 | 100% | hu1810-4 | 137.5 | 99.1% |
| hu1803-5 | 79.03 | 100% | hu1810-5 | 216.4 | 86.39% |
| hu1803-6 | 77.48 | 100% | hu1810-6 | 156.8 | 100% |
| hu1803-7 | 73.04 | 100% | hu1810-7 | 119.3 | 100% |
| hu1803-8 | 85.92 | 100% | hu1810-8 | 132.9 | 100% |
| hu1803-9 | 78.39 | 100% | hu1810-9 | 124.7 | 90.4% |
| hu1803-10 | 76.67 | 100% | hu1810-10 | 95.56 | 97.42% |
| hu1803-11 | 88.72 | 100% | hu1810-11 | 91.32 | 99.61% |
| hu1803-12 | 86.92 | 100% | hu1810-12 | 75.91 | 99.65% |

### In vitro biological evaluation

### Test Example 4: Stability of GLP-1/GCGR antibody bispecific protein in PBS

200 µg of the antibody bispecific protein to be tested was dissolved in 1ml 1×PBS (pH 7.4) and stored in a 37°C incubator; samples were taken at days 0 and 14 respectively, and maintenance of the intact heavy chain was detected by LC-MS with Agilent 6530 Q-TOF. The results are shown in Table 14 below. Each of the GLP-1 peptide bispecific proteins with mutations has a greatly increased stability compared to the bispecific protein comprising GLP-1A (SEQ ID NO: 91), and hu1803-9B, hu1803-9D and hu1803-9G show better stability.

**Table 14: Detection of stability of GLP-1/GCGR antibody bispecific protein**

| Protein | Day 14 |
|---|---|
| hu1803-9A | 70.12% |
| hu1803-9B | ≥97% |
| hu1803-9C | 93.98% |
| hu1803-9D | ≥97% |
| hu1803-9E | 94.15% |
| hu1803-9F | 94.65% |
| hu1803-9G | ≥97% |

### Test Example 5: Blocking assay of cell-based GCGR binding

The experimental principle and steps are the same as Test Example 2.

### Test samples:

1) GCGR antibodies (hu1803-9, hu1810-12)
2) Bispecific proteins: hu1803-9B and hul803-9D.

**Table 15. Antagonistic activity of bispecific protein against GCGR**

| Protein | Antagonistic activity against human GCGR | |
|---|---|---|
| | IC50 (nM) | Imax (%) |
| hu1803-9 | 54 | 100 |
| hu1803-9B | 67 | 100 |
| hu1803-9D | 79 | 100 |

**Table 16. Antagonistic activity of humanized antibody against mouse and cynomolgus monkey GCGR**

| Antibody | Mouse GCGR | | Cynomolgus monkey GCGR | |
|---|---|---|---|---|
| | IC 50 (nM) | Imax (%) | IC 50 (nM) | Imax (%) |
| hu1803-9 | 11.22 | 100% | 220 | 100% |
| hu1810-12 | 17.54 | 97% | 886 | 99% |

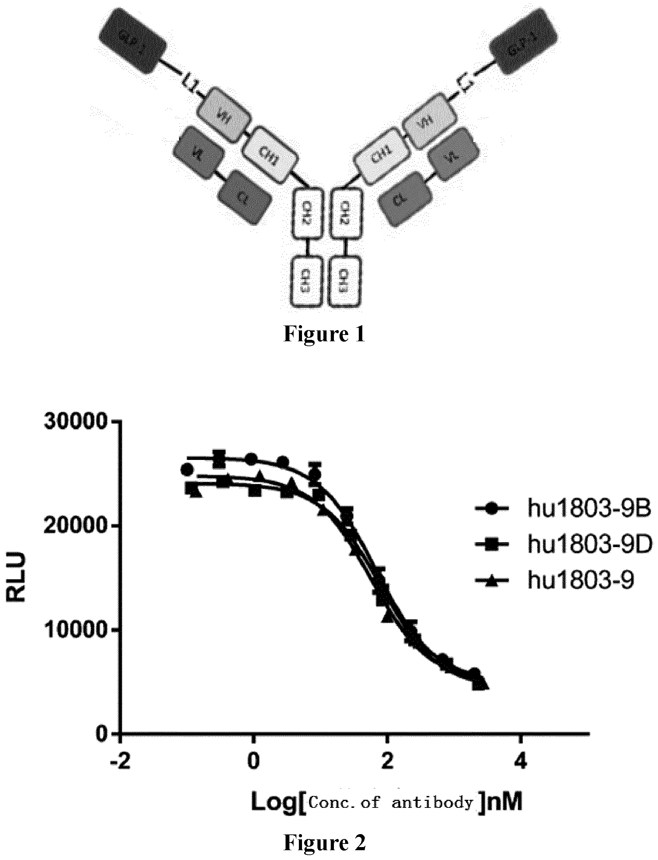

Figure 2 and Table 15 show that both hu1803-9B and hu1803-9D can completely inhibit the antagonistic activity of GCGR, and have comparable efficacy and IC50 (the concentration required to inhibit 50% of the maximum activity) to GCGR monoclonal antibody, indicating that both hu1803-9B and hu1803-9D retain the complete biological activity of the GCGR antibody part. In addition to the verified antagonistic activities of hu1803-9 and the bispecific protein comprising hu1803-9 in inhibiting human GCGR, it was further proved (see Table 16) that both hu1803-9 and hu1810-12 have the antagonistic activity to inhibit mouse and cynomolgus GCGR.

### Test Example 6: Activation assay of cell-based GLP-1R binding

### 1. Purpose:

The purpose is to evaluate the activating activity of the GLP-1 part of the GLP-1/GCGR antibody bispecific protein on GLP-1R.

### 2. Test principle:

The expression of luciferase gene downstream of CRE can be induced by the binding of cAMP to CRE. The change in fluorescence signal emitted by the binding of luciferase to its substrate can reflect the inhibition efficiency. CRE was cloned upstream of the luciferase gene, and was co-transfected into CHO-K1 cells with a plasmid comprising the GLP-1R gene. Monoclonal cells highly expressing both CRE and GLP-1R were selected. Both GLP-1/GCGR antibody bispecific protein and Dulaglutide (a positive control) can bind to GLP-1R, activate the signal transduction downstream of the GLP-1R, and stimulate the expression of downstream cAMP. The agnostic activities of GLP-1/GCGR antibody bispecific proteins for GLP-1R can be evaluated by measuring the changes in fluorescence signal.

### 3. Test samples:

1) Positive control: Dulaglutide
2) hu1803-9B, hul803-9D.

### 4. Experimental procedures

a. Cell suspension was prepared with fresh cell culture medium, and was added into a 96-well cell culture plate of 90µl culture system, 25000 cells/well, and incubated at 5% CO₂, 37°C for 16 hours.
b. 10µl of each of the prepared proteins to be tested was added into each well, and incubated at 5% CO2, 37°C for 5 hours.
c. 100µl of detection solution ONE Glo (Promega) was added to each well, and placed in the dark at room temperature for 7 minutes.
d. The fluorescence was detected on the microplate reader Victor3, and the EC50 values of the GLP-1/GCGR antibody bispecific proteins for activating GLP-1R by binding were calculated.

**Table 17. The activating activity of bispecific proteins on GLP-1R**

| Protein | EC50 (nM) | Emax (%) |
|---|---|---|
| Dulaglutide | 0.21 | 100 |
| hu1803-9B | 0.22 | 100 |
| hu1803-9D | 0.32 | 100 |

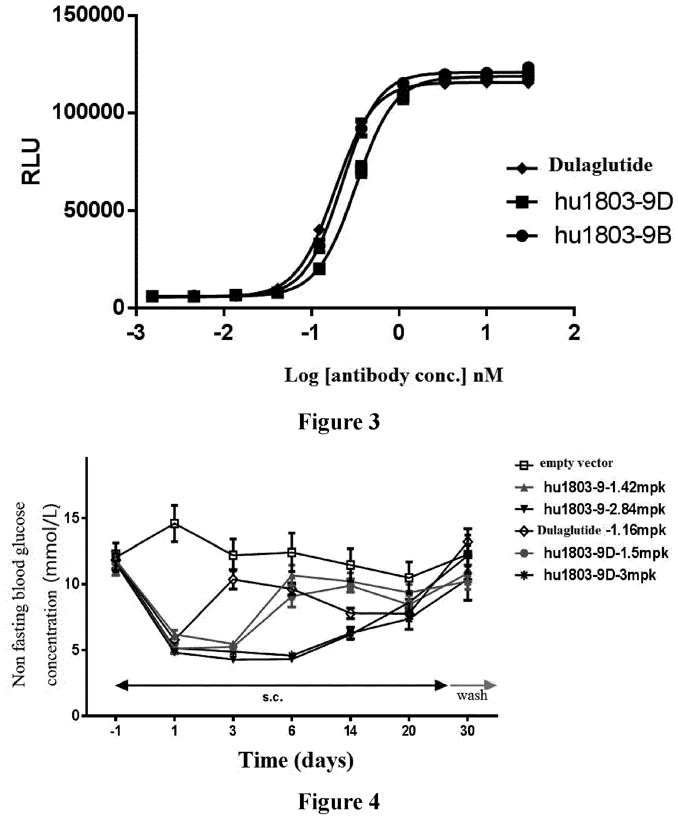

Figure 3 and Table 17 show that both hu1803-9B and hu1803-9D can completely activate the GLP-1R, and have comparable efficacy and EC50 (the concentration required to activate 50% of the maximum activity) to that of the positive control (Dulaglutide), indicating that both hu1803-9B and hu1803-9D retain the complete biological activity of the GLP-1 part.

**Table 18. The activating activity of bispecific proteins on human GLP-1R**

| Sample | EC50 (nM) | Emax% |
|---|---|---|
| ch1805-D | 0.11 | 108 |
| ch1808-D | 0.14 | 107 |
| ch1817-D | 0.30 | 106 |

Table 18 shows that all of the chimeric antibodies ch1805-D, ch1808-D and ch1817-D linked to GLP1 peptide can completely activate GLP-1R, and have comparable efficacy and EC50 (the concentration required to activate 50% of the maximum activity) to that of the positive control (Dulaglutide), indicating that the bispecific proteins formed by linking various GCGR antibodies to the GLP1 peptide in a manner as disclosed herein do not affect the biological activity of the GLP1 peptide.

### Pharmacokinetic evaluation

### Test Example 7: In vivo pharmacokinetic detection in C57 mice

Four laboratory C57 mice, female, were kept at 12/12 hours light/dark cycle regulation, constant temperature of 24± 3°C, humidity 50-60%, and ad libitum access to water and diet. Mice were purchased from Jiesijie Laboratory Animal Co., Ltd. On the day of the test, equimolar hu1803-9D and the positive control drug Dulaglutide were injected into tail vein of C57 mice, at a dose of 6 mg/kg and 2.35 mg/kg respectively, and at an injection volume of 10 ml/kg.

Since both the GLP-1/GCGR antibody bispecific protein of the present disclosure and the positive control Dulaglutide have cross-activity with mice, the time of drug metabolism in mice is shorter. The selected time points for blood collections were: 0h, 1h, 24h (day 2) on day 1 post-administration, and day 3. Blood was collected from the retinal vein of the mouse, 150 µl each time (1.5 µl of DPP-4 inhibitor was added to the blood collection tube before blood collection). The collected blood sample was placed at 4°C for half an hour until coagulation, and then centrifuged at 14000×g for 5 minutes at 4°C. The supernatant (about 80µl) was colletced and stored at -80°C immediately.

The detection process is described as follows:
a. 1µg/mL anti-GLP1 (Novus, NBP1-05180) antibody was plated at 4 °C overnight.
b. Washed 4 times with 250 µl 1×PBST, 200 µl PBS comprising 5% milk was added, and blocked at 37°C for 3 hours.
c. Washed 4 times with 250 µl 1×PBST, 100 µl of test drugs gradient diluted with mouse blank serum was added, and incubated at 37°C for 2 hours.
d. Washed 3 times with 250 µl 1×PBST.
e. 100 µl horseradish peroxidase-labeled secondary antibody anti-human IgG Fc was added to each well, and incubated at 37°C for 1 hour.
f. Washed 3 times with 250 µl 1×PBST.
g. 100 µl TMB was added to each well, incubated at room temperature for 10 minutes and then 100 µl 1M H₂SO₄ was added to stop the reaction.
h. Absorbance was measured at 450nm on the microplate reader, and the data were analyzed with Graphpad Prism 5.

**Table 19. T1/2 of bispecific protein in mice**

| Test drug | Mode of administration | T1/2 (h) |
|---|---|---|
| hu1803-9D | IV (6 mg/kg) | 23.4 |
| Dulaglutide | IV (2.35 mg/kg) | 10 |

The results of PK analysis show that the half-life of the bispecific protein molecule hu1803-9D of the present disclosure in mice is about 23.4 h, which is twice as much as the half-life of the positive control drug.

### Evaluation of in vivo biological activity

### Test Example 8: In vivo drug efficacy test in ob/ob mice

1. The mouse strain used in this test was diabetic ob/ob mice and wild-type mice of the same age (Institute of Model Animals, Nanjing University). The purpose is to observe the treatment effect of the GLP-1/GCGR antibody bispecific protein on diabetes-related indicators such as blood glucose, glycosylated hemoglobin, body weight and food intake after continuous administrations in ob/ob mice.

Before the test, the animal models were divided into 6 groups according to the random and fasting weight, random and fasting blood glucose on the day of test, respectively:
Model control group, 2.84mg/kg and 1.42mg/kg GCGR monoclonal antibody groups (hu1803-9), 1.16mg/kg positive control group (Dulaglutide) and 3mg/kg and 1.5mg/kg hul803-9D. The mice in control group were injected subcutaneously (S.C.) with phosphate buffer, and mice in each group were injected subcutaneously once a week (9:00 AM), for a total of 4 times (Table 20).

**Table 20. Test group and dosing situation**

| **Group** | **Treatment** | **Dose** | **Dosing frequency** | **Mode of administration** |
|---|---|---|---|---|
| 1 | ob/ob mouse model control group | PBS | Once a week×4 weeks | S.C. |
| 2 | GCGR monoclonal antibody, low-dose group | 1.42mpk | Once a week×4 weeks | S.C. |
| 3 | GCGR monoclonal antibody, high-dose group | 2.84mpk | Once a week×4 weeks | S.C. |
| 4 | Positive control, high-dose group | 1.16mpk | Once a week×4 weeks | S.C. |
| 4 | hu1803-9D low-dose group | 1.5mpk | Once a week×4 weeks | S.C. |
| 5 | hu1803-9D high-dose group | 3mpk | Once a week×4 weeks | S.C. |

### 2. Experimental procedures

a. Fasting and random body weight were measured once a week, and food and water intake were measured once a day.
b. The blood glucose was randomly measured before the first administration and on days 1, 2, 3, and 7 after the administration, and then the blood glucose was measured once a week afterwards. The blood glucose after 6 hours of fasting was measured before the first administration and on days 3 and 7 after the administration, and then the fasting blood glucose was measured once a week.
c. On day 26 of administration, after the animals were fasted for 6 hours (8:00-14:00), a single dose of 2g/kg glucose solution was intraperitoneally administered, and the glucose administration time was recorded as 0. The animals were tested for blood glucose at 0 min before the glucose administration, and 15, 30, 60, 90, and 120 minutes after the glucose administration. A glucose tolerance curve was drawn on the basis of the blood glucose data versus time, and the area under the curve (AUC) was calculated.
d. After the experiment, the mice were fasted for 6 hours (8:00-14:00) and then euthanized. Blood samples were taken from the heart, the whole blood was divided into two parts, one part of about 30µl was added into a centrifuge tube pre-added with anticoagulant and stored for the determination of glycosylated hemoglobin, and the other part was allowed to rest on the bench and then centrifuged to obtain serum for the determination of TG, FFA, CHOL, HDL and LDL levels.
e. Data were analyzed by graphpad Prism 6 software, and Student-t test was used for statistical analysis of data.

### 3. Experimental results:

### 1) Effect of long-term administration on random blood glucose concentrations in ob/ob mice:

As shown in Figure 4, during the entire experiment, the random blood glucose is maintained at a high level in the model control group of ob/ob mice. The random blood glucose levels in each of administration groups decreased to varying degrees after subcutaneous injection of each agent at different doses for once a week, showing a favorable dose efficiency which is significantly lower than those in the model control group. Administrations of 2.84 mg/kg of hu1803-9 and 3 mg/kg of hu1803-9D exhibit significantly improved effects in lowering the blood glucose level compared to the other test groups, while the 3 mg/kg of hu1803-9D administered on days 3, 6, 14 and 30 show better effects on lowering the random blood glucose.

### 2) The effect of long-term administration on the fasting blood glucose in ob/ob mice:

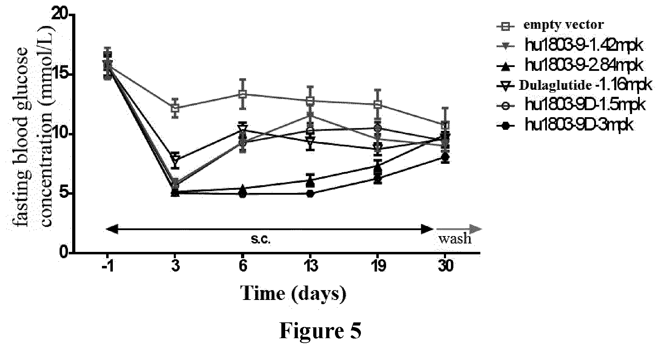

As shown in Figure 5, during the entire experiment, the random blood glucose was maintained at a high level in the model control group of ob/ob mice. The fasting blood glucose levels in each of administration groups decreased to various degrees after subcutaneous injection of each agent at different doses for once a week, showing a favorable dose efficiency which is significantly lower than those in the model control group. Similar to the test of random blood glucose concentration, administrations of 2.84 mg/kg of hu1803-9 and 3 mg/kg of hu1803-9D exhibit significantly improved effects in lowering the blood glucose level compared to the other test groups, while the 3 mg/kg of hu1803-9D administered on days 3, 6, 13 and 30 show better effects on lowering the fasting blood glucose.

### 3) Effects of long-term administration on glycosylated hemoglobin (HbA1c) in ob/ob mice:

The results are shown in Table 21. The glycosylated hemoglobin level in each of administration groups decreased to varying degrees 30 days after once-a -week subcutaneous injection of each agent at different doses, and significantly lower than those in the model control group (P<0.05). The glycosylated hemoglobin levels in the 3mg/kg and 1.5 mg/kg hu1803-9D groups were 5.5±0.2% and 4.7±0.1%, respectively, showing a significant dose-efficacy relationship; among them, the 3 mg/kg hu1803-9D group has a lower level than that in the equimolar 1.16 mg/kg positive control Dulaglutide and 2.84mg/kg GCGR monoclonal antibody hu1803-9 (P<0.05).

**Table 21. The effect of long-term administration on HbAlc % in ob/ob mice**

| **Group** | **HbA1c (%)** | **percentage of increase %** |
|---|---|---|
| ob/ob model control group | 6.3±0.3 | - |
| hu1803-9-1.42mpk | 5.2±0.1 | 17.46% |
| hu1803-9-2.84mpk | 5.2±0.1 | 17.46% |
| Dulaglutide-1.16mpk | 5.6±0.1 | 11.1% |
| hu1803-9D-1.5mpk | 5.5±0.2 | 12.7% |
| hu1803-9D-3mpk | 4.7±0.1 | 25.4% |

### Test Example 9: Competitive ELISA assay of GCGR antibody

The GCGR epitopes bound by GCGR antibodies were classified by ELISA assay, in which the competitive binding of the biotin-labeled antibodies with different concentrations of the standard antibody to GCGR-overexpressing CHO cells was detected. The method for preparing the cell plate was the same as that of Test Example 1. The following specific procedures are as follows:
The antibody was labelled according to the biotin labeling kit instructions (Dojindo Molecular Technologies, Inc. LK03). The unlabeled test antibody was diluted to a range of concentrations with sample dilution solution in a 96-well cell plate, 50µl/well, and incubated at 37°C for 2 hours. After the incubation was finished, the plate was washed 3 times with PBST, 50µlwell of biotin-labeled antibody diluted with sample dilution solution was added, at a concentration of 0.1 µg/ml, incubated at 37°C for 2 hours, the plate was washed 3 times with PBST, and HRP-labeled goat-anti-human secondary antibody (JacksoW ImmuWo Research, Cat Wo. 109-035-003) was added, and incubated at 37°C for 1 hour. The plate was washed 3 times with PBST, 50µlwell of TMB chromogenic substrate (KPL, Cat No. 52-00-03) was added, incubated at room temperature for 10 min, and 50µlwell of 1M H₂SO₄ was added to stop the reaction. The absorbance value was read by a microplate reader (Thermo scientific Multiskan MK3) at a wavelength of 450 nm, and the data was analyzed with GraphPad Prism 5.

The lower the concentration of the biotin-labeled antibody bound to the cell plate for competition, the lower the OD value is, and vice versa. The competition efficiency was calculated according to the formula:
IC% = (test antibody highest OD ₄₅₀ₙₘ - test antibody minimum OD ₄₅₀ₙₘ) / (test antibody highest OD₄₅₀ₙₘ - labeled antibody minimum OD ₄₅₀ₙₘ), the results are shown in the table 22 below.

**Table 22. Competitive binding relationship among antibodies**

| | Competitive antibody | | | | |
|---|---|---|---|---|---|
| Antibody | ch1808 | hu1803-9 | hu1810-12 | ch1817 | ch1822 |
| ch1805 | 100% | 100% | 100% | - | - |
| ch1808 | 100% | 46.60% | 54.30% | - | - |
| hu1803-9 | 100% | 100% | 100% | - | - |
| hu1810-12 | 100% | 100% | 100% | - | - |
| ch1817 | - | - | - | 100% | 100% |
| ch1822 | - | - | - | 75.40% | 100% |

The results show that ch1817 and ch1822 have very close epitopes; ch1808, hu1803-9 and hu1810-12 have very close epitopes; while the epitopes bound by ch1808, hu1803-9 and hu1810-12 are presumed to be within the range of epitopes bound by ch1805.

## Claims

1. An anti-GCGR monoclonal antibody or antigen-binding fragment thereof, comprising a combination of a heavy chain variable region and a light chain variable region selected from the group consisting of:
a) the heavy chain variable region comprising HCDR1, HCDR2 and HCDR3 regions as shown in SEQ ID NOs: 48, 49 and 50, respectively, and
the light chain variable region comprising LCDR1, LCDR2 and LCDR3 regions as shown in SEQ ID NOs: 51, 52 and 53, respectively; or
b) the heavy chain variable region comprising HCDR1, HCDR2 and HCDR3 regions as shown in SEQ ID NOs: 38, 39 and 54, respectively, and
the light chain variable region comprising LCDR1, LCDR2, and LCDR3 regions as shown in SEQ ID NOs: 55, 56 and 57, respectively.

2. The anti-GCGR monoclonal antibody or antigen-binding fragment thereof according to claim 1, comprising a combination of a heavy chain variable region and a light chain variable region selected from the group consisting of:
i) the heavy chain variable region comprising HCDR1, HCDR2 and HCDR3 regions as shown in SEQ ID NOs: 14, 15 and 16, respectively, and
the light chain variable region comprising LCDR1, LCDR2, and LCDR3 regions as shown in SEQ ID NOs: 17, 18 and 19, respectively;
ii) the heavy chain variable region comprising HCDR1, HCDR2 and HCDR3 regions as shown in SEQ ID NOs: 20, 21 and 22, respectively, and
the light chain variable region comprising LCDR1, LCDR2, and LCDR3 regions as shown in SEQ ID NOs: 23, 24 and 25, respectively;
iii) the heavy chain variable region comprising HCDR1, HCDR2 and HCDR3 regions as shown in SEQ ID NOs: 26, 27 and 28, respectively, and
the light chain variable region comprising LCDR1, LCDR2, and LCDR3 regions as shown in SEQ ID NOs: 29, 30 and 31, respectively;
iv) the heavy chain variable region comprising HCDR1, HCDR2 and HCDR3 regions as shown in SEQ ID NOs: 32, 33 and 34, respectively, and
the light chain variable region comprising LCDR1, LCDR2, and LCDR3 regions as shown in SEQ ID NOs: 35, 36 and 37, respectively;
v) the heavy chain variable region comprising HCDR1, HCDR2 and HCDR3 regions as shown in SEQ ID NOs: 38, 39 and 40, respectively, and
the light chain variable region comprising LCDR1, LCDR2, and LCDR3 regions as shown in SEQ ID NOs: 41, 42 and 43, respectively; and
vi) the heavy chain variable region comprising HCDR1, HCDR2 and HCDR3 regions as shown in SEQ ID NOs: 38, 39 and 44, respectively, and
the light chain variable region comprising LCDR1, LCDR2, and LCDR3 regions as shown in SEQ ID NOs: 45, 46 and 47, respectively.

3. The anti-GCGR monoclonal antibody or antigen-binding fragment thereof according to claim 1 or 2, which is a murine antibody or antigen-binding fragment thereof, a chimeric antibody or antigen-binding fragment thereof, or a humanized antibody or antigen-binding fragment thereof.

4. The anti-GCGR monoclonal antibody or antigen-binding fragment thereof according to claim 3, the humanized antibody comprising a framework region derived from a human antibody or a framework region variant thereof, wherein:
the framework region variant has at most 10 amino acid back mutations in the light chain framework region and/or the heavy chain framework region of the human antibody, respectively,
preferably, the amino acid back mutation is selected from:
aa) one or more back mutation(s) of 42G, 44V, 71Y and 87F comprised in the light chain variable region, and/or
one or more back mutation(s) of 38K, 481, 67A, 69F, 71A, 73P, 78A and 93S comprised in the heavy chain variable region; or
ab) one or more back mutation(s) of 38L, 44V, 59S, 70E and 71Y comprised in the light chain variable region, and/or
one or more back mutation(s) of 38K, 481, 66K, 67A, 69L, 73R, 78M and 94S comprised in the heavy chain variable region.

5. The anti-GCGR monoclonal antibody or antigen-binding fragment thereof according to claim 3, comprising a combination of a light chain variable region and a heavy chain variable region selected from the group consisting of:
c) the heavy chain variable region, the sequence of which is as shown in any one of SEQ ID NOs: 2, 61, 62, 63 and 64, or has at least 90% sequence identity to any one of SEQ ID NOs: 2, 61, 62, 63 and 64; and
the light chain variable region, the sequence of which is as shown in any one of SEQ ID NOs:3, 58, 59 and 60 or has at least 90% sequence identity to any one of SEQ ID NOs: 3, 58, 59 and 60;
d) the heavy chain variable region, the sequence of which is as shown in SEQ ID NO: 4 or has at least 90% sequence identity to SEQ ID NO: 4; and
the light chain variable region, the sequence of which is as shown in SEQ ID NO: 5 or has at least 90% sequence identity to SEQ ID NO: 5;
e) the heavy chain variable region, the sequence of which is as shown in SEQ ID NO: 6 or has at least 90% sequence identity to SEQ ID NO: 6; and
the light chain variable region, the sequence of which is as shown in SEQ ID NO: 7 or has at least 90% sequence identity to SEQ ID NO: 7;
f) the heavy chain variable region, the sequence of which is as shown in any one of SEQ ID NOs: 8, 68, 69, 70 and 71, or has at least 90% sequence identity to any one of SEQ ID NOs: 8, 68, 69, 70 and 71 ; and
the light chain variable region, the sequence of which is as shown in any one of SEQ ID NOs: 9, 65, 66 and 67 or has at least 90% sequence identity to any one of SEQ ID NOs: 9, 65, 66 and 67;
g) the heavy chain variable region, the sequence of which is as shown in SEQ ID NO: 10 or has at least 90% sequence identity to SEQ ID NO: 10; and
the light chain variable region, the sequence of which is as shown in SEQ ID NO: 11 or has at least 90% sequence identity to SEQ ID NO: 11; and
h) the heavy chain variable region, the sequence of which is as shown in SEQ ID NO: 12 or has at least 90% sequence identity to SEQ ID NO: 12; and
the light chain variable region, the sequence of which is as shown in SEQ ID NO: 13 or has at least 90% sequence identity to SEQ ID NO: 13.

6. The anti-GCGR monoclonal antibody or antigen-binding fragment thereof according to any one of claims 1 to 5, wherein the antibody is a full-length antibody, further comprising antibody constant region(s),
preferably, the heavy chain constant region is selected from the group consisting of human IgG1, IgG2, IgG3 and IgG4 constant regions and conventional variants thereof, and the light chain constant region is selected from the group consisting of human antibody κ and λ chain constant regions and conventional variants thereof,
more preferably, comprising the human antibody heavy chain constant region as shown in SEQ ID NO: 72 and the human antibody light chain constant region as shown in SEQ ID NO: 73.

7. The anti-GCGR monoclonal antibody or antigen-binding fragment thereof according to claim 6, comprising a combination of a heavy chain and a light chain selected from any one of the group consisting of:
j) the heavy chain as shown in SEQ ID NO: 74, 76 or 78 or having at least 85% sequence identity thereto, and the light chain as shown in SEQ ID NO: 75, 77 or 79 or having at least 85% sequence identity thereto;
k) the heavy chain as shown in SEQ ID NO: 80 or having at least 85% sequence identity thereto, and the light chain as shown in SEQ ID NO: 81 or having at least 85% sequence identity thereto;
1) the heavy chain as shown in SEQ ID NO: 82 or having at least 85% sequence identity thereto, and the light chain as shown in SEQ ID NO: 83 or having at least 85% sequence identity thereto;
m) the heavy chain as shown in SEQ ID NO: 84 or having at least 85% sequence identity thereto, and the light chain as shown in SEQ ID NO: 85 or having at least 85% sequence identity thereto;
n) the heavy chain as shown in SEQ ID NO: 86 or having at least 85% sequence identity thereto, and the light chain as shown in SEQ ID NO: 87 or having at least 85% sequence identity thereto; and
o) the heavy chain as shown in SEQ ID NO: 88 or having at least 85% sequence identity thereto, and the light chain as shown in SEQ ID NO: 89 or having at least 85% sequence identity thereto.

8. An anti-GCGR monoclonal antibody or antigen-binding fragment thereof, comprising combination of a heavy chain variable region and a light chain variable region selected from the group consisting of:
ac) the heavy chain variable region comprising the same HCDR1, HCDR2 and HCDR3 regions as those of the heavy chain variable region as shown in SEQ ID NO: 2, and
the light chain variable region comprising the same LCDR1, LCDR2 and LCDR3 regions as those of the light chain variable region as shown in SEQ ID NO: 3;
ad) the heavy chain variable region comprising the same HCDR1, HCDR2 and HCDR3 regions as those of the heavy chain variable region as shown in SEQ ID NO: 4, and
the light chain variable region comprising the same LCDR1, LCDR2 and LCDR3 regions as those of the light chain variable region as shown in SEQ ID NO: 5;
ae) the heavy chain variable region comprising the same HCDR1, HCDR2 and HCDR3 regions as those of the heavy chain variable region as shown in SEQ ID NO: 6, and
the light chain variable region comprising the same LCDR1, LCDR2 and LCDR3 regions as those of the light chain variable region as shown in SEQ ID NO: 7;
af) the heavy chain variable region comprising the same HCDR1, HCDR2 and HCDR3 regions as those of the heavy chain variable region as shown in SEQ ID NO: 8, and
the light chain variable region comprising the same LCDR1, LCDR2 and LCDR3 regions as those of the light chain variable region as shown in SEQ ID NO: 9;
ag) the heavy chain variable region comprising the same HCDR1, HCDR2 and HCDR3 regions as those of the heavy chain variable region as shown in SEQ ID NO: 10, and
the light chain variable region comprising the same LCDR1, LCDR2 and LCDR3 regions as those of the light chain variable region as shown in SEQ ID NO: 11; and
ah) the heavy chain variable region comprising the same HCDR1, HCDR2 and HCDR3 regions as those of the heavy chain variable region as shown in SEQ ID NO: 12, and
the light chain variable region comprising the same LCDR1, LCDR2 and LCDR3 regions as those of the light chain variable region as shown in SEQ ID NO: 13.

9. The anti-GCGR monoclonal antibody or antigen-binding fragment thereof according to any one of claims 1 to 8, wherein the antigen-binding fragment is selected from the group consisting of Fab, Fab', F(ab')2, single-chain antibody, dimerized V region (diabody) and disulfide-stabilized V region (dsFv).

10. A bispecific protein comprising a GLP-1 peptide and an anti-GCGR antibody or antigen-binding fragment thereof, wherrin the GLP-1 peptide is covalently linked to the anti-GCGR antibody or antigen-binding fragment thereof via a peptide bond or a linker.

11. The bispecific protein according to claim 10, wherein:
the carboxyl terminus of the GLP-1 peptide is linked to the amino terminus of the heavy chain variable region of the anti-GCGR monoclonal antibody or antigen-binding fragment thereof via a peptide bond or a linker; or
the carboxyl terminus of the GLP-1 peptide is linked to the amino terminus of the light chain variable region of the anti-GCGR antibody or antigen-binding fragment thereof via a peptide bond or a linker.

12. The bispecific protein according to claim 11, wherein the anti-GCGR antibody or antigen-binding fragment thereof is the anti-GCGR monoclonal antibody or antigen-binding fragment thereof according to any one of claims 1 to 9.

13. The bispecific protein according to any one of claims 10 to 12, wherein:
the GLP-1 peptide is the GLP-1 peptide as shown in SEQ ID NO: 91 or variant thereof,
the GLP-1 peptide variant comprises one or more amino acid mutation(s) of Q17E, I23V K28R and G30R based on the GLP-1 peptide as shown in SEQ ID NO: 91,
preferably, the GLP-1 peptide variant comprises Q17E mutation, or comprises Q17E and 123V mutations.

14. The bispecific protein according to claim 13, wherein the sequence of the GLP-1 peptide variant is as shown in SEQ ID NO: 92, 93, 94, 95, 96, 97, 98 or 99.

15. The bispecific protein according to claim 14, the bispecific protein comprising a first polypeptide chain and a second polypeptide chain, the first polypeptide chain comprising the heavy chain of the anti-GCGR monoclonal antibody according to any one of claims 1 to 9; and the second polypeptide chain comprising the light chain of the anti-GCGR monoclonal antibody according to any one of claims 1 to 9; wherein:
(ai) the first polypeptide chain comprises a polypeptide selected from any one of SEQ ID NOs: 100, 101, 102, 103, 104, 105, 106, 107 and 108, and
the second polypeptide chain comprises a polypeptide as shown in SEQ ID NO: 79;
(aj) the first polypeptide chain comprises a polypeptide as shown in SEQ ID NO: 109, and
the second polypeptide chain comprises a polypeptide as shown in SEQ ID NO: 81;
(ak) the first polypeptide chain comprises a polypeptide as shown in SEQ ID NO: 110, and the second polypeptide chain comprises a polypeptide as shown in SEQ ID NO: 83;
(al) the first polypeptide chain comprises a polypeptide as shown in SEQ ID NO: 111, and the second polypeptide chain comprises a polypeptide as shown in SEQ ID NO: 85;
(am) the first polypeptide chain comprises a polypeptide as shown in SEQ ID NO: 112, and the second polypeptide chain comprises a polypeptide as shown in SEQ ID NO: 87; or
(an) the first polypeptide chain comprises a polypeptide as shown in SEQ ID NO: 113, and the second polypeptide chain comprises a polypeptide as shown in SEQ ID NO: 89.

16. A GLP-1 peptide variant comprising one or more amino acid mutation(s) of Q17E, I23V, K28R and G30R based on the GLP-1 peptide as shown in SEQ ID NO: 91, preferably, the GLP-1 peptide variants comprising Q17E mutation, or comprising Q17E and 123V mutations.

17. The GLP-1 peptide variant according to claim 16, wherein the sequence of the GLP-1 peptide variant is as shown in SEQ ID NO: 92, 93, 94, 95, 96, 97, 98 or 99.

18. A pharmaceutical composition comprising:
a therapeutically effective amount of the anti-GCGR monoclonal antibody or antigen-binding fragment thereof according to any one of claims 1 to 9, or the bispecific protein according to any one of claims 10 to 15, or the GLP-1 peptide variant according to claim 16 or 17, and
one or more pharmaceutically acceptable carriers, diluents, buffers or excipients.

19. An isolated nucleic acid molecule encoding the anti-GCGR monoclonal antibody or antigen-binding fragment thereof according to any one of claims 1 to 9, or the bispecific protein according to any one of claims 10 to 15, or the GLP-1 peptide variant according to claim 16 or 17.

20. A recombinant vector comprising the isolated nucleic acid molecule of claim 19.

21. A host cell transformed with the recombinant vector of claim 20, the host cell being selected from the group consisting of prokaryotic cells and eukaryotic cells, preferably eukaryotic cells, more preferably mammalian cells or insect cells.

22. A method for preparing the anti-GCGR monoclonal antibody or antigen-binding fragment thereof according to any one of claims 1 to 9, or the bispecific protein according to any one of claims 10 to 15, or the GLP-1 peptide variant according to claim 16 or 17, the method comprising:
culturing the host cell according to claim 21 to generate the anti-GCGR monoclonal antibody or antigen-binding fragment thereof according to any one of claims 1 to 9, or the bispecific protein according to any one of claims 10 to 15, or the GLP-1 peptide variant according to claim 16 or 17, and
recovering the monoclonal antibody or antigen-binding fragment thereof, or the bispecific protein, or the GLP-1 peptide variant from the culture.

23. A reagent for detecting human GCGR in a sample, which comprises the anti-GCGR monoclonal antibody or antigen-binding fragment thereof according to any one of claims 1 to 9.

24. A method for lowering the blood glucose concentration in a subject, the method comprising:
administering to the subject a therapeutically effective amount of the anti-GCGR monoclonal antibody or antigen-binding fragment thereof according to any one of claims 1 to 9, or the bispecific protein according to any one of claims 10 to 15, or the GLP-1 peptide variant according to claim 16 or 17, or the pharmaceutical composition according to claim 18,
preferably, the therapeutically effective amount is 0.1 to 3000 mg of the anti-GCGR monoclonal antibody or antigen-binding fragment thereof according to any one of claims 1 to 9, or the bispecific protein according to any one of claims 10 to 15 , or the GLP-1 peptide variant according to claim 16 or 17 in a unit dose.

25. A method for the treatment of a metabolic disorder, the method comprising:
administering to a subject a therapeutically effective amount of the anti-GCGR monoclonal antibody or antigen-binding fragment thereof according to any one of claims 1 to 9, or the bispecific protein according to any one of claims 10 to 15, or the GLP-1 peptide variant according to claim 16 or 17, or the pharmaceutical composition according to claim 18;
preferably, the metabolic disorder is selected from the group consisting of: metabolic syndrome, obesity, impaired glucose tolerance, diabetes, diabetic ketoacidosis, hyperglycemia, hyperglycemic hyperosmolar syndrome, perioperative hyperglycemia, hyperinsulinemia, insulin resistance syndrome, impaired fasting glucose, dyslipidemia, atherosclerosis and prediabetic conditions.
